# EUROPEAN PATENT SPECIFICATION

(11) **EP 2 124 817 B1**
(45) Date of publication and mention of the grant of the patent: **22.12.2021**
(21) Application number: 08731329.2
(22) Date of filing: 04.03.2008
(51) Int. Cl.: A61F 2/06, A61L 31/12, A61L 31/14, A61L 31/16

(54) **BIOABSORBABLE COATINGS FOR MEDICAL DEVICES**
BIOABSORBIERBARE BESCHICHTUNGEN FÜR MEDIZINISCHE VORRICHTUNGEN
REVÊTEMENTS BIOABSORBABLES POUR DISPOSITIFS MÉDICAUX

(30) Priority: 09.03.2007 US 906174 P
(43) Date of publication of application: 02.12.2009
(73) Proprietor: ICON Medical Corp., Atlanta, GA 30332 (US)
(72) Inventor: ROTH, Noah M., Atlanta, GA 30329 (US); BRODBECK, William, Hudson, OH 44236 (US); KONDABATNI, Kishore, Atlanta, GA 30318 (US)
(74) Representative: Grosse Schumacher Knauer von Hirschhausen
(86) International application number: PCT/US2008/055766
(87) International publication number: WO 2008/112458

(56) References cited:
- EP-A2- 0 875 218
- WO-A2-03/065881
- WO-A2-2006/096251
- WO-A2-2006/110197
- US-A1- 2006 106 453
- US-A1- 2006 161 242
- US-A1- 2006 198 869
- US-A1- 2007 123 973
- E. ALT ET AL.: "Inhibition of Neointima FormationAfter Experimental Coronary Artery Stenting", CIRCULATION, vol. 101, 2000, pages 1453-1458, XP002714772,
- None

## Description

The present invention claims priority on United States Patent Application Serial No. 60/906,174 filed March 9, 2007.

The invention relates to a medical device comprising a stent. Disclosed is an implant for use within a body, and more particularly an expandable graft which is useful in repairing various types of body passageways, and even more particularly to an expandable graft which is useful in repairing blood vessels narrowed or occluded by disease.

### BACKGROUND OF THE INVENTION

Medical treatment of various illnesses or diseases commonly includes the use of one or more medical devices. Two types of medical devices that are commonly used to repair various types of body passageways are an expandable graft or stent, or a surgical graft. These devices have been implanted in various areas of the mammalian anatomy.

Old age, dietary habits and primary genetics can also lead to a common disease, atherosclerosis. Atherosclerotic plaques and blockages consist of lipids, fibroblasts and fibrin that proliferate and cause obstruction of a vessel. As the obstruction grows, the blood flow diminishes and reaches a level that is insufficient to meet the biological needs of one or more organs. The end result is defined as ischemia.

One purpose of a stent is to open a blocked or partially blocked body passageway.

When a stent is used in a blood vessel, the stent is used to open the occluded vessel to achieve improved blood flow which is necessary to provide for the anatomical function of an organ. The procedure of opening a blocked or partially blocked body passageway commonly includes the use of one or more stents in combination with other medical devices such as, but not limited to, an introducer sheath, a guiding catheter, a guide wire, an angioplasty balloon, etc.

During the insertion of the stent, some disruption of the native body passageway can occur. This disruption to the body passageway can start a cascade of biological occurrences that can hinder the function of the implanted stent. When a stent is inserted into a blood vessel, a) platelets can be activated, b) smooth muscle cells can migrate and/or c) endothelial cells, which protect the vessel, can be disrupted thus leading to the cascade of clot formation. Clot formation can lead to the failure of the stent. The accumulation of platelets about the implanted stent is known as thrombosis.

Another medical procedure that is utilized frequently involves bypass (heart surgery), or peripheral (non-cardiac) grafting. This medical procedure entails using a surgical graft constructed of an artificial material that replaces or by-passes the diseased portion of the vessel. This procedure is accomplished by ligating the diseased portion of the vessel, temporarily stopping blood flow, and physically sewing in the surgical with a suture. The failure of the medical procedure commonly occurs at the suture (anastomoses) site. When the surgical graft is connected to a blood vessel, a) platelets can be activated, b) smooth muscle cells can migrate and/or c) endothelial cells, which protect the vessel, can be disrupted thus leading to the cascade of clot formation. The clot formation can lead to the failure of the surgical graft. The accumulation of platelets about the sutured regions is also known as thrombosis. This failure can then lead to repeat procedures, amputation and/or other medical complications.

During and after a medical procedure, the patient is commonly placed on aggressive anti-platelet and/or anti-coagulation therapy. A major concern and side effect of such treatment is an increased incidence of bleeding complications. These bleeding complications can make the most routine procedure such as getting your teeth cleaned prohibited.

Many other types of diseases are treatable with stents, catheters, surgical grafts, and/or other devices inserted into vessels or other locations in the body. In addition, various types of orthopedic devices can be used to treat various diseased and/or damaged areas of a body. One desirable technique would be to deliver one or more biological agents directly to the site that has been treated and/or at the site of potential failure once a medical device has been inserted in the treatment site. In one non-limiting example, it would be desirable to have a medical device and/or a medical method or technique that can be used to deliver an anti-platelet and/or other medication to the region of a body passageway which has been treated by a stent or by another interventional technique. In another and/or alternative non-limiting example, it would be desirable to have a medical device that could deliver one or more biological agents over the short term (e.g, seconds, minutes, hours, days) with a potential burst effect of the one or more biological agents, and/or the long term (e.g., days, weeks, months, years) after the initial implantation of the medical device. In still another and/or alternative non-limiting example, it would be desirable to provide control over the delivery rate of one or more biological agents from the medical device, thus limiting or eliminating the systemic effects of taking a drug (e.g, orally, intravenously, etc.) over extended periods of time.

WO2006096251 discloses stents comprising metal alloys based on molybdenum and rhenium which are coated with biologically active agents.

In view of the present state of medical device technology, there is a need and demand for a medical device that can be inserted into a treatment site and which has improved procedural success rates.

### SUMMARY OF THE INVENTION

The invention is defined by the claims. Any subject matter falling outside the scope of the claims is provided for information purposes only.

The previously mentioned short-comings of prior art medical devices are addressed by the novel medical device of the present invention according to claim 1. The medical device of the present invention can be designed to be inserted into a treatment site such that the medical device has improved procedural success rates. The improved procedural success rate by the medical device of the present invention can be achievable without the need for or with a significant reduction in need for aggressive anti-platelet and/or anti-coagulation therapy after insertion of the medical device in the treatment area. The improved medical procedure disclosed, (not falling under the scope of the claims) can be at least partially obtained by the use of a medical device that 1) is formed of one or more materials that enhances the physical properties of the medical device, and/or 2) includes one or more biological agents (e.g., anti-platelet medication, etc.) that can be controllably and/or uncontrollably delivered at, adjacent to and/or into a treatment site by the medical device. The medical device in accordance with the present invention comprises a stent. The medical device is directed for use in a body passageway.

As used herein, the term "body passageway" is defined to be any passageway or cavity in a living organism (e.g., bile duct, bronchiole tubes, nasal cavity, blood vessels, heart, esophagus, trachea, stomach, fallopian tube, uterus, ureter, urethra, the intestines, lymphatic vessels, nasal passageways, eustachian tube, acoustic meatus, subarachnoid space, and central and peripheral nerve conduits, etc.). The techniques employed to deliver the device to a treatment area include, but are not limited to angioplasty, vascular anastomoses, transplantation, implantation, surgical implantation, subcutaneous introduction, minimally invasive surgical procedures, interventional procedures, and any combinations thereof. For vascular applications, the term "body passageway" primarily refers to blood vessels and chambers in the heart. The device can be an expandable stent suitable for endovascular delivery and expandable by a balloon and/or other means (e.g., by its own internal forces "self expandable") and can have many shapes and forms. Such shapes are limited to stents, disclosed in United States Patent or Publication Nos. 6,206,916; 6,436,133; 6,776,794; 6,805,707; 6,955,686; 2002/0032478; 2002/0045935; 2004/0093076; 2004/0093077; 2004/0172128; 2005/0004657; 2005/0075716; 2006/0009836; 2006/0085059; 2006/0100690; 2006/0106452; 2006/0106453; 2006/0142843; D481,139; and all the prior art cited in these patents and patent publications.

The device which is in the form of a stent is designed to be maneuvered into a treatment area (e.g., body passageway, etc.) and then expanded in the treatment area to enable better or proper fluid flow through the body passageway. The device comprises a stent.

In one non-limiting aspect, the medical device has one or more features that at least partially result in the inhibition or prevention of thrombosis after the medical device has been implanted in a treatment area. These features include, but are not limited to, 1) the shape and/or profile of the medical device, 2) the one or more materials that are used to at least partially form the medical device, and/or 3) the one or more biological agents that are at least partially coated on, contained therein and/or included in the medical device. As a result, the need or use of body-wide standard aggressive anti-platelet and/or anti-coagulation therapy for extended periods of time to inhibit or prevent the occurrence of thrombosis is not required in conjunction with the medical device.

In the past, the use of body-wide therapy was used by the patient long after the patient left the hospital or other type of medical facility. This body-wide therapy could last days, weeks, months or sometimes over a year after surgery. The medical device can be applied or inserted into a treatment area and 1) merely require reduced use of body wide therapy after application or insertion of the medical device, or 2) does not require use of body wide therapy after application or insertion of the medical device. As such, the medical device can be designed to be inserted in a treatment area without any temporary use and/or without extended use of body wide aggressive anti-platelet and/or anti-coagulation therapy after the medical device has been inserted in the treatment area. This method of treating a treatment area with a medical device while inhibiting or preventing the occurrence of thrombosis at or near the treatment area is a significant improvement over past treatment procedures. In one non-limiting example, no body-wide therapy is needed after the insertion of the medical device into a patient. In another and/or alternative non-limiting example, short term use of body-wide therapy is needed or used after the insertion of the medical device into a patient. Such short term use can be terminated after the release of the patient from the hospital or other type of medical facility, or one to two days or weeks after the release of the patient from the hospital or other type of medical facility; however, it will be appreciated that other time periods of body-wide therapy can be used. As a result of the use of the medical device of the present invention, the use of body-wide therapy after a medical procedure involving the insertion of a medical device into a treatment area can be significantly reduced or eliminated.

In another non-limiting aspect, the medical device can include, contain and/or be coated with one or more chemical agents that include, but are not limited to a substance, pharmaceutical, biologic, veterinary product, drug, and analogs or derivatives otherwise formulated and/or designed to prevent, inhibit and/or treat one or more clinical and/or biological events, and/or to promote healing. Non-limiting examples of clinical events that can be addressed by one or more agents include, but are not limited to viral, fungus and/or bacteria infection; vascular diseases and/or disorders; digestive diseases and/or disorders; reproductive diseases and/or disorders; lymphatic diseases and/or disorders; cancer; implant rejection; pain; nausea; swelling; arthritis; bone diseases and/or disorders; organ failure; immunity diseases and/or disorders; cholesterol problems; blood diseases and/or disorders; lung diseases and/or disorders; heart diseases and/or disorders; brain diseases and/or disorders; neuralgia diseases and/or disorders; kidney diseases and/or disorders; ulcers; liver diseases and/or disorders; intestinal diseases and/or disorders; gallbladder diseases and/or disorders; pancreatic diseases and/or disorders; psychological disorders; respiratory diseases and/or disorders; gland diseases and/or disorders; skin diseases and/or disorders; hearing diseases and/or disorders; oral diseases and/or disorders; nasal diseases and/or disorders; eye diseases and/or disorders; fatigue; genetic diseases and/or disorders; burns; scarring and/or scars; trauma; weight diseases and/or disorders; addiction diseases and/or disorders; hair loss; cramps; muscle spasms; tissue repair; nerve repair; neural regeneration and/or the like. In one non-limiting embodiment, the one or more chemical agents that can be include with, contained in and/or be coated on the medical device include, but are not limited to, an anti-platelet compound and/or anticoagulant compound such as, but not limited to, warfarin (Coumadin), warfarin derivatives, aspirin, aspirin derivatives, clopidogrel, clopidogrel derivatives, ticlopadine, ticlopadine derivatives, hirdun, hirdun derivatives, dipyridamole, dipyridamole derivatives, trapidil, trapidil derivatives, taxol, taxol derivatives, cytochalasin, cytochalasin derivatives, paclitaxel, paclitaxel derivatives, rapamycin, rapamycin derivatives, GM-CSF, GM-CSF derivatives, heparin, heparin derivatives, low molecular weight heparin, low molecular weight heparin derivatives, or combinations thereof. One specific non-limiting example of an anti-thrombotic inhibitor that can be include with, contained in and/or be coated on the medical device includes 1) huridin and/or derivatives, and/or 2) alagors (e.g., bivalirudin, etc.) and/or derivatives. As can be appreciated, one or more other anti-thrombotic chemical agents can be used with the medical device. Non-limiting examples of chemical agents that can be used include, but are not limited to, 5-Fluorouracil and/or derivatives thereof; ACE inhibitors and/or derivatives thereof; acenocoumarol and/or derivatives thereof; acyclovir and/or derivatives thereof; actilyse and/or derivatives thereof; adrenocorticotropic hormone and/or derivatives thereof; adriamycin and/or derivatives thereof; chemical agents that modulate intracellular Ca2+ transport such as L-type (e.g., diltiazem, nifedipine, verapamil, etc.) or T-type Ca2+ channel blockers (e.g., amiloride, etc.); alpha-adrenergic blocking agents and/or derivatives thereof; alteplase and/or derivatives thereof; amino glycosides and/or derivatives thereof (e.g., gentamycin, tobramycin, etc.); angiopeptin and/or derivatives thereof; angiostatic steroid and/or derivatives thereof; angiotensin II receptor antagonists and/or derivatives thereof; anistreplase and/or derivatives thereof; antagonists of vascular epithelial growth factor and/or derivatives thereof; anti-biotics; anti-coagulant compounds and/or derivatives thereof; anti-fibrosis compounds and/or derivatives thereof; antifungal compounds and/or derivatives thereof; anti-inflammatory compounds and/or derivatives thereof; Anti-Invasive Factor and/or derivatives thereof; anti-metabolite compounds and/or derivatives thereof (e.g., staurosporin, trichothecenes, and modified diphtheria and ricin toxins, Pseudomonas exotoxin, etc.); anti-matrix compounds and/or derivatives thereof (e.g., colchicine, tamoxifen, etc.); anti-microbial agents and/or derivatives thereof; anti-migratory agents and/or derivatives thereof (e.g., caffeic acid derivatives, nilvadipine, etc.); anti-mitotic compounds and/or derivatives thereof; anti-neoplastic compounds and/or derivatives thereof; anti-oxidants and/or derivatives thereof; anti-platelet compounds and/or derivatives thereof; anti-proliferative and/or derivatives thereof; anti-thrombogenic agents and/or derivatives thereof; argatroban and/or derivatives thereof; ap-1 inhibitors and/or derivatives thereof (e.g., for tyrosine kinase, protein kinase C, myosin light chain kinase, Ca2+/calmodulin kinase II, casein kinase II, etc.); aspirin and/or derivatives thereof; azathioprine and/or derivatives thereof; $-Estradiol and/or derivatives thereof; $-1-anticollagenase and/or derivatives thereof; calcium channel blockers and/or derivatives thereof; calmodulin antagonists and/or derivatives thereof (e.g., H7, etc.); CAPTOPRIL and/or derivatives thereof; cartilage-derived inhibitor and/or derivatives thereof; ChIMP-3 and/or derivatives thereof; cephalosporin and/or derivatives thereof (e.g., cefadroxil, cefazolin, cefaclor, etc.); chloroquine and/or derivatives thereof; chemotherapeutic compounds and/or derivatives thereof (e.g., 5-fluorouracil, vincristine, vinblastine, cisplatin, doxyrubicin, adriamycin, tamocifen, etc.); chymostatin and/or derivatives thereof; CILAZAPRIL and/or derivatives thereof; clopidigrel and/or derivatives thereof; clotrimazole and/or derivatives thereof; colchicine and/or derivatives thereof; cortisone and/or derivatives thereof; coumadin and/or derivatives thereof; curacin-A and/or derivatives thereof; cyclosporine and/or derivatives thereof; cytochalasin and/or derivatives thereof (e.g., cytochalasin A, cytochalasin B, cytochalasin C, cytochalasin D, cytochalasin E, cytochalasin F, cytochalasin G, cytochalasin H, cytochalasin J, cytochalasin K, cytochalasin L, cytochalasin M, cytochalasin N, cytochalasin O, cytochalasin P, cytochalasin Q, cytochalasin R, cytochalasin S, chaetoglobosin A, chaetoglobosin B, chaetoglobosin C, chaetoglobosin D, chaetoglobosin E, chaetoglobosin F, chaetoglobosin G, chaetoglobosin J, chaetoglobosin K, deoxaphomin, proxiphomin, protophomin, zygosporin D, zygosporin E, zygosporin F, zygosporin G, aspochalasin B, aspochalasin C, aspochalasin D, etc.); cytokines and/or derivatives thereof; desirudin and/or derivatives thereof; dexamethazone and/or derivatives thereof; dipyridamole and/or derivatives thereof; eminase and/or derivatives thereof; endothelin and/or derivatives thereof endothelial growth factor and/or derivatives thereof; epidermal growth factor and/or derivatives thereof; epothilone and/or derivatives thereof; estramustine and/or derivatives thereof; estrogen and/or derivatives thereof; fenoprofen and/or derivatives thereof; fluorouracil and/or derivatives thereof; flucytosine and/or derivatives thereof; forskolin and/or derivatives thereof; ganciclovir and/or derivatives thereof; glucocorticoids and/or derivatives thereof (e.g., dexamethasone, betamethasone, etc.); glycoprotein IIb/IIIa platelet membrane receptor antibody and/or derivatives thereof; GM-CSF and/or derivatives thereof; griseofulvin and/or derivatives thereof; growth factors and/or derivatives thereof (e.g., VEGF; TGF; IGF; PDGF; FGF, etc.); growth hormone and/or derivatives thereof; heparin and/or derivatives thereof; hirudin and/or derivatives thereof; hyaluronate and/or derivatives thereof; hydrocortisone and/or derivatives thereof; ibuprofen and/or derivatives thereof; immunosuppressive agents and/or derivatives thereof (e.g., adrenocorticosteroids, cyclosporine, etc.); indomethacin and/or derivatives thereof; inhibitors of the sodium/calcium antiporter and/or derivatives thereof (e.g., amiloride, etc.); inhibitors of the IP3 receptor and/or derivatives thereof; inhibitors of the sodium/hydrogen antiporter and/or derivatives thereof (e.g., amiloride and derivatives thereof, etc.); insulin and/or derivatives thereof; Interferon alpha 2 Macroglobulin and/or derivatives thereof; ketoconazole and/or derivatives thereof; Lepirudin and/or derivatives thereof; LISINOPRIL and/or derivatives thereof; LOVASTATIN and/or derivatives thereof; marevan and/or derivatives thereof; mefloquine and/or derivatives thereof; metalloproteinase inhibitors and/or derivatives thereof; methotrexate and/or derivatives thereof; metronidazole and/or derivatives thereof; miconazole and/or derivatives thereof; monoclonal antibodies and/or derivatives thereof; mutamycin and/or derivatives thereof; naproxen and/or derivatives thereof; nitric oxide and/or derivatives thereof; nitroprusside and/or derivatives thereof; nucleic acid analogues and/or derivatives thereof (e.g., peptide nucleic acids, etc.); nystatin and/or derivatives thereof; oligonucleotides and/or derivatives thereof; paclitaxel and/or derivatives thereof; penicillin and/or derivatives thereof; pentamidine isethionate and/or derivatives thereof; phenindione and/or derivatives thereof; phenylbutazone and/or derivatives thereof; phosphodiesterase inhibitors and/or derivatives thereof; Plasminogen Activator Inhibitor-1 and/or derivatives thereof; Plasminogen Activator Inhibitor-2 and/or derivatives thereof; Platelet Factor 4 and/or derivatives thereof; platelet derived growth factor and/or derivatives thereof; plavix and/or derivatives thereof; POSTMI 75 and/or derivatives thereof; prednisone and/or derivatives thereof; prednisolone and/or derivatives thereof; probucol and/or derivatives thereof; progesterone and/or derivatives thereof; prostacyclin and/or derivatives thereof; prostaglandin inhibitors and/or derivatives thereof; protamine and/or derivatives thereof; protease and/or derivatives thereof; protein kinase inhibitors and/or derivatives thereof (e.g., staurosporin, etc.); quinine and/or derivatives thereof; radioactive agents and/or derivatives thereof (e.g., Cu-64, Ca-67, Cs-131, Ga-68, Zr-89, Ku-97, Tc-99m, Rh-105, Pd-103,Pd-109, In-111,I-123, I-125, I-131, Re-186, Re-188, Au-198, Au-199, Pb-203, At-211, Pb-212, Bi-212, H3P32O4, etc.); rapamycin and/or derivatives thereof; receptor antagonists for histamine and/or derivatives thereof; refludan and/or derivatives thereof; retinoic acids and/or derivatives thereof; revasc and/or derivatives thereof; rifamycin and/or derivatives thereof; sense or anti-sense oligonucleotides and/or derivatives thereof (e.g., DNA, RNA, plasmid DNA, plasmid RNA, etc.); seramin and/or derivatives thereof; steroids; seramin and/or derivatives thereof; serotonin and/or derivatives thereof; serotonin blockers and/or derivatives thereof; streptokinase and/or derivatives thereof; sulfasalazine and/or derivatives thereof; sulfonamides and/or derivatives thereof (e.g., sulfamethoxazole, etc.); sulphated chitin derivatives; Sulphated Polysaccharide Peptidoglycan Complex and/or derivatives thereof; TH1 and/or derivatives thereof (e.g., Interleukins-2, -12, and -15, gamma interferon, etc.); thioprotese inhibitors and/or derivatives thereof; taxol and/or derivatives thereof (e.g., taxotere, baccatin, 10-deacetyltaxol, 7-xylosyl-10-deacetyltaxol, cephalomannine, 10-deacetyl-7-epitaxol, 7 epitaxol, 10-deacetylbaccatin III, 10-deacetylcephaolmannine, etc.); ticlid and/or derivatives thereof; ticlopidine and/or derivatives thereof; tick anti-coagulant peptide and/or derivatives thereof; thioprotese inhibitors and/or derivatives thereof; thyroid hormone and/or derivatives thereof; Tissue Inhibitor of Metalloproteinase-1 and/or derivatives thereof; Tissue Inhibitor of Metalloproteinase-2 and/or derivatives thereof; tissue plasma activators; TNF and/or derivatives thereof, tocopherol and/or derivatives thereof; toxins and/or derivatives thereof; tranilast and/or derivatives thereof; transforming growth factors alpha and beta and/or derivatives thereof; trapidil and/or derivatives thereof; triazolopyrimidine and/or derivatives thereof; vapiprost and/or derivatives thereof; vinblastine and/or derivatives thereof; vincristine and/or derivatives thereof; zidovudine and/or derivatives thereof. As can be appreciated, the chemical agent can include one or more derivatives of the above listed compounds and/or other compounds. In one non-limiting embodiment, the chemical agent includes, but is not limited to, trapidil, Trapidil derivatives, taxol, taxol derivatives (e.g., taxotere, baccatin, 10-deacetyltaxol, 7-xylosyl-10-deacetyltaxol, cephalomannine, 10-deacetyl-7-epitaxol, 7 epitaxol, 10-deacetylbaccatin III, 10-deacetylcephaolmannine, etc.), cytochalasin, cytochalasin derivatives (e.g., cytochalasin A, cytochalasin B, cytochalasin C, cytochalasin D, cytochalasin E, cytochalasin F, cytochalasin G, cytochalasin H, cytochalasin J, cytochalasin K, cytochalasin L, cytochalasin M, cytochalasin N, cytochalasin O, cytochalasin P, cytochalasin Q, cytochalasin R, cytochalasin S, chaetoglobosin A, chaetoglobosin B, chaetoglobosin C, chaetoglobosin D, chaetoglobosin E, chaetoglobosin F, chaetoglobosin G, chaetoglobosin J, chaetoglobosin K, deoxaphomin, proxiphomin, protophomin, zygosporin D, zygosporin E, zygosporin F, zygosporin G, aspochalasin B, aspochalasin C, aspochalasin D, etc.), paclitaxel, paclitaxel derivatives, rapamycin, rapamycin derivatives, GM-CSF (granulo-cytemacrophage colony-stimulating-factor), GM-CSF derivatives, statins or HMG-CoA reductase inhibitors forming a class of hypolipidemic agents, combinations, or analogs thereof, or combinations thereof. The type and/or amount of chemical agent included in the device and/or coated on the device can vary. When two or more chemical agents are included in and/or coated on the device, the amount of two or more chemical agents can be the same or different. The type and/or amount of chemical agent included on, in and/or in conjunction with the device are generally selected to address one or more clinical events. Typically the amount of chemical agent included on, in and/or used in conjunction with the device is about 0.01-100ug per mm² amd/or at least about 0.01 weight percent of device; however, other amounts can be used. In one non-limiting embodiment, the device can be partially of fully coated and/or impregnated with one or more chemical agents to facilitate in the success of a particular medical procedure. The amount of two of more chemical agents on, in and/or used in conjunction with the device can be the same or different. The one or more chemical agents can be coated on and/or impregnated in the device by a variety of mechanisms such as, but not limited to, spraying (e.g., atomizing spray techniques, etc.), flame spray coating, powder deposition, dip coating, flow coating, dip-spin coating, roll coating (direct and reverse), sonication, brushing, plasma deposition, depositing by vapor deposition, MEMS technology, and rotating mold deposition. In another non-limiting embodiment, the type and/or amount of chemical agent included on, in and/or in conjunction with the device is generally selected for the treatment of one or more clinical events. Typically the amount of chemical agent included on, in and/or used in conjunction with the device is about 0.01-100ug per mm² and/or at least about 0.01-100 weight percent of the device; however, other amounts can be used. The amount of two of more chemical agents on, in and/or used in conjunction with the device can be the same or different. For instance, portions of the device to provide local and/or systemic delivery of one or more chemical agents in and/or to a body passageway to a) inhibit or prevent thrombosis, in-stent restenosis, vascular narrowing and/or restenosis after the device has been inserted in and/or connected to a body passageway, b) at least partially passivate, remove, encapsulate, and/or dissolve lipids, fibroblast, fibrin, etc. in a body passageway so as to at least partially remove such materials and/or to passivate such vulnerable materials (e.g., vulnerable plaque, etc.) in the body passageway in the region of the device and/or downstream of the device. As can be appreciated, the one or more chemical agents can have many other or additional uses. In still another and/or alternative non-limiting example, the device is coated with and/or includes one or more chemical agents such as, but not limited to chemical agents associated with thrombolytics, vasodilators, anti-hypertensive agents, antimicrobial or anti-biotic, anti-mitotic, anti-proliferative, anti-secretory agents, non-steroidal anti-inflammatory drugs, immunosuppressive agents, growth factors and growth factor antagonists, endothelial growth factors and growth factor antagonists, antitumor and/or chemotherapeutic agents, anti-polymerases, anti-viral agents, anti-body targeted therapy agents, hormones, anti-oxidants, biologic components, radio-therapeutic agents, radiopaque agents and/or radio-labeled agents. In addition to these chemical agents, the device can be coated with and/or include one or more chemical agents that are capable of inhibiting or preventing any adverse biological response by and/or to the device that could possibly lead to device failure and/or an adverse reaction by human or animal tissue. A wide range of chemical agents thus can be used.

In a further non-limiting aspect, the one or more chemical agents on and/or in the device, when used on the device, can be released in a controlled manner so the area in question to be treated is provided with the desired dosage of chemical agent over a sustained period of time. As can be appreciated, controlled release of one or more chemical agents on the device is not always required and/or desirable. As such, one or more of the chemical agents on and/or in the device can be uncontrollably released from the device during and/or after insertion of the device in the treatment area. It can also be appreciated that one or more chemical agents on and/or in the device can be controllably released from the device and one or more chemical agents on and/or in the device can be uncontrollably released from the device. It can also be appreciated that one or more chemical agents on and/or in one region of the device can be controllably released from the device and one or more chemical agents on and/or in the device can be uncontrollably released from another region on the device. As such, the device can be designed such that 1) all the chemical agent on and/or in the device is controllably released, 2) some of the chemical agent on and/or in the device is controllably released and some of the chemical agent on the device is non-controllably released, or 3) none of the chemical agent on and/or in the device is controllably released. The device can also be designed such that the rate of release of the one or more chemical agents from the device is the same or different. The device can also be designed such that the rate of release of the one or more chemical agents from one or more regions on the device is the same or different. Non-limiting arrangements that can be used to control the release of one or more chemical agent from the device include a) at least partially coat one or more chemical agents with one or more polymers, b) at least partially incorporate and/or at least partially encapsulate one or more chemical agents into and/or with one or more polymers, c) insert one or more chemical agents in pores, passageway, cavities, etc. in the device and at least partially coat or cover such pores, passageway, cavities, etc. with one or more polymers, and/or incorporate one or more chemical agents in the one or more polymers that at least partially form the device. As can be appreciated, other or additional arrangements can be used to control the release of one or more chemical agent from the device. The one or more polymers used to at least partially control the release of one or more chemical agent from the device can be porous or non-porous. The one or more chemical agents can be inserted into and/or applied to one or more surface structures and/or micro-structures on the device, and/or be used to at least partially form one or more surface structures and/or micro-structures on the device. As such, the one or more chemical agents on the device can be 1) coated on one or more surface regions of the device, 2) inserted and/or impregnated in one or more surface structures and/or micro-structures, etc. of the device, and/or 3) form at least a portion or be included in at least a portion of the structure of the device. When the one or more chemical agents are coated on the device, the one or more chemical agents can, but is not required to, 1) be directly coated on one or more surfaces of the device, 2) be mixed with one or more coating polymers or other coating materials and then at least partially coated on one or more surfaces of the device, 3) be at least partially coated on the surface of another coating material that has been at least partially coated on the device, and/or 4) be at least partially encapsulated between a) a surface or region of the device and one or more other coating materials and/or b) two or more other coating materials. As can be appreciated, many other coating arrangements can be additionally or alternatively used. When the one or more chemical agents are inserted and/or impregnated in one or more portions of the device, one or more surface structure and/or micro-structures of the device, and/or one or more surface structures and/or micro-structures of the device, 1) one or more other polymers can be applied at least partially over the one or more surface structure and/or micro-structures, surface structures and/or micro-structures of the device, 2) one or more polymers can be combined with one or more chemical agents, and/or 3) one or more polymers can be coated over or more portions of the body of the device; however, this is not required. As such, the one or more chemical agents can be 1) embedded in the structure of the device; 2) positioned in one or more surface structure and/or micro-structures of the device; 3) encapsulated between two polymer coatings; 4) encapsulated between the base structure and a polymer coating; 5) mixed in the base structure of the device that includes at least one polymer coating; or 6) one or more combinations of 1, 2, 3, 4 and/or 5. In addition or alternatively, the one or more coatings of the one or more polymers on the device can include 1) one or more coatings of non-porous polymers; 2) one or more coatings of a combination of one or more porous polymers and one or more non-porous polymers; 3) one or more coating of porous polymer, or 4) one or more combinations of options 1, 2, and 3. As can be appreciated different chemical agents can be located in and/or between different polymer coating layers and/or on and/or the structure of the device. As can also be appreciated, many other and/or additional coating combinations and/or configurations can be used. In a further non-limiting embodiment, the device can be embedded with and/or impregnated with one or more chemical agents using a solvent to temporarily and/or permanently increase the porosity of the structure of a non-porous and/or porous polymer coating and/or device and be used to transport one or more chemical agents into the matrix of the device. One or more solvents can be used to transport one or more chemical agents. Solvent suitability is a function of compatibility with one or more chemical agents and one or more materials of the device. Non-limiting examples of solvents include Dimethyl sulfoxide (DMSO), chloroform, ethylene, methanol, ethyl acetate, and the broader class of biocompatible or non-biocompatible solvents. The concentration of one or more chemical agents, the type of polymer, the type and/or shape of surface structure and/or micro-structures in the device and/or the coating thickness of one or more chemical agents can be used to control the release time, the release rate and/or the dosage amount of one or more chemical agents; however, other or additional combinations can be used. As such, the chemical agent and polymer system combination and location on the device can be numerous. As can also be appreciated, one or more chemical agents can be deposited on the top surface of the device to provide an initial uncontrolled burst effect of the one or more chemical agents prior to 1) the control release of the one or more chemical agents through one or more layers of polymer system that include one or more nonporous polymers and/or 2) the uncontrolled release of the one or more chemical agents through one or more layers of polymer system. The one or more chemical agents and/or polymers can be coated on and/or impregnated in the device by a variety of mechanisms such as, but not limited to, spraying (e.g., atomizing spray techniques, etc.), flame spray coating, powder deposition, dip coating, flow coating, dip-spin coating, roll coating (direct and reverse), sonication, brushing, plasma deposition, depositing by vapor deposition, MEMS technology, and rotating mold deposition. The thickness of each polymer layer and/or layer of chemical agent is generally at least about 0.01 µm and is generally less than about 150 µm. In one non-limiting embodiment, the thickness of a polymer layer and/or layer of chemical agent is about 0.02-75µm, more particularly about 0.05-50 µm, and even more particularly about 1-30 µm. When the device includes and/or is coated with one or more chemical agents such that at least one of the chemical agents is at least partially controllably released from the device, the need or use of body-wide therapy for extended periods of time can be reduced or eliminated. In the past, the use of body-wide therapy was used by the patient long after the patient left the hospital or other type of medical facility. This body-wide therapy could last days, weeks, months or sometimes over a year after surgery. The device can be applied or inserted into a treatment area and 1) merely requires reduced use and/or extended use of systemic therapy after application or insertion of the device or 2) does not require use and/or extended use of systemic therapy after application or insertion of the device. As can be appreciated, use and/or extended use of systemic therapy can be used after application or insertion of the device at the treatment area. In one non-limiting example, no body-wide therapy is needed after the insertion of the device into a patient. In another and/or alternative non-limiting example, short term use of systemic therapy is needed or used after the insertion of the device into a patient. Such short term use can be terminated after the release of the patient from the hospital or other type of medical facility, or one to two days or weeks after the release of the patient from the hospital or other type of medical facility; however, it will be appreciated that other time periods of systemic therapy can be used. As a result of the use of the device, the use of systemic therapy after a medical procedure involving the insertion of a device into a treatment area can be significantly reduced or eliminated.

In another non-limiting aspect, controlled release of one or more chemical agents from the device, when controlled release is desired, can be accomplished by using one or more non-porous polymer layers and/or by use of one or more biodegradable polymers used to at least partially form the device; however, other and/or additional mechanisms can be used to controllably release the one or more chemical agents. The one or more chemical agents can be at least partially controllably released by molecular diffusion through the one or more non-porous polymer layers and/or from the one or more biodegradable polymers used to at least partially form the device. When one or more non-porous polymer layers are used, the one or more polymer layers are typically biocompatible polymers; however, this is not required. One or more non-porous polymers can be applied to the device without the use of chemical, solvents, and/or catalysts; however, this is not required. In one non-limiting example, the non-porous polymer can be at least partially applied by, but not limited to, vapor deposition and/or plasma deposition. The non-porous polymer can be selected so as to polymerize and cure merely upon condensation from the vapor phase; however, this is not required. The application of the one or more nonporous polymer layers can be accomplished without increasing the temperature above ambient temperature (e.g., 65-90°F (18°C - 32°C)); however, this is not required. The non-porous polymer system can be mixed with one or more chemical agents prior to being formed into at least a portion of the device and/or be coated on the device, and/or be coated on a device that previously included one or more chemical agents; however, this is not required. The use or one or more non-porous polymers allows for accurate controlled release of the chemical agent from the device. The controlled release of one or more chemical agents through the nonporous polymer is at least partially controlled on a molecular level utilizing the motility of diffusion of the chemical agent through the non-porous polymer. In one non-limiting example, the one or more non-porous polymer layers can include, but are not limited to, polyamide, parylene (e.g., parylene C, parylene N) and/or a parylene derivative.

In still another non-limiting aspect, controlled release of one or more chemical agents from the device, when controlled release is desired, can be accomplished by using one or more polymers that form a chemical bond with one or more chemical agents. In one non-limiting example, at least one chemical agent includes trapidil, trapidil derivative or a salt thereof that is covalently bonded to at least one polymer such as, but not limited to, an ethylene-acrylic acid copolymer. The ethylene is the hydrophobic group and acrylic acid is the hydrophilic group. The mole ratio of the ethylene to the acrylic acid in the copolymer can be used to control the hydrophobicity of the copolymer. The degree of hydrophobicity of one or more polymers can also be used to control the release rate of one or more chemical agents from the one or more polymers. The amount of chemical agent that can be loaded with one or more polymers may be a function of the concentration of anionic groups and/or cationic groups in the one or more polymers. For chemical agents that are anionic, the concentration of chemical agent that can be loaded on the one or more polymers is generally a function of the concentration of cationic groups (e.g. amine groups and the like) in the one or more polymer and the fraction of these cationic groups that can ionically bind to the anionic form of the one or more chemical agents. For chemical agents that are cationic (e.g., trapidil, etc.), the concentration of chemical agent that can be loaded on the one or more polymers is generally a function of the concentration of anionic groups (i.e., carboxylate groups, phosphate groups, sulfate groups, and/or other organic anionic groups) in the one or more polymers, and the fraction of these anionic groups that can ionically bind to the cationic form of the one or more chemical agents. As such, the concentration of one or more chemical agents that can be bound to the one or more polymers can be varied by controlling the amount of hydrophobic and hydrophilic monomer in the one or more polymers, by controlling the efficiency of salt formation between the chemical agent, and/or the anionic/cationic groups in the one or more polymers. In still another non-limiting aspect, controlled release of one or more chemical agents from the device, when controlled release is desired, can be accomplished by using one or more polymers that include one or more induced cross-links. These one or more cross-links can be used to at least partially control the rate of release of the one or more chemical agents from the one or more polymers. The cross-linking in the one or more polymers can be instituted by a number of techniques such as, but not limited to, using catalysts, using radiation, using heat, and/or the like. The one or more cross-links formed in the one or more polymers can result in the one or more chemical agents to become partially or fully entrapped within the cross-linking, and/or form a bond with the cross-linking. As such, the partially or fully chemical agent takes longer to release itself from the crosslinking, thereby delaying the release rate of the one or more chemical agents from the one or more polymers. Consequently, the amount of chemical agent, and/or the rate at which the chemical agent is released from the device over time can be at least partially controlled by the amount or degree of cross-linking in the one or more polymers. In still a further and or alternative aspect , a variety of polymers can be coated on the device and/or be used to form at least a portion of the device. The one or more polymers can be used on the medical for a variety of reasons such as, but not limited to, 1) forming a portion of the device, 2) improving a physical property of the device (e.g., improve strength, improve durability, improve biocompatibility, reduce friction, etc.), 3) forming a protective coating on one or more surface structures on the device, 4) at least partially forming one or more surface structures on the medical device, and/or 5) at least partially controlling a release rate of one or more chemical agents from the device. As can be appreciated, the one or more polymers can have other or additional uses on the device. The one or more polymers can be porous, non-porous, biostable, biodegradable (i.e., dissolves, degrades, is absorbed, or any combination thereof in the body), and/or biocompatible. When the device is coated with one or more polymers, the polymer can include 1) one or more coatings of non-porous polymers; 2) one or more coatings of a combination of one or more porous polymers and one or more non-porous polymers; 3) one or more coatings of one or more porous polymers and one or more coatings of one or more non-porous polymers; 4) one or more coatings of porous polymer, or 5) one or more combinations of options 1, 2, 3 and 4. The thickness of one or more of the polymer layers can be the same or different. When one or more layers of polymer are coated onto at least a portion of the device, the one or more coatings can be applied by a variety of techniques such as, but not limited to, vapor deposition and/or plasma deposition, spraying, dip-coating, roll coating, sonication, atomization, brushing and/or the like; however, other or additional coating techniques can be used. The one or more polymers that can be coated on the device and/or used to at least partially form the device can be polymers that are considered to be biodegradable; polymers that are considered to be biostable; and/or polymers that can be made to be biodegradable and/or biodegradable with modification. Non-limiting examples of polymers that are considered to be biodegradable include, but are not limited to, aliphatic polyesters; poly(glycolic acid) and/or copolymers thereof (e.g., poly(glycolide trimethylene carbonate); poly(caprolactone glycolide)); poly(lactic acid) and/or isomers thereof (e.g., poly-L(lactic acid) and/or poly-D Lactic acid) and/or copolymers thereof (e.g. DL-PLA), with and without additives (e.g. calcium phosphate glass), and/or other copolymers (e.g., poly(caprolactone lactide), poly(lactide glycolide), poly(lactic acid ethylene glycol)); poly(ethylene glycol); poly(ethylene glycol) diacrylate; poly(lactide); polyalkylene succinate; polybutylene diglycolate; polyhydroxybutyrate (PHB); polyhydroxyvalerate (PHV); polyhydroxybutyrate/polyhydroxyvalerate copolymer (PHB/PHV); poly(hydroxybutyrate-covalerate); polyhydroxyalkaoates (PHA); polycaprolactone; poly(caprolactone-polyethylene glycol) copolymer; poly(valerolactone); polyanhydrides; poly(orthoesters) and/or blends with polyanhydrides; poly(anhydride-co-imide); polycarbonates (aliphatic); poly(hydroxyl-esters); polydioxanone; polyanhydrides; polyanhydride esters; polycyanoacrylates; poly(alkyl 2-cyanoacrylates); poly(amino acids); poly(phosphazenes); poly(propylene fumarate); poly(propylene fumarate-co-ethylene glycol); poly(fumarate anhydrides); fibrinogen; fibrin; gelatin; cellulose and/or cellulose derivatives and/or cellulosic polymers (e.g., cellulose acetate, cellulose acetate butyrate, cellulose butyrate, cellulose ethers, cellulose nitrate, cellulose propionate, cellophane); chitosan and/or chitosan derivatives (e.g., chitosan NOCC, chitosan NOOC-G); alginate; polysaccharides; starch; amylase; collagen; polycarboxylic acids; poly(ethylester-co-carboxylate carbonate) (and/or other tyrosine derived polycarbonates); poly(iminocarbonate); poly(BPA-iminocarbonate); poly(trimethylene carbonate); poly(iminocarbonate-amide) copolymers and/or other pseudo-poly(amino acids); poly(ethylene glycol); poly(ethylene oxide); poly(ethylene oxide)/poly(butylene terephthalate) copolymer; poly(epsilon-caprolactone-dimethyltrimethylene carbonate); poly(ester amide); poly(amino acids) and conventional synthetic polymers thereof; poly(alkylene oxalates); poly(alkylcarbonate); poly(adipic anhydride); nylon copolyamides; NO-carboxymethyl chitosan NOCC); carboxymethyl cellulose; copoly(ether-esters) (e.g., PEO/PLA dextrans); polyketals; biodegradable polyethers; biodegradable polyesters; polydihydropyrans; polydepsipeptides; polyarylates (L-tyrosine-derived) and/or free acid polyarylates; polyamides (e.g., Nylon 66, polycaprolactam); poly(propylene fumarate-co-ethylene glycol) (e.g., fumarate anhydrides); hyaluronates; poly-p-dioxanone; polypeptides and proteins; polyphosphoester; polyphosphoester urethane; polysaccharides; pseudo-poly(amino acids); starch; terpolymer; (copolymers of glycolide, lactide, or dimethyltrimethylene carbonate); rayon; rayon triacetate; latex; and/copolymers, blends, and/or composites of above. Non-limiting examples of polymers that considered to be biostable include, but are not limited to, parylene; parylene c; parylene f; parylene n; parylene derivatives; maleic anyhydride polymers; phosphorylcholine; poly n-butyl methacrylate (PBMA); polyethylene-co-vinyl acetate (PEVA); PBMA/PEVA blend or copolymer; polytetrafluoroethene (Teflon^{®}) and derivatives; poly-paraphenylene terephthalamide (Kevlar^{®}); poly(ether ether ketone) (PEEK); poly(styrene-b-isobutylene-bstyrene) (Translute^{™}); tetramethyldisiloxane (side chain or copolymer); polyimides polysulfides; poly(ethylene terephthalate); poly(methyl methacrylate); poly(ethylene-co-methyl methacrylate); styrene-ethylene/butylene-styrene block copolymers; ABS; SAN; acrylic polymers and/or copolymers (e.g., n-butyl-acrylate, n-butyl methacrylate, 2-ethylhexyl acrylate, lauryl-acrylate, 2-hydroxy-propyl acrylate, polyhydroxyethyl, methacrylate/methylmethacrylate copolymers); glycosaminoglycans; alkyd resins; elastin; polyether sulfones; epoxy resin; poly(oxymethylene); polyolefins; polymers of silicone; polymers of methane; polyisobutylene; ethylene-alphaolefin copolymers; polyethylene; polyacrylonitrile; fluorosilicones; poly(propylene oxide); polyvinyl aromatics (e.g. polystyrene); poly(vinyl ethers) (e.g. polyvinyl methyl ether); poly(vinyl ketones); poly(vinylidene halides) (e.g. polyvinylidene fluoride, polyvinylidene chloride); poly(vinylpyrolidone); poly(vinylpyrolidone)/vinyl acetate copolymer; polyvinylpridine prolastin or silk-elastin polymers (SELP); silicone; silicone rubber; polyurethanes (polycarbonate polyurethanes, silicone urethane polymer) (e.g., chronoflex varieties, bionate varieties); vinyl halide polymers and/or copolymers (e.g. polyvinyl chloride); polyacrylic acid; ethylene acrylic acid copolymer; ethylene vinyl acetate copolymer; polyvinyl alcohol; poly(hydroxyl alkylmethacrylate); Polyvinyl esters (e.g. polyvinyl acetate); and/or copolymers, blends, and/or composites of above. Non-limiting examples of polymers that can be made to be biodegradable with modification include, but are not limited to, hyaluronic acid (hyanluron); polycarbonates; polyorthocarbonates; copolymers of vinyl monomers; polyacetals; biodegradable polyurethanes; polyacrylamide; polyisocyanates; polyamide; and/or copolymers, blends, and/or composites of above. As can be appreciated, other and/or additional polymers and/or derivatives of one or more of the above listed polymers can be used. The one or more polymers can be coated on and/or impregnated in the device by a variety of mechanisms such as, but not limited to, spraying (e.g., atomizing spray techniques, etc.), flame spray coating, powder deposition, dip coating, flow coating, dip-spin coating, roll coating (direct and reverse), sonication, brushing, plasma deposition, depositing by vapor deposition, MEMS technology, and rotating mold. The thickness of each polymer layer is generally at least about 0.01 µm and is generally less than about 150 µm; however, other thicknesses can be used. In one non-limiting embodiment, the thickness of a polymer layer and/or layer of chemical agent is about 0.02-75µm, more particularly about 0.05 - 50 µm, and even more particularly about 1-30 µm. As can be appreciated, other thicknesses can be used. In one non-limiting embodiment, that at least a portion of the body includes and/or is coated with parylene, PLGA, POE, PGA, PLLA, PAA, PEG, PDLLA, PCL, PDS, PDLGA, chitosan and/or copolymers, blends, and/or composites of above and/or derivatives of one or more of these polymers. In another and/or alternative non-limiting embodiment, at least a portion of the body includes and/or is coated with a nonporous polymer that includes, but is not limited to, polyamide, parylene c, parylene n and/or a parylene derivative. In still another and/or alternative non-limiting embodiment, at least a portion of the body includes and/or is coated with poly(ethylene oxide), poly(ethylene glycol), and poly(propylene oxide), polymers of silicone, methane, tetrafluoroethylene (including TEFLON brand polymers), tetramethyldisiloxane, and the like.

In anothernon-limiting aspect, the medical device, when including and/or is coated with one or more chemical agents, can include and/or can be coated with one or more chemical agents that are the same or different in different regions of the medical device and/or have differing amounts and/or concentrations in differing regions of the medical device. For instance, the medical device can a) be coated with and/or include one or more biologicals on at least one portion of the medical device and at least another portion of the medical device is not coated with and/or includes biological agent; b) be coated with and/or include one or more biologicals on at least one portion of the medical device that is different from one or more biologicals on at least another portion of the medical device; c) be coated with and/or include one or more biologicals at a concentration on at least one portion of the medical device that is different from the concentration of one or more biologicals on at least another portion of the medical device; etc.

In still another non-limiting aspect, one or more surfaces of the medical device can be treated to achieve the desired coating properties of the one or more chemical agents and one or more polymers coated on the medical device. Such surface treatment techniques include, but are not limited to, cleaning, buffing, smoothing, etching (chemical etching, plasma etching, etc.), etc. When an etching process is used, various gasses can be used for such a surface treatment process such as, but not limited to, carbon dioxide, nitrogen, oxygen, Freon, helium, hydrogen, etc. The plasma etching process can be used to clean the surface of the medical device, change the surface properties of the medical device so as to affect the adhesion properties, lubricity properties, etc. of the surface of the medical device. As can be appreciated, other or additional surface treatment processes can be used prior to the coating of one or more chemical agents and/or polymers on the surface of the medical device. In one non-limiting manufacturing process, one or more portions of the medical device are cleaned and/or plasma etched; however, this is not required. Plasma etching can be used to clean the surface of the medical device, and/or to form one or more non-smooth surfaces on the medical device to facilitate in the adhesion of one or more coatings of chemical agents and/or one or more coatings of polymer on the medical device. The gas for the plasma etching can include carbon dioxide and/or other gasses. Once one or more surface regions of the medical device have been treated, one or more coatings of polymer and/or biological agent can be applied to one or more regions of the medical device. For instance, 1) one or more layers of porous or non-porous polymer can be coated on an outer and/or inner surface of the medical device, 2) one or more layers of biological agent can be coated on an outer and/or inner surface of the medical device, or 3) one or more layers of porous or non-porous polymer that includes one or more chemical agents can be coated on an outer and/or inner surface of the medical device. The one or more layers of biological agent can be applied to the medical device by a variety of techniques (e.g., dipping, rolling, brushing, spraying, particle atomization, etc.). One non-limiting coating technique is by an ultrasonic mist coating process wherein ultrasonic waves are used to break up the droplet of biological agent and form a mist of very fine droplets. These fine droplets have an average droplet diameter of about 0.1-3 microns. The fine droplet mist facilitates in the formation of a uniform coating thickness and can increase the coverage area on the medical device.

In still yet another non-limiting aspect, one or more portions of the medical device can 1) include the same or different chemical agents, 2) include the same or different amount of one or more chemical agents, 3) include the same or different polymer coatings, 4) include the same or different coating thicknesses of one or more polymer coatings, 5) have one or more portions of the medical device controllably release and/or uncontrollably release one or more chemical agents, and/or 6) have one or more portions of the medical device controllably release one or more chemical agents and one or more portions of the medical device uncontrollably release one or more chemical agents.

In yet another non-limiting aspect, the device can include a marker material that facilitates enabling the device to be properly positioned in a body passageway. The marker material is typically designed to be visible to electromagnetic waves (e.g., x-rays, microwaves, visible light, infrared waves, ultraviolet waves, etc.); sound waves (e.g., ultrasound waves, etc.); magnetic waves (e.g., MRI, etc.); and/or other types of electromagnetic waves (e.g., microwaves, visible light, infrared waves, ultraviolet waves, etc.). In one non-limiting embodiment, the marker material is visible to x-rays (i.e., radiopaque). The marker material can form all or a portion of the device and/or be coated on one or more portions (flaring portion and/or body portion; at ends of device; at or near transition of body portion and flaring section; etc.) of the device. The location of the marker material can be on one or multiple locations on the device. The size of the one or more regions that include the marker material can be the same or different. The marker material can be spaced at defined distances from one another so as to form ruler-like markings on the device to facilitate in the positioning of the device in a body passageway. The marker material can be a rigid or flexible material. The marker material can be a biostable or biodegradable material. When the marker material is a rigid material, the marker material is typically formed of a metal material (e.g., metal band, metal plating, etc.); however, other or additional materials can be used. When the marker material is a flexible material, the marker material typically is formed of one or more polymers that are marker materials in-of-themselves and/or include one or more metal powders and/or metal compounds. In one non-limiting embodiment, the flexible marker material includes one or more metal powders in combinations with parylene, PLGA, POE, PGA, PLLA, PAA, PEG, PDLLA, PCL, PDS, PDLGA, chitosan and/or derivatives of one or more of these polymers.

In another and/or alternative non-limiting embodiment, the flexible marker material includes one or more metals and/or metal powders of aluminum, barium, bismuth, cobalt, copper, chromium, gold, iron, stainless steel, titanium, vanadium, nickel, zirconium, niobium, lead, molybdenum, platinum, yttrium, calcium, rare earth metals, magnesium, rhenium, zinc, silver, depleted radioactive elements, tantalum and/or tungsten; and/or compounds thereof. The marker material can be coated with a polymer protective material; however, this is not required. When the marker material is coated with a polymer protective material, the polymer coating can be used to 1) at least partially insulate the marker material from body fluids, 2) facilitate in retaining the marker material on the device, 3) at least partially shielding the marker material from damage during a medical procedure and/or 4) provide a desired surface profile on the device. As can be appreciated, the polymer coating can have other or additional uses. The polymer protective coating can be a biostable polymer or a biodegradable polymer (e.g., degrades and/or is absorbed). The coating thickness of the protective coating polymer material, when used, is typically less than about 300 microns; however, other thickness can be used. In one non-limiting embodiment, the protective coating materials include parylene, PLGA, POE, PGA, PLLA, PAA, PEG, PDLLA, PCL, PDS, PDLGA, chitosan and/or copolymers, blends, and/or composites of above and/or derivatives of one or more of these polymers.

In still another and/or alternative aspect of the invention, the medical device according to claim 1, which comprises a stent, can be an expandable device that can be expanded by use of another device (e.g., balloon, etc.) and/or is self expanding. The expandable medical device can be fabricated from a material that has no or substantially no shape memory characteristics or can be fabricated from a material having shape-memory characteristics.

In a further non-limiting aspect, the device or one or more regions of the device can be constructed by use of one or more microfabrication and/or micromachining technology used in creating Micro-Electro-Mechanical Systems (MEMS, e.g., micro-machining, laser micro machining, micro-molding, etc.); however, other or additional manufacturing techniques can be used. The device can include one or more surface structures (e.g., pore, channel, pit, rib, slot, notch, bump, teeth, well, hole, groove, etc.). These structures can be at least partially formed by MEMS technology and/or other types of technology. The device can include one or more micro-structures (e.g., micro-needle, micro-pore, micro-cylinder, micro-cone, micro-pyramid, micro-tube, microparallelopiped, micro-prism, micro-hemisphere, teeth, rib, ridge, ratchet, hinge, zipper, zip-tie like structure, etc.) on the inner, outer, or edge surface of the device. Non-limiting examples of structures that can be formed on the stent, are illustrated in United States Patent Publication Nos. 2004/0093076 and 20040093077 . Typically, the micro-structures, when formed, extend from or into the outer surface no more than about 1000 microns, and more typically less than about 1000 microns; however, other sizes can be used. The micro-structures can be clustered together or disbursed throughout the surface of the device. Similar shaped and/or sized micro-structures and/or surface structures can be used, or different shaped and/or sized microstructures can be used. When one or more surface structures and/or micro-structures are designed to extend from the outer and/or inner surface of the device, the one or more surface structures and/or micro-structures can be formed in the extended position and/or be designed so as to extend from the device during and/or after deployment of the device in a treatment area. The micro-structures and/or surface structures can be designed to contain one or more agents and/or be connected to a passageway, cavity, etc. containing one or more agents; however, this is not required. The one or more surface structures and/or micro-structures can be used to engage and/or penetrate surrounding tissue or organs once the device has been positioned on and/or in a patient; however, this is not required. In another further non-limiting aspect, the micro-structures and/or surface structures can be design to modify surface friction between the device and/or additional devices. For example, micro-structures and/or surface structures created on the inner surface of the device may be used to increase retention of a stent, graft, and/or other suitable device on a delivery catheter. In another further non-limiting aspect, the micro-structures and/or surface structures can be design to create a system of undulations and/or crevasses used to facilitate growth of tissue. In one non-limiting aspect, the micro-structures and/or surface structures can be created on a film that could further be rolled into a shunt for neural regeneration, where the micro-structures and/or surface structures can provide a lattice to support and/or facilitate nerve growth. The one or more surface structures and/or micro-structures can be used to facilitate in forming or maintaining a shape of a device (i.e., see devices in United States Patent Publication Nos. 2004/0093076 and 2004/0093077). The one or more surface structures and/or micro-structures can be at least partially formed by MEMS technology; however, this is not required. In one non-limiting embodiment, the one or more surface structures and/or microstructures can be at least partially formed of an agent, polymer, agent polymer mixture, and/or layering of polymer and agent. One or more of the surface structures and/or micro-structures can include one or more internal passageways that can include one or more materials (e.g., agent, polymer, etc.); however, this is not required. In another further non-limiting aspect, one or more internal passageways can be either connected and/or separated in part. The one or more surface structures and/or micro-structures can be formed by a variety of processes (e.g., machining, chemical modifications, chemical reactions, MEMS technology, etching, laser cutting, etc.). The one or more coatings and/or one or more surface structures and/or micro-structures of the device can be used for a variety of purposes such as, but not limited to, 1) increasing the bonding and/or adhesion of one or more agents, adhesives, marker materials and/or polymers to the device, 2) changing the appearance or surface characteristics of the device, and or 3) controlling the release rate of one or more agents. The one or more microstructures and/or surface structures can be biostable, biodegradable, etc. One or more regions of the device that are at least partially formed by MEMS technology can be biostable, biodegradable, etc. The device or one or more regions of the device can be at least partially covered and/or filled with a protective material so as to at least partially protect one or more regions of the device, and/or one or more microstructures and/or surface structures on the device from damage. One or more regions of the device, and/or one or more micro-structures and/or surface structures on the device can be damaged when the device is 1) packaged and/or stored, 2) unpackaged, 3) connected to and/or otherwise secured and/or placed on another device, 4) inserted into a treatment area, 5) handled by a user, and/or 6) form a barrier between one or more micro-structures and/or surface structures and fluids in the body passageway. As can be appreciated, the device can be damaged in other or additional ways. The protective material can be used to protect the device and one or more micro-structures and/or surface structures from such damage. The protective material can include one or more polymers previously identified above. The protective material can be 1) biostable and/or biodegradable and/or 2) porous and/or non-porous. In one non-limiting design, the polymer is at least partially biodegradable so as to at least partially expose one or more micro-structure and/or surface structure to the environment after the device has been at least partially inserted into a treatment area. In another and/or additional non-limiting design, the protective material includes, but is not limited to, sugar (e.g., glucose, fructose, sucrose, etc.), carbohydrate compound, salt (e.g., NaCl, etc.), parylene, PLGA, POE, PGA, PLLA, PAA, PEG, PDLLA, PCL, PDS, PDLGA, chitosan and/or copolymers, blends, and/or composites of above and/or derivatives of one or more of these polymers; however, other and/or additional materials can be used. In still another and/or additional non-limiting design, the thickness of the protective material is generally less than about 300 microns, and typically less than about 150 microns; however, other thicknesses can be used depending upon the material chose of the protective material. The protective material can be coated by one or more mechanisms previously described herein.

In still yet another non-limiting aspect, the device can include and/or be used with a physical hindrance. The physical hindrance can include, but is not limited to, an adhesive, a sheath, a magnet, tape, wire, string, etc. The physical hindrance can be used to 1) physically retain one or more regions of the device in a particular form or profile, 2) physically retain the device on a particular deployment device, 3) protect one or more surface structures and/or micro-structures on the device, and/or 4) form a barrier between one or more surface regions, surface structures and/or microstructures on the device and the fluids in a body passageway. As can be appreciated, the physical hindrance can have other and/or additional functions. The physical hindrance is typically a biodegradable material; however, a biostable material can be used. The physical hindrance can be designed to withstand sterilization of the device; however, this is not required. The physical hindrance can be applied to, included in and/or be used in conjunction with one or more devices. Additionally or alternatively, the physical hindrance can be designed to be used with and/or in conjunction with a device for a limited period of time and then 1) disengage from the device after the device has been partially or fully deployed and/or 2) dissolve and/or degrade during and/or after the device has been partially or fully deployed; however, this is not required. Additionally or alternatively, the physical hindrance can be designed and be formulated to be temporarily used with a device to facilitate in the deployment of the device; however, this is not required. In one non-limiting use of the physical hindrance, the physical hindrance is designed or formulated to at least partially secure a device to another device that is used to at least partially transport the device to a location for treatment. In another and/or alternative nonlimiting use of the physical hindrance, the physical hindrance is designed or formulated to at least partially maintain the device in a particular shape or form until the device is at least partially positioned in a treatment location. In still another and/or alternative nonlimiting use of the physical hindrance, the physical hindrance is designed or formulated to at least partially maintain and/or secure one type of device to another type of medical instrument or device until the device is at least partially positioned in a treatment location. The physical hindrance can also or alternatively be designed and formulated to be used with a device to facilitate in the use of the device. In one non-limiting use of the physical hindrance, when in the form of an adhesive, can be formulated to at least partially secure a device to a treatment area so as to facilitate in maintaining the device at the treatment area. For instance, the physical hindrance can be used in such use to facilitate in maintaining a device on or at a treatment area until the device is properly secured to the treatment area by sutures, stitches, screws, nails, rod, etc; however, this is not required. Additionally or alternatively, the physical hindrance can be used to facilitate in maintaining a device on or at a treatment area until the device has partially or fully accomplished its objective. The physical hindrance is typically a biocompatible material so as to not cause unanticipated adverse effects when properly used. The physical hindrance can be biostable or biodegradable (e.g., degrades and/or is absorbed, etc.). When the physical hindrance includes or is one or more adhesives, the one or more adhesives can be applied to the device by, but is not limited to, spraying (e.g., atomizing spray techniques, etc.), flame spray coating, powder deposition, dip coating, flow coating, dip-spin coating, roll coating (direct and reverse), sonication, brushing, plasma deposition, depositing by vapor deposition, MEMS technology, and rotating mold deposition on the device. The physical hindrance can also or alternatively form at least a part of the device. One or more regions and/or surfaces of a device can also or alternatively include the physical hindrance. The physical hindrance can include one or more agents and/or other materials (e.g., marker material, polymer, etc.); however, this is not required. When the physical hindrance is or includes an adhesive, the adhesive can be formulated to controllably release one or more agents in the adhesive and/or coated on and/or contained within the device; however, this is not required. The adhesive can also or alternatively control the release of one or more agents located on and/or contained in the device by forming a penetrable or non-penetrable barrier to such agents; however, this is not required. The adhesive can include and/or be mixed with one or more polymers; however, this is not required. The one or more polymers can be used to 1) control the time of adhesion provided by said adhesive, 2) control the rate of degradation of the adhesive, and/or 3) control the rate of release of one or more agents from the adhesive and/or diffusing or penetrating through the adhesive layer; however, this is not required. When the physical hindrance includes a sheath, the sheath can be designed to partially or fully encircle the device. The sheath can be designed to be physically removed from the device after the device is deployed to a treatment area; however, this is not required. The sheath can be formed of a biodegradable material that at least partially degrades over time to at least partially expose one or more surface regions, micro-structures and/or surface structures of the device; however, this is not required. The sheath can include and/or be at least partially coated with one or more biological agents. The sheath includes one or more polymers; however, this is not required. The one or more polymers can be used for a variety of reasons such as, but not limited to, 1) forming a portion of the sheath, 2) improving a physical property of the sheath (e.g., improve strength, improve durability, improve biocompatibility, reduce friction, etc.), and/or 3 at least partially controlling a release rate of one or more agents from the sheath. As can be appreciated, the one or more polymers can have other or additional uses on the sheath.

In still a further non-limiting aspect, the medical device can be fully ui partially formed of a base material that has biostable or bioabsorbable properties. The medical device can be at least partially formed of one or more polymers, chemical agents, metals (e.g., aluminum, barium, bismuth, calcium, carbon, cobalt, copper, chromium, depleted radioactive elements, gold, iron, lead, molybdenum, magnesium, nickel, niobium, platinum, rare earth metals, rhenium, silver, tantalum, titanium, tungsten, vanadium, yttrium, zinc, zirconium, and/or alloys thereof (e.g., stainless steel, nitinol, Cr-Co, Mo-Re, Ta-W, Mg-Zr, Mg-Zn, brass, etc.)), ceramics, and/or fiber reinforced materials (e.g., carbon fiber material, fiberglass, etc.). The medical device generally includes one or more materials that impart the desired properties to the medical device so as to withstand the manufacturing process that is needed to produce the medical device. These manufacturing processes can include, but are not limited to, laser cutting, etching, grinding, water cutting, spark erosion, crimping, annealing, drawing, pilgering, electroplating, electro-polishing, chemical polishing, ion beam deposition or implantation, sputter coating, vacuum deposition, etc.

In an aspect of the present invention, the medical device is fully or partially formed of a base material that is at least partially made of a novel metal alloy having improved properties as compared to past medical devices that were form of stainless steel, or cobalt-chromium alloys. The novel metal alloy used to at least partially form the medical device can improve one or more properties (e.g., strength, durability, hardness, biostability, bendability, coefficient of friction, radial strength, flexibility, tensile strength, longitudinal lengthening, stress-strain properties, improved recoil properties, radiopacity, heat sensitivity, biocompatibility, etc.) of such medical device. These one or more physical properties of the novel metal alloy can be achieved in the medical device without increasing the bulk, volume or weight of the medical device, and in some instances can be obtained even when the volume, bulk and/or weight of the medical device is reduced as compared to medical devices that are at least partially formed from traditional stainless steel or cobalt and chromium alloy materials. The novel metal alloy that is used to at least partially form the medical device can thus 1) increase the radiopacity of the medical device, 2) increase the radial strength of the medical device, 3) increase the tensile strength of the medical device, 4) improve the stress-strain properties of the medical device, 5) improve the crimping and/or expansion properties of the medical device. 6) improve the bendability and/or flexibility of the medical device, 7) improve the strength and/or durability of the medical device, 8) increase the hardness of the medical device, 9) improve the longitudinal lengthening properties of the medical device, 10) improved recoil properties of the medical device, 11) improve the friction coefficient of the medical device, 12) improve the heat sensitivity properties of the medical device, 13) improve the biostability and/or biocompatibility properties of the medical device, and/or 14) enable smaller, thinner and/or lighter weight medical devices to be made. It is believed that a smaller, thinner and/or lighter weight medical device comprising a stent, can be inserted in a body passageway and result in a decreased incidence of thrombosis. It is believed that such a medical device will result in a less adverse response by the body when the medical device is inserted in the body passageway. As such, the medical device can be used without any biological agent included in, contained in, and/or coated on the medical device (not forming part of the invention) and still result in a reduction in the incidence of thrombosis. As such, the need for extended use of body wide aggressive anti-platelet and/or anti-coagulation therapy after the medical device has been inserted in the treatment area can be reduced or eliminated by use of the novel alloy.

According to the present invention, the medical device that includes the novel metal alloy is a stent for use in a body passageway; however, it can be appreciated that other types of medical devices (not forming part of the invention) could be at least partially formed from the novel metal alloy. As used herein, the term "body passageway" is defined to be any passageway or cavity in a living organism (e.g., bile duct, bronchiole tubes, nasal cavity, blood vessels, heart, esophagus, trachea, stomach, fallopian tube, uterus, ureter, urethra, the intestines, lymphatic vessels, nasal passageways, eustachian tube, acoustic meatus, etc.). The techniques employed to deliver the medical device to a treatment area include, but are not limited to, angioplasty, vascular anastomoses, interventional procedures, and any combinations thereof. For vascular applications, the term "body passageway" primarily refers to blood vessels and chambers in the heart. The stent can be an expandable stent that is expandable by a balloon and/or other means. The stent can have many shapes and forms. Such shapes can include, but are not limited to, stents disclosed in United States Patent Nos. 6,206,916 and 6,436,133; and all the prior art cited in these patents.

According to the invention, the medical device includes at least about 60 weight percent of the novel metal alloy. In yet another and/or alternative non-limiting embodiment of the invention, the medical device includes at least about 70 weight percent of the novel metal alloy. In still yet another and/or alternative non-limiting embodiment of the invention, the medical device includes at least about 85 weight percent of the novel metal alloy. In a further and/or alternative non-limiting embodiment of the invention, the medical device includes at least about 90 weight percent of the novel metal alloy. In still a further and/or alternative non-limiting embodiment of the invention, the medical device includes at least about 95 weight percent of the novel metal alloy. In yet a further and/or alternative non-limiting embodiment of the invention, the medical device includes about 100 weight percent of the novel metal alloy.

In still another non-limiting aspect, the novel metal alloy that is used to form all or part of the medical device 1) is not clad, metal sprayed, plated and/or formed (e.g., cold worked, hot worked, etc.) onto another metal, or 2) does not have another metal or metal alloy metal sprayed, plated, clad and/or formed onto the novel metal alloy. It will be appreciated that in some applications, the novel metal alloy of the present invention may be clad, metal sprayed, plated and/or formed onto another metal, or another metal or metal alloy may be plated, metal sprayed, clad and/or formed onto the novel metal alloy when forming all or a portion of a medical device.

The novel metal alloy that is used to form all or a portion of the medical device includes as primary metals rhenium and molybdenum, wherein a "primary metal" is a metal component of the metal alloy that is not a metal impurity, the metal alloy being a solid solution or homogenous solution including the primary metals rhenium and molybdenum in at least about 95 wt.-%, or a solid solution including the primary metals rhenium and molybdenum at about 95-99 wt.-%.

The novel metal alloy can include one or more other metals such as, but not limited to, boron, calcium, chromium, cobalt, copper, gold, iron, lead, magnesium, manganese, mercury, nickel, niobium, platinum, rare earth metals, silicon, silver, sulfur, tantalum, tin, titanium, tungsten, yttrium, zinc, zirconium, and/or alloys thereof.

The novel metal alloy that is used to form all or a portion of the medical device is a novel metal alloy that includes at least about 95 weight percent molybdenum and rhenium. In another and/or alternative non-limiting composition, the content of molybdenum and rhenium in the novel metal alloy is at least about 97 weight percent. In still another and/or alternative non-limiting composition, the content of molybdenum and rhenium in the novel metal alloy is at least about 98 weight percent. In yet another and/or alternative non-limiting composition, the content of molybdenum and rhenium in the novel metal alloy is at least about 99 weight percent. In still yet another and/or alternative non-limiting composition, the content of molybdenum and rhenium in the novel metal alloy is at least about 99.5 weight percent. In a further one non-limiting composition, the content of molybdenum and rhenium in the novel metal alloy is at least about 99.9 weight percent. In still a further and/or alternative non-limiting composition, the content of molybdenum and rhenium in the novel metal alloy is at least about 99.95 weight percent. In yet a further and/or alternative non-limiting composition, the content of molybdenum and rhenium in the novel metal alloy is at least about 99.99 weight percent. As can be appreciated, other weight percentages of the rhenium and molybdenum content of the novel metal alloy can be used. In one non-limiting composition, the purity level of the novel metal alloy is such so as to produce a solid solution of the novel metal alloy. A solid solution or homogeneous solution is defined as a metal alloy that includes two or more primary metals and the combined weight percent of the primary metals is at least about 95 weight percent, typically at least about 99 weight percent, more typically at least about 99.5 weight percent, even more typically at least about 99.8 weight percent, and still even more typically at least about 99.9 weight percent. A primary metal is a metal component of the metal alloy that is not a metal impurity. A solid solution of a novel metal alloy that includes rhenium and molybdenum as the primary metals is an alloy that includes at least about 95-99 weight percent rhenium and molybdenum. It is believed that a purity level of less than 95 weight percent molybdenum and rhenium adversely affects one or more physical properties of the metal alloy that are useful or desired in forming and/or using a medical device. In one embodiment, the rhenium content of the novel metal alloy in accordance with the present invention is at least about 40 weight percent. In one non-limiting composition, the rhenium content of the novel metal alloy is at least about 45 weight percent. In still another and/or alternative non-limiting composition, the rhenium content of the novel metal alloy is about 45-50 weight percent. In yet another and/or alternative non-limiting composition, the rhenium content of the novel metal alloy is about 47-48 weight percent. In still yet another and/or alternative non-limiting composition, the rhenium content of the novel metal alloy is about 47.6-49.5 weight percent. In still another and/or alternative non-limiting composition, the rhenium content of the novel metal alloy is about 47.15-47.5 weight percent. As can be appreciated, other weight percentages of the rhenium content of the novel metal alloy can be used. In another and/or alternative embodiment, the molybdenum content of the novel metal alloy in accordance with the present invention is at least about 40 weight percent. In one non-limiting composition, the molybdenum content of the novel metal alloy is at least about 45 weight percent. In another and/or alternative non-limiting composition, the molybdenum content of the novel metal alloy is at least about 50 weight percent. In still another and/or alternative non-limiting composition, the molybdenum content of the novel metal alloy is about 50-60 percent. In yet another and/or alternative non-limiting composition, the molybdenum content of the novel metal alloy is about 50-56 weight percent. As can be appreciated, other weight percentages of the molybdenum content of the novel metal alloy can be used.

The addition of controlled amounts of titanium, yttrium, and/or zirconium to the molybdenum and rhenium alloy has been found to form a metal alloy that has improved physical properties over a metal alloy that principally includes molybdenum and rhenium. For instance, the addition of controlled amounts of titanium, yttrium, and/or zirconium to the molybdenum and rhenium alloy can result in 1) an increase in yield strength of the alloy as compared to a metal alloy that principally includes molybdenum and rhenium, 2) an increase in tensile elongation of the alloy as compared to a metal alloy that principally includes molybdenum and rhenium, 3) an increase in ductility of the alloy as compared to a metal alloy that principally includes molybdenum and rhenium, 4) a reduction in grain size of the alloy as compared to a metal alloy that principally includes molybdenum and rhenium, 5) a reduction in the amount of free carbon, oxygen and/or nitrogen in the alloy as compared to a metal alloy that principally includes molybdenum and rhenium, and/or 6) a reduction in the tendency of the alloy to form micro-cracks during the forming of the alloy into a medical device as compared to the forming of a medical device from a metal alloy that principally includes molybdenum and rhenium. In still one non-limiting composition, the content of molybdenum and rhenium and the at least one additional metal in the novel metal alloy is at least about 98 weight percent. In yet another and/or alternative non-limiting composition, the content of molybdenum and rhenium and the at least one additional metal in the novel metal alloy is at least about 99 weight percent. In still yet another and/or alternative non-limiting composition, the content of molybdenum and rhenium and the at least one additional metal in the novel metal alloy is at least about 99.5 weight percent. In a further one non-limiting composition, the content of molybdenum and rhenium and the at least one additional metal in the novel metal alloy is at least about 99.9 weight percent. In still a further and/or alternative non-limiting composition, the content of molybdenum and rhenium and the at least one additional metal in the novel metal alloy is at least about 99.95 weight percent. In yet a further and/or alternative non-limiting composition, the content of molybdenum and rhenium and the at least one additional metal in the novel metal alloy is at least about 99.99 weight percent. As can be appreciated, other weight percentages of the content of molybdenum and rhenium and the at least one additional metal in the novel metal alloy can be used. In one non-limiting composition, the purity level of the novel metal alloy is such so as to produce a solid solution of a rhenium and molybdenum and the at least one additional metal. A solid solution of a novel metal alloy that includes rhenium and molybdenum and the at least one additional metal of titanium, yttrium and/or zirconium as the primary metals is an alloy that includes at least about 95-99 weight percent rhenium and molybdenum and the at least one additional metal. It is believed that a purity level of less than 95 weight percent molybdenum and rhenium and the at least one additional metal adversely affects one or more physical properties of the metal alloy that are useful or desired in forming and/or using a medical device. In one embodiment, the rhenium content of the novel metal alloy in accordance with the present invention is at least about 40 weight percent. In one non-limiting composition, the rhenium content of the novel metal alloy is at least about 45 weight percent. In still another and/or alternative non-limiting composition, the rhenium content of the novel metal alloy is about 45-50 weight percent. In yet another and/or alternative non-limiting composition, the rhenium content of the novel metal alloy is about 47-48 weight percent. As can be appreciated, other weight percentages of the rhenium content of the novel metal alloy can be used. In another and/or alternative embodiment, the molybdenum content of the novel metal alloy is at least about 40 weight percent. In one non-limiting composition, the molybdenum content of the novel metal alloy is at least about 45 weight percent. In another and/or alternative non-limiting composition, the molybdenum content of the novel metal alloy is at least about 50 weight percent. In still another and/or alternative non-limiting composition, the molybdenum content of the novel metal alloy is about 50-60 percent. In yet another and/or alternative non-limiting composition, the molybdenum content of the novel metal alloy is about 50-56 weight percent. As can be appreciated, other weight percentages of the molybdenum content of the novel metal alloy can be used. The combined content of titanium, yttrium and zirconium in the novel metal alloy is less than about 5 weight percent, typically no more than about 1 weight percent, and more typically no more than about 0.5 weight percent. A higher weight percent content of titanium, yttrium and/or zirconium in the novel metal alloy can begin to adversely affect the brittleness of the novel metal alloy. When titanium is included in the novel metal alloy, the titanium content is typically less than about 1 weight percent, more typically less than about 0.6 weight percent, even more typically about 0.05-0.5 weight percent, still even more typically about 0.1-0.5 weight percent. As can be appreciated, other weight percentages of the titanium content of the novel metal alloy can be used. When zirconium is included in the novel metal alloy, the zirconium content is typically less than about 0.5 weight percent, more typically less than about 0.3 weight percent, even more typically about 0.01-0.25 weight percent, still even more typically about 0.05-0.25 weight percent. As can be appreciated, other weight percentages of the zirconium content of the novel metal alloy can be used. When titanium and zirconium are included in the novel metal alloy, the weight ratio of titanium to zirconium is about 1-10:1, typically about 1.5-5:1, and more typically about 1.75-2.5:1. When yttrium is included in the novel metal alloy, the yttrium content is typically less than about 0.3 weight percent, more typically less than about 0.2 weight percent, and even more typically about 0.01-0.1 weight percent. As can be appreciated, other weight percentages of the yttrium content of the novel metal alloy can be used. The inclusion of titanium, yttrium and/or zirconium in the novel metal alloy is believed to result in a reduction of oxygen trapped in the solid solution of the novel metal alloy. The reduction of trapped oxygen enables the formation of a smaller grain size in the novel metal alloy and/or an increase in the ductility of the novel metal alloy. The reduction of trapped oxygen in the novel metal alloy can also increase the yield strength of the novel metal alloy as compared to alloys of only molybdenum and rhenium (i.e., 2-10% increase). The inclusion of titanium, yttrium and/or zirconium in the novel metal alloy is also believed to cause a reduction in the trapped free carbon in the novel metal alloy. The inclusion of titanium, yttrium and/or zirconium in the novel metal alloy is believed to form carbides with the free carbon in the novel metal alloy. This carbide formation is also believed to improve the ductility of the novel metal alloy and to also reduce the incidence of cracking during the forming of the metal alloy into a medical device (e.g., stent, etc.). As such, the novel metal alloy exhibits increased tensile elongation as compared to alloys of only molybdenum and rhenium (i.e., 1-8% increase). The inclusion of titanium, yttrium and/or zirconium in the novel metal alloy is also believed to cause a reduction in the trapped free nitrogen in the novel metal alloy. The inclusion of titanium, yttrium and/or zirconium in the novel metal alloy is believed to form carbo-nitrides with the free carbon and tree nitrogen in the novel metal alloy. This carbo-nitride formation is also believed to improve the ductility of the novel metal alloy and to also reduce the incidence of cracking during the forming of the metal alloy into a medical device (e.g., stent, etc.). As such, the novel metal alloy exhibits increased tensile elongation as compared to alloys of only molybdenum and rhenium (i.e., 1-8% increase). The reduction in the amount of free carbon, oxygen and/or nitrogen in the novel metal alloy is also believed to increase the density of the novel metal alloy (i.e., 1-5% increase). The formation of carbides, carbo-nitrides, and/or oxides in the novel metal alloy results in the formation of dispersed second phase particles in the novel metal alloy, thereby facilitating in the formation of small grain sizes in the metal alloy.

In still another and/or alternative non-limiting aspect, the novel metal alloy includes less than about 5 weight percent other metals and/or impurities. A high purity level of the novel metal alloy results in the formation of a more homogeneous alloy, which in turn results in a more uniform density throughout the novel metal alloy, and also results in the desired yield and ultimate tensile strengths of the novel metal alloy. The density of the novel metal alloy is generally at least about 12 gm/cc, and typically at least about 13-13.5 gm/cc. This substantially uniform high density of the novel metal alloy significantly improves the radiopacity of the novel metal alloy. In one non-limiting composition, the novel metal alloy includes less than about 1 weight percent other metals and/or impurities. In another and/or alternative non-limiting composition, the novel metal alloy includes less than about 0.5 weight percent other metals and/or impurities. In still another and/or alternative non-limiting composition, the novel metal alloy includes less than about 0.4 weight percent other metals and/or impurities. In yet another and/or alternative non-limiting composition, the novel metal alloy includes less than about 0.2 weight percent other metals and/or impurities. In still yet another and/or alternative non-limiting composition, the novel metal alloy includes less than about 0.1 weight percent other metals and/or impurities. In still another and/or alternative non-limiting composition, the novel metal alloy includes less than about 0.08 weight percent other metals and/or impurities. In yet another and/or alternative non-limiting composition, the novel metal alloy includes less than about 0.06 weight percent other metals and/or impurities. In a further and/or alternative non-limiting composition, the novel metal alloy includes less than about 0.05 weight percent other metals and/or impurities. In still a further and/or alternative non-limiting composition, the novel metal alloy includes less than about 0.02 weight percent other metals and/or impurities. In yet a further and/or alternative non-limiting composition, the novel metal alloy includes less than about 0.01 weight percent other metals and/or impurities. As can be appreciated, other weight percentages of the amount of other metals and/or impurities in the novel metal alloy can exist.

In yet another and/or alternative non-limiting aspect, the novel metal alloy includes a certain amount of carbon and oxygen. These two elements have been found to affect the forming properties and brittleness of the novel metal alloy. The controlled atomic ratio of carbon and oxygen in the novel metal alloy also can be used to minimize the tendency of the novel metal alloy to form micro-cracks during the forming of the novel metal alloy into a medical device, and/or during the use and/or expansion of the medical device in a body passageway. The control of the atomic ratio of carbon to oxygen in the novel metal alloy allows for the redistribution of oxygen in the metal alloy so as to minimize the tendency of micro-cracking in the novel metal alloy during the forming of the novel metal alloy into a medical device, and/or during the use and/or expansion of the medical device in a body passageway. The atomic ratio of carbon to oxygen in the alloy is believed to be important to minimize the tendency of micro-cracking in the novel metal alloy, improve the degree of elongation of the novel metal alloy, both of which can affect one or more physical properties of the metal alloy that are useful or desired in forming and/or using the medical device. It was previously believed by applicants that a carbon to oxygen atomic ratio of less than about 2:1 would adversely affect the properties of a medical device such as, but not limited to a stent. Upon further investigation, it has been found that a stent when exposed to body temperatures can be formed of the novel metal alloy with a carbon to oxygen atomic ratio that is less than about 2:1; however, it is still believed that the properties of the stent are better when the carbon to oxygen atomic ratio is greater than about 2:1. It is believed that for certain applications of the novel metal alloy when operating in temperatures of about 40-120F (5°C - 50°C) and that the oxygen content is below a certain amount, the carbon to oxygen atomic ratio can be as low as about 0.2:1. In one non-limiting formulation, the carbon to oxygen atomic ratio in the novel metal alloy is generally at least about 0.4:1 (i.e., weight ratio of about 0.3:1). In another non-limiting formulation, the carbon to oxygen atomic ratio in the novel metal alloy is generally at least about 0.5:1 (i.e., weight ratio of about 0.375:1). In still another non-limiting formulation, the carbon to oxygen atomic ratio in the novel metal alloy is generally at least about 1:1 (i.e., weight ratio of about 0.75:1). In yet another non-limiting formulation, the carbon to oxygen atomic ratio in the novel metal alloy is generally at least about 2:1 (i.e., weight ratio of about 1.5:1). In still yet another non-limiting formulation, the carbon to oxygen atomic ratio in the novel metal alloy is generally at least about 2.5:1 (i.e., weight ratio of about 1.88:1). In still another non-limiting formulation, the carbon to oxygen atomic ratio in the novel metal alloy is generally at least about 3:1 (i.e., weight ratio of about 2.25:1). In yet another non-limiting formulation, the carbon to oxygen atomic ratio in the novel metal alloy is generally at least about 4:1 (i.e., weight ratio of about 3:1). In still yet another non-limiting formulation, the carbon to oxygen atomic ratio in the novel metal alloy is generally at least about 5:1 (i.e., weight ratio of about 3.75:1). In still another non-limiting formulation, the carbon to oxygen atomic ratio in the novel metal alloy is generally about 2.5-50:1 (i.e., weight ratio of about 1.88-37.54:1). In a further non-limiting formulation, the carbon to oxygen atomic ratio in the novel metal alloy is generally about 2.5-20:1 (i.e., weight ratio of about 1.88-15:1). In a further non-limiting formulation, the carbon to oxygen atomic ratio in the novel metal alloy is generally about 2.5-13.3:1 (i.e., weight ratio of about 1.88-10:1). In still a further non-limiting formulation, the carbon to oxygen atomic ratio in the novel metal alloy is generally about 2.5-10:1 (i.e., weight ratio of about 1.88-7.5:1). In yet a further non-limiting formulation, the carbon to oxygen atomic ratio in the novel metal alloy is generally about 2.5-5:1 (i.e., weight ratio of about 1.88-3.75:1). As can be appreciated, other atomic ratios of the carbon to oxygen in the novel metal alloy can be used. The carbon to oxygen ratio can be adjusted by intentionally adding carbon to the novel metal alloy until the desired carbon to oxygen ratio is obtained. Typically the carbon content of the novel metal alloy is less than about 0.2 weight percent. Carbon contents that are too large can adversely affect the physical properties of the novel metal alloy. In one non-limiting formulation, the carbon content of the novel metal alloy is less than about 0.1 weight percent of the novel metal alloy. In another non-limiting formulation, the carbon content of the novel metal alloy is less than about 0.05 weight percent of the novel metal alloy. In still another non-limiting formulation, the carbon content of the novel metal alloy is less than about 0.04 weight percent of the novel metal alloy. When carbon is not intentionally added to the novel metal alloy, the novel metal alloy can include up to about 150 ppm carbon, typically up to about 100 ppm carbon, and more typically less than about 50 ppm carbon. The oxygen content of the novel metal alloy can vary depending on the processing parameters used to form the novel metal alloy. Generally, the oxygen content is to be maintained at very low levels. In one non-limiting formulation, the oxygen content is less than about 0.1 weight percent of the novel metal alloy. In another non-limiting formulation ,the oxygen content is less than about 0.05 weight percent of the novel metal alloy. In still another non-limiting formulation ,the oxygen content is less than about 0.04 weight percent of the novel metal alloy. In yet another non-limiting formulation ,the oxygen content is less than about 0.03 weight percent of the novel metal alloy. In still yet another non-limiting formulation, the novel metal alloy includes up to about 100 ppm oxygen. In a further non-limiting formulation, the novel metal alloy includes up to about 75 ppm oxygen. In still a further non-limiting formulation, the novel metal alloy includes up to about 50 ppm oxygen. In yet a further non-limiting formulation, the novel metal alloy includes up to about 30 ppm oxygen. In still yet a further non-limiting formulation, the novel metal alloy includes less than about 20 ppm oxygen. In yet a further non-limiting formulation, the novel metal alloy includes less than about 10 ppm oxygen. As can be appreciated, other amounts of carbon and/or oxygen in the novel metal alloy can exist. It is believed that the novel metal alloy will have a very low tendency to form micro-cracks during the formation of the medical device (e.g., stent, etc.) and after the medical device has been inserted into a patient by closely controlling the carbon to oxygen ration when the oxygen content exceed a certain amount in the novel metal alloy. In one non-limiting arrangement, the carbon to oxygen atomic ratio in the novel metal alloy is at least about 2.5:1 when the oxygen content is greater than about 100 ppm in the novel metal alloy.

In still yet another and/or alternative non-limiting aspect, the novel metal alloy includes a controlled amount of nitrogen. Large amounts of nitrogen in the novel metal alloy can adversely affect the ductility of the novel metal alloy. This can in turn adversely affect the elongation properties of the novel metal alloy. A too high of nitrogen content in the novel metal alloy can begin to cause the ductility of the novel metal alloy to unacceptably decrease, thus adversely affect one or more physical properties of the metal alloy that are useful or desired in forming and/or using the medical device. In one non-limiting formulation, the novel metal alloy includes less than about 0.001 weight percent nitrogen. In another non-limiting formulation, the novel metal alloy includes less than about 0.0008 weight percent nitrogen. In still another non-limiting formulation, the novel metal alloy includes less than about 0.0004 weight percent nitrogen. In yet another non-limiting formulation, the novel metal alloy includes less than about 30 ppm nitrogen. In still yet another non-limiting formulation, the novel metal alloy includes less than about 25 ppm nitrogen. In still another non-limiting formulation, the novel metal alloy includes less than about 10 ppm nitrogen. In yet another non-limiting formulation, the novel metal alloy includes less than about 5 ppm nitrogen. As can be appreciated, other amounts of nitrogen in the novel metal alloy can exist. The relationship of carbon, oxygen and nitrogen in the novel metal alloy is also believed to be important. It is believed that the nitrogen content should be less than the content of carbon or oxygen in the novel metal alloy. In one non-limiting formulation, the atomic ratio of carbon to nitrogen is at least about 2:1 (i.e., weight ratio of about 1.71:1). In another non-limiting formulation, the atomic ratio of carbon to nitrogen is at least about 3:1 (i.e., weight ratio of about 2.57:1). In still another non-limiting formulation, the atomic ratio of carbon to nitrogen is about 4-100:1 (i.e., weight ratio of about 3.43-85.7:1). In yet another non-limiting formulation, the atomic ratio of carbon to nitrogen is about 4-75:1 (i.e., weight ratio of about 3.43-64.3:1). In still another non-limiting formulation, the atomic ratio of carbon to nitrogen is about 4-50:1 (i.e., weight ratio of about 3.43-42.85:1). In yet another non-limiting formulation, the atomic ratio of carbon to nitrogen is about 4-35:1 (i.e., weight ratio of about 3.43-30:1). In still yet another non-limiting formulation, the atomic ratio of carbon to nitrogen is about 4-25:1 (i.e., weight ratio of about 3.43-21.43:1). In a further non-limiting formulation, the atomic ratio of oxygen to nitrogen is at least about 1.2:1 (i.e., weight ratio of about 1.37:1). In another non-limiting formulation, the atomic ratio of oxygen to nitrogen is at least about 2:1 (i.e., weight ratio of about 2.28:1). In still another non-limiting formulation, the atomic ratio of oxygen to nitrogen is about 3-100:1 (i.e., weight ratio of about 3.42-114.2:1). In yet another non-limiting formulation, the atomic ratio of oxygen to nitrogen is at least about 3-75:1 (i.e., weight ratio of about 3.42-85.65:1). In still yet another non-limiting formulation, the atomic ratio of oxygen to nitrogen is at least about 3-55:1 (i.e., weight ratio of about 3.42-62.81:1). In yet another non-limiting formulation, the atomic ratio of oxygen to nitrogen is at least about 3-50:1 (i.e., weight ratio of about 3.42-57.1:1).

In a further and/or alternative non-limiting aspect, the novel metal alloy has several physical properties that positively affect the medical device when at least partially formed of the novel metal alloy. In one non-limiting embodiment, the average hardness of the novel metal alloy tube used to form the medical device is generally at least about 60 (HRC) at 77F (25°C). In one non-limiting aspect of this embodiment, the average hardness of the novel metal alloy tube used to form the medical device is generally at least about 70 (HRC) at 77F (25°C), and typically about 80-100 (HRC) at 77F (25°C). In another and/or alternative non-limiting embodiment, the average ultimate tensile strength of the novel metal alloy used to form the medical device is generally at least about 60 UTS (ksi). [Here and in the following, 1 ksi corresponds to 6.894757293178 newton/millimeter²] In non-limiting aspect of this embodiment, the average ultimate tensile strength of the novel metal alloy used to form the medical device is generally at least about 70 UTS (ksi); typically about 80-150 UTS (ksi), and more typically about 100-150 UTS (ksi). In still another and/or alternative non-limiting embodiment, the average yield strength of the novel metal alloy used to form the medical device is at least about 70 ksi. In one non-limiting aspect of this embodiment, the average yield strength of the novel metal alloy used to form the medical device is at least about 80 ksi, and typically about 100-140 (ksi). In yet another and/or alternative non-limiting embodiment, the average grain size of the novel metal alloy used to form the medical device is greater than 5 ASTM (e.g., ASTM E 112-96). The small grain size of the novel metal alloy enables the medical device to have the desired elongation and ductility properties that are useful in enabling the medical device to be formed, crimped and/or expanded. In one non-limiting aspect of this embodiment, the average grain size of the novel metal alloy used to form the medical device is about 5.2-10 ASTM, typically, about 5.5-9 ASTM, more typically about 6-9 ASTM, still more typically about 6-8 ASTM, even more typically, about 6-7 ASTM, and still even more typically about 6.5-7 ASTM. In still yet another and/or alternative non-limiting embodiment, the average tensile elongation of the novel metal alloy used to form the medical device is at least about 25%. An average tensile elongation of at least 25% for the novel metal alloy is important to enable the medical device to be properly expanded when positioned in the treatment area of a body passageway. A medical device that does not have an average tensile elongation of at least about 25% can form micro-cracks and/or break during the forming, crimping and/or expansion of the medical device. In one non-limiting aspect of this embodiment, the average tensile elongation of the novel metal alloy used to form the medical device is about 25-35%. The unique combination of the rhenium content in the novel metal alloy in combination with achieving the desired purity and composition of the alloy and the desired grain size of the novel metal alloy results in 1) a medical device having the desired high ductility at about room temperature, 2) a medical device having the desired amount of tensile elongation, 3) a homogeneous or solid solution of a metal alloy having high radiopacity, 4) a reduction or prevention of microcrack formation and/or breaking of the metal alloy tube when the metal alloy tube is sized and/or cut to form the medical device, 5) a reduction or prevention of microcrack formation and/or breaking of the medical device when the medical device is crimped onto a balloon and/or other type of medical device for insertion into a body passageway, 6) a reduction or prevention of microcrack formation and/or breaking of the medical device when the medical device is bent and/or expanded in a body passageway, 7) a medical device having the desired ultimate tensile strength and yield strength, 8) a medical device that can have very thin wall thicknesses and still have the desired radial forces needed to retain the body passageway on an open state when the medical device has been expanded, and/or 9) a medical device that exhibits less recoil when the medical device is crimped onto a delivery system and/or expanded in a body passageway.

Several non-limiting examples of the novel metal alloy in accordance with the present invention are set forth below:

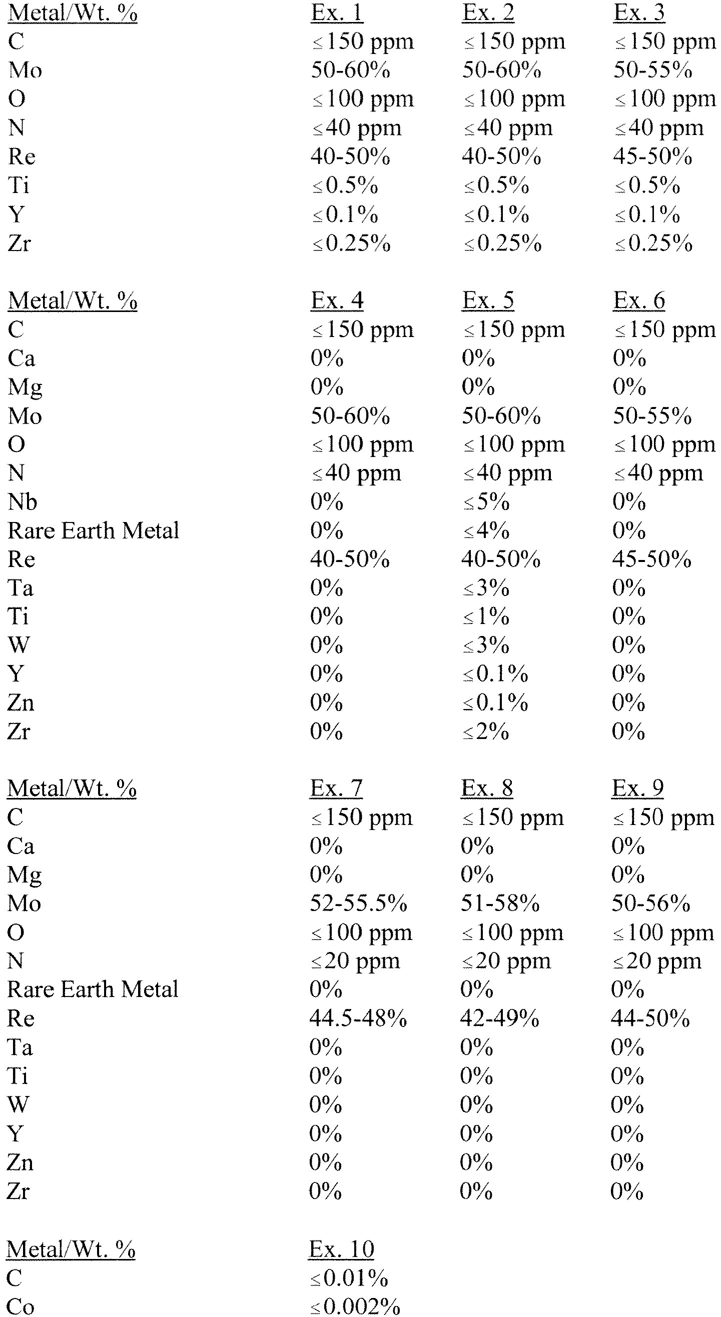

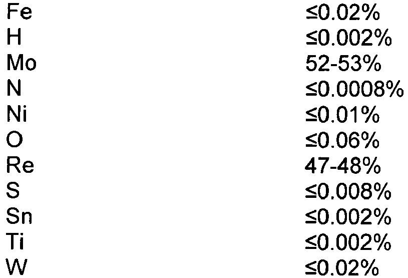

In examples 1-10 above, the novel metal alloy is principally formed of rhenium and molybdenum, in a solid solution or homogenous solution or homogenous solution including rhenium and molybdenum in at least 95 wt%, or in a solution including rhenium and molybdenum at about 95 - 99 wt%. The novel metal alloy may also include controlled amounts of titanium, yttrium and/or zirconium. The content of other metals and/or impurities is less than about 0.2 weight percent of the novel metal alloy. In examples 1-9 above, the ratio of carbon to oxygen is at least about 2.5:1 (i.e., weight ratio of carbon to oxygen of at least about 1.88:1). In example 10, the ratio of carbon to oxygen is at least about 0.4:1 (i.e., weight ratio of carbon to oxygen of at least about 0.3:1). In examples 1-10, the nitrogen content is less than the carbon content and the oxygen content. In examples 1-10, the atomic ratio of carbon to nitrogen is at least about 4:1 (i.e., weight ratio of about 3.43:1). In examples 1-10, the atomic ratio of oxygen to nitrogen is at least about 3:1 (i.e., weight ratio of about 3.42:1). In examples 1-10, the average grain size of novel metal alloy is about 6-10 ASTM, the tensile elongation of the metal alloy is about 25-35%, the average density of the metal alloy is at least about 13.4 gm/cc, the average yield strength of the metal alloy is about 98-122 (ksi), the average ultimate tensile strength of the metal alloy is about 100-150 UTS (ksi), and the average hardness of the metal alloy is about 80-100 (HRC) at 77F (25°C).

Additional non-limiting examples of the novel metal alloy in accordance with the present invention are set forth below:

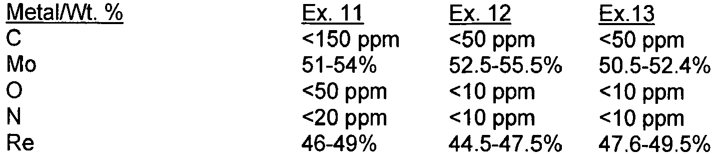

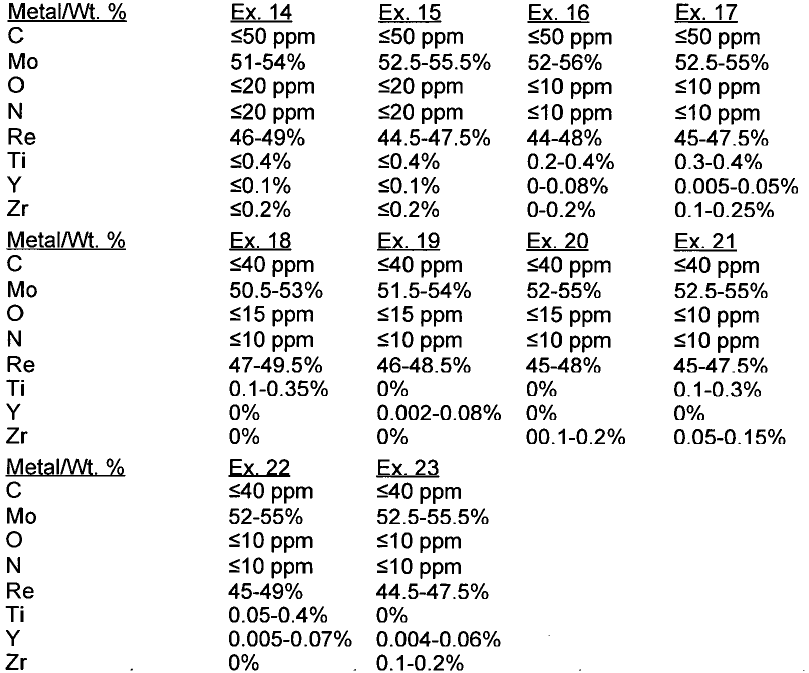

In examples 14-23 above, the novel metal alloy is principally formed of rhenium and molybdenum and at least one metal of titanium, yttrium and/or zirconium, and the content of other metals and/or impurities is less than about 0.1 weight percent of the novel metal alloy, the atomic ratio of carbon to oxygen is about 2.5-10:1, the atomic ratio of carbon to nitrogen is at least about 4:1, the atomic ratio of oxygen to nitrogen is at least about 3:1, the average grain size of the novel metal alloy is about 6-9 ASTM, the tensile elongation of the metal alloy is about 25-35%, the average density of the metal alloy is at least about 13.6 gm/cc, the average yield strength of the metal alloy is at least about 110 (ksi), the average ultimate tensile strength of the metal alloy is about 100-150 UTS (ksi), and the average hardness of the metal alloy is about 80-100 (HRC) at 77F (25°C).

In another and/or alternative non-limiting aspect, the use of novel metal alloy in the medical device can increase the strength of the medical device as compared with stainless steel or chromium-cobalt alloys, thus less quantity of novel metal alloy can be used in the medical device to achieve similar strengths as compared to medical devices formed of different metals. As such, the resulting medical device can be made smaller and less bulky by use of the novel metal alloy without sacrificing the strength and durability of the medical device. Such a medical device can have a smaller profile, thus can be inserted in smaller areas, openings and/or passageways. The novel metal alloy also can increase the radial strength of the medical device. For instance, the thickness of the walls of the medical device and/or the wires used to form the medical device can be made thinner and achieve a similar or improved radial strength as compared with thicker walled medical devices formed of stainless steel or cobalt and chromium alloy. The novel metal alloy also can improve stress-strain properties, bendability and flexibility of the medical device, thus increase the life of the medical device. For instance, the medical device can be used in regions that subject the medical device to bending. Due to the improved physical properties of the medical device from the novel metal alloy, the medical device has improved resistance to fracturing in such frequent bending environments. In addition or alternatively, the improved bendability and flexibility of the medical device due to the use of the novel metal alloy can enable the medical device to be more easily inserted into a body passageway. The novel metal alloy can also reduce the degree of recoil during the crimping and/or expansion of the medical device. For example, the medical device better maintains its crimped form and/or better maintains its expanded form after expansion due to the use of the novel metal alloy. As such, when the medical device is to be mounted onto a delivery device when the medical device is crimped, the medical device better maintains its smaller profile during the insertion of the medical device in a body passageway. Also, the medical device better maintains its expanded profile after expansion so as to facilitate in the success of the medical device in the treatment area. In addition to the improved physical properties of the medical device by use of the novel metal alloy, the novel metal alloy has improved radiopaque properties as compared to standard materials such as stainless steel or cobalt-chromium alloy, thus reducing or eliminating the need for using marker materials on the medical device. For instance, the novel metal alloy is at least about 10-20% more radiopaque than stainless steel or cobalt-chromium alloy. Specifically, the novel metal alloy can be at least about 33% more radiopaque than cobalt-chromium alloy and at least about 41.5% more radiopaque than stainless steel.

In still yet another and/or alternative non-limiting aspect, the medical device that is at least partially formed from the novel metal alloy can be formed by a variety of manufacturing techniques. In one non-limiting embodiment, the medical device can be formed from a rod or tube of the novel metal alloy. If a solid rod of the novel metal alloy is formed, the rod can be cut or drilled (e.g., gun drilled, EDM, etc.) to form a cavity or passageway partially or fully through the rod. The rod or tube can be cleaned, polished, annealed, drawn, etc. to obtain the desired cross-sectional area or diameter and/or wall thickness of the metal tube. After the metal tube has been formed to the desired cross-sectional area or diameter and wall thickness, the metal tube can be formed into a medical device by a process such as, but not limited to, laser cutting, etching, etc. After the medical device has been formed, the medical device can be cleaned, polished, sterilized, etc. for final processing of the medical device. As can be appreciated, other or additional process steps can be used to at least partially form the medical device from the novel metal alloy.

In a further and/or alternative non-limiting aspect, the novel alloy used to at least partially form the medical device is initially formed into a rod or a tube of novel metal alloy. The novel metal alloy rod or tube can be formed by various techniques such as, but not limited to, 1) melting the novel metal alloy and/or metals that form the novel metal alloy (e.g., vacuum arc melting, etc.) and then extruding and/or casting the novel metal alloy into a rod or tube, 2) melting the novel metal alloy and/or metals that form the novel metal alloy, forming a metal strip and then rolling and welding the strip into a tube, or 3) consolidating metal power of the novel metal alloy and/or metal powder of metals that form the novel metal alloy. The rod or tube, however formed, generally has a length of about 48 inches or less; however, longer lengths can be formed. The average outer diameter of the rod or tube is generally less than about 2 inches [here and in the following 1 inch corresponds to 2.54 cm] (i.e., less than about 3.14 sq. in. cross-sectional area), more typically less than about 1 inch outer diameter, and even more typically no more than about 0.5 inch outer diameter; however, larger rod or tube diameter sizes can be formed. In one non-limiting configuration for a tube, the tube has an inner diameter of about 0.31 inch plus or minus about 0.002 inch and an outer diameter of about 0.5 inch plus or minus about 0.002 inch. The wall thickness of the tube is about 0.095 inch plus or minus about 0.002 inch. As can be appreciated, this is just one example of many different sized tubes that can be formed. In one non-limiting process, the rod or tube can be formed from one or more ingots of metal or metal alloy. In one non-limiting process, an arc melting process (e.g., vacuum arc melting process, etc.) can be used to form the one or more ingots. In another non-limiting process, rhenium powder and molybdenum powder can be placed in a crucible (e.g., silica crucible, etc.) and heated under a controlled atmosphere (e.g., vacuum environment, carbon monoxide environment, hydrogen and argon environment, helium, argon, etc.) by an induction melting furnace. It can be appreciated that other or additional processes can be used to form the one or more ingots. Once the ingots are formed, the metal ingots can be cast, extruded through a die, etc. to form the rod or tube. During an extrusion process, the ingots are generally heated; however, this is not required. A close-fitting rod can be used during the extrusion process to form the tube; however, this is not required. In another and/or additional non-limiting process, the tube of the novel metal alloy can be formed from a strip or sheet of novel metal alloy. The strip or sheet of novel metal alloy can be formed into a tube by rolling the edges of the sheet or strip and then welding together the edges of the sheet or strip. The welding of the edges of the sheet or strip can be accomplished in several ways such as, but not limited to, a) holding the edges together and then e-beam welding the edges together in a vacuum, b) positioning a thin strip of novel metal alloy above and/or below the edges of the rolled strip or sheet to be welded, then welding the one or more strips along the rolled strip or sheet edges, and then grinding off the outer strip, or c) laser welding the edges of the rolled sheet or strip in a vacuum, oxygen reducing atmosphere, or inert atmosphere. In still another and/or additional non-limiting process, the rod or tube of the novel metal alloy is formed by consolidating metal power. In this process, fine particles of molybdenum and rhenium along with any additives are mixed to form a homogenous blend of particles. Typically the average particle size of the metal powders is less than about 200 mesh (e.g., less than 74 microns). A larger average particle size can interfere with the proper mixing of the metal powders and/or adversely affect one or more physical properties of the rod or tube formed from the metal powders. In one non-limiting embodiment, the average particle size of the metal powders is less than about 230 mesh (e.g., less than 63 microns). In another and/or alternative non-limiting embodiment, the average particle size of the metal powders is about 2-63 microns, and more particularly about 5-40 microns. As can be appreciated, smaller average particle sizes can be used. The purity of the metal powders should be selected so that the metal powders contain very low levels of carbon, oxygen and nitrogen. Typically the carbon content of the molybdenum metal powder is less than about 100 ppm, the oxygen content of the molybdenum metal powder is less than about 50 ppm, and the nitrogen content of the molybdenum metal powder is less than about 20 ppm. Typically, the carbon content of the rhenium metal powder is less than about 100 ppm, the oxygen content of the rhenium metal powder is less than about 50 ppm, and the nitrogen content of the rhenium metal powder is less than about 20 ppm. Typically, metal powder having a purity grade of at least 99.9 and more typically at least about 99.95 should be used to obtain the desired purity of the powders of molybdenum and rhenium. When titanium, yttrium and/or zirconium powder is added to the metal powder mixture, the amount of carbon, oxygen and nitrogen in the power should also be minimized. Typically, metal powder having a purity grade of at least 99.8 and more typically at least about 99.9 should be used to obtain the desired purity of the powders of titanium, yttrium and/or zirconium. Carbon can be intentionally added to obtain a certain carbon to oxygen atomic ratio in the novel metal alloy; however, this is not required. The blend of metal powder is then pressed together to form a solid solution of the novel metal alloy into a rod or tube. Typically the pressing process is by an isostatic process(i.e., uniform pressure applied from all sides on the metal powder). When the metal powders are pressed together isostatically, cold isostatic pressing (CIP) is typically used to consolidate the metal powders; however, this is not required. The pressing process can be preformed in an inert atmosphere, an oxygen reducing atmosphere (e.g., hydrogen, argon and hydrogen mixture, etc.) and/or under a vacuum; however, this might not be required. The average density of the rod or tube that is achieved by pressing together the metal powders is about 80-90% of the final average density of the rod or tube or about 70-96% the minimum theoretical density of the novel metal alloy. Pressing pressures of at least about 300 MPa are generally used. Generally the pressing pressure is about 400-700MPa; however, other pressures can be used. After the metal powders are pressed together, the pressed metal powders are sintered at high temperature (e.g., 2000-2900°C) to fuse the metal powders together to form the solid metal rod or tube. The sintering of the consolidated metal powder can be preformed in an oxygen reducing atmosphere (e.g., helium, argon, hydrogen, argon and hydrogen mixture, etc.) and/or under a vacuum; however, this might not be required. At the high sintering temperatures, a high hydrogen atmosphere will reduce both the amount of carbon and oxygen in the formed rod or tube. The sintered metal powder generally has an as-sintered average density of about 90-99% the minimum theoretical density of the novel metal alloy. Typically, the sintered rod or tube has a final average density of at least about 12 gm/cc, typically at least about 12.5 gm/cc, and more typically about 13-14 gm/cc. A rod or tube formed by compressed and sintered metal powders typically has an average concentricity deviation that is less than a rod or tube formed by an arc melting and molding process, extrusion process, or a sheet and welding process; however, this is not always the situation. Generally, the average concentricity deviation of the rod or tube that is formed from compressed and sintered metal powders is less than about 20%, typically about 1-18%, and more typically about 1-5%.

In still a further and/or alternative non-limiting aspect, when a solid rod of the novel metal alloy is formed, the rod is then formed into a tube prior to reducing the outer cross-sectional area or diameter of the rod. The rod can be formed into a tube by a variety of processes such as, but not limited to, cutting or drilling (e.g., gun drilling, etc.) or by cutting (e.g., EDM, etc.). The cavity or passageway formed in the rod typically is formed fully through the rod; however, this is not required.

In yet a further and/or alternative non-limiting aspect, the rod or tube can be cleaned and/or polished after the rod or tube has been form; however, this is not required. Typically the rod or tube is cleaned and/or polished prior to being further processed; however, this is not required. When a rod of the novel metal alloy is formed into a tube, the formed tube is typically cleaned and/or polished prior to being further process; however, this is not required. When the rod or tube is resized and/or annealed as discussed in detail below, the resized and/or annealed rod or tube is typically cleaned and/or polished prior to and/or after each or after a series of resizing and/or annealing processes; however, this is not required. The cleaning and/or polishing of the rod or tube is used to remove impurities and/or contaminants from the surfaces of the rod or tube. Impurities and contaminants can become incorporated into the novel metal alloy during the processing of the rod or tube. The inadvertent incorporation of impurities and contaminants in the rod or tube can result in an undesired amount of carbon, nitrogen and/or oxygen, and/or other impurities in the novel metal alloy. The inclusion of impurities and contaminants in the novel metal alloy can result in premature micro-cracking of the novel metal alloy and/or an adverse affect on one or more physical properties of the novel metal alloy (e.g., decrease in tensile elongation, increased ductility, etc.). The cleaning of the novel metal alloy can be accomplished by a variety of techniques such as, but not limited to, 1) using a solvent (e.g., acetone, methyl alcohol, etc.) and wiping the novel metal alloy with a Kimwipe or other appropriate towel, 2) by at least partially dipping or immersing the novel metal alloy in a solvent and then ultrasonically cleaning the novel metal alloy, and/or 3) by at least partially dipping or immersing the novel metal alloy in a pickling solution. As can be appreciated, the novel metal alloy can be cleaned in other or additional ways. If the novel metal alloy is to be polished, the novel metal alloy is generally polished by use of a polishing solution that typically includes an acid solution; however, this is not required. In one non-limiting example, the polishing solution includes sulfuric acid; however, other or additional acids can be used. In one non-limiting polishing solution, the polishing solution can include by volume 60-95% sulfuric acid and 5-40% de-ionized water (DI water). Typically, the polishing solution that includes an acid will increase in temperature during the making of the solution and/or during the polishing procedure. As such, the polishing solution is typically stirred and/or cooled during making of the solution and/or during the polishing procedure. The temperature of the polishing solution is typically about 20-100°C, and typically greater than about 25°C. One non-limiting polishing technique that can be used is an electro-polishing technique. When an electro-polishing technique is used, a voltage of about 2-30V, and typically about 5-12V is applied to the rod or tube during the polishing process; however, it will be appreciated that other voltages can be used. The time used to polish the novel metal alloy is dependent on both the size of the rod or tube and the amount of material that needs to be removed from the rod or tube. The rod or tube can be processed by use of a two-step polishing process wherein the novel metal alloy piece is at least partially immersed in the polishing solution for a given period (e.g., 0.1-15 minutes, etc.), rinsed (e.g., DI water, etc.) for a short period of time (e.g., 0.02-1 minute, etc.), and then flipped over and at least partially immersed in the solution again for the same or similar duration as the first time; however, this is not required. The novel metal alloy can be rinsed (e.g., DI water, etc.) for a period of time (e.g., 0.01-5 minutes, etc.) before rinsing with a solvent (e.g., acetone, methyl alcohol, etc.); however, this is not required. The novel metal alloy can be dried (e.g., exposure to the atmosphere, maintained in an inert gas environment, etc.) on a clean surface. These polishing procedures can be repeated until the desired amount of polishing of the rod or tube is achieved. The rod or tube can be uniformly electropolished or selectively electropolished. When the rod or tube is selectively electropolished, the selective electropolishing can be used to obtain different surface characteristics of the rod or tube and/or selectively expose one or more regions of the rod or tube; however, this is not required.

In still yet a further and/or alternative non-limiting aspect, the rod or tube is resized to the desired dimension of the medical device. In one non-limiting embodiment, the cross-sectional area or diameter of the rod or tube is reduced to a final rod or tube dimension in a single step or by a series of steps. The reduction of the outer cross-sectional area or diameter of the rod may be obtained by either centerless grinding, turning, electropolishing, drawing process etc. During the reduction the tube, the outer tube cross-sectional area or diameter, the inner tube cross-sectional area or diameter and/or wall thickness of the tube are typically reduced; however, this is not required. The outer cross-sectional area or diameter size of the rod or tube is typically reduced by the use of one or more drawing processes. During the drawing process, care should be taken to not form micro-cracks in the rod or tube during the reduction of the rod or tube outer cross-sectional area or diameter. Generally, the rod or tube should not be reduced in cross-sectional area by more about 25% each time the rod or tube is drawn through a reducing mechanism (e.g., a die, etc.). In one non-limiting process step, the rod or tube is reduced in cross-sectional area by about 0.1-20% each time the rod or tube is drawn through a reducing mechanism. In another and/or alternative non-limiting process step, the rod or tube is reduced in cross-sectional area by about 1-15% each time the rod or tube is drawn through a reducing mechanism. In still another and/or alternative non-limiting process step, the rod or tube is reduced in cross-sectional area by about 2-15% each time the rod or tube is drawn through reducing mechanism. In yet another one non-limiting process step, the rod or tube is reduced in cross-sectional area by about 5-10% each time the rod or tube is drawn through reducing mechanism. In another and/or alternative non-limiting embodiment of the invention, the rod or tube of novel metal alloy is drawn through a die to reduce the cross-sectional area of the rod or tube. The tube drawing process is typically a cold drawing process or a plug drawing process through a die. When a cold drawing or mandrel drawing process is used, a lubricant (e.g., molybdenum paste, grease, etc.) is typically coated on the outer surface of the tube and the tube is then drawn though the die. Typically, little or no heat is used during the cold drawing process. After the tube has been drawn through the die, the outer surface of the tube is typically cleaned with a solvent to remove the lubricant so as to limit the amount of impurities that are incorporated in the novel metal alloy. This cold drawing process can be repeated several times until the desired outer cross-sectional area or diameter, inner cross-sectional area or diameter and/or wall thickness of the tube is achieved. A plug drawing process can also or alternatively be used to size the tube. The plug drawing process typically does not use a lubricant during the drawing process. The plug drawing process typically includes a heating step to heat the tube prior and/or during the drawing of the tube through the die. The elimination of the use of a lubricant can reduce the incidence of impurities being introduced into the metal alloy during the drawing process. During the plug drawing process, the tube can be protected from oxygen by use of a vacuum environment, a non-oxygen environment (e.g., hydrogen, argon and hydrogen mixture, nitrogen, nitrogen and hydrogen, etc.) or an inert environment. One non-limiting protective environment includes argon, hydrogen or argon and hydrogen; however, other or additional inert gasses can be used. As indicated above, the rod or tube is typically cleaned after each drawing process to remove impurities and/or other undesired materials from the surface of the rod or tube; however, this is not required. Typically the rod or tube should be shielded from oxygen and nitrogen when the temperature of the rod or tube is increased to above 500°C, and typically above 450°C, and more typically above 400°C. When the rod or tube is heated to temperatures above about 400-500 °C, the rod or tube has a tendency to begin form nitrides and/or oxides in the presence of nitrogen and oxygen. In these higher temperature environments, a hydrogen environment, argon and hydrogen environment, etc. is generally used. When the rod or tube is drawn at temperatures below 400-500°C, the tube can be exposed to air with little or no adverse affects; however, an inert or slightly reducing environment is generally more desirable.

In still a further and/or alternative non-limiting aspect, the rod or tube during the drawing process can be nitrided. The nitride layer on the rod or tube can function as a lubricating surface during the drawing process to facilitate in the drawing of the rod or tube. The rod or tube is generally nitrided in the presence of nitrogen or a nitrogen mixture (e.g., 97% N-3%H, etc.) for at least about 1 minute at a temperature of at least about 400°C. In one-limiting nitriding process, the rod or tube is heated in the presence of nitrogen or a nitrogen-hydrogen mixture to a temperature of about 400-800°C for about 1-30 minutes. In one non-limiting embodiment, the surface of the rod or tube is nitrided prior to at least one drawing step for the rod or tube. In one non-limiting aspect of this embodiment, the surface of the rod or tube is nitrided prior to a plurality of drawing steps. In another non-limiting aspect of this invention, after the rod or tube has been annealed, the rod or tube is nitrided prior to being drawn. In another and/or alternative non-limiting embodiment, the rod or tube is cleaned to remove nitride compounds on the surface of the rod or tube prior to annealing the rod to tube. The nitride compounds can be removed by a variety of steps such as, but not limited to, and grit blasting, polishing, etc. After the rod or tube has been annealed, the rod or tube can be again nitrided prior to one or more drawing steps; however, this is not required. As can be appreciated, the complete outer surface of the tube can be nitrided or a portion of the outer surface of the tube can be nitrided. Nitriding only selected portions of the outer surface of the tube can be used to obtain different surface characteristics of the tube; however, this is not required.

In still yet a further and/or alternative non-limiting aspect, the rod or tube is annealed after one or more drawing processes. The metal alloy rod or tube can be annealed after each drawing process or after a plurality of drawing processes. The metal alloy rod or tube is typically annealed prior to about a 60% cross-sectional area size reduction of the metal alloy rod or tube. In other words, the rod or tube should not be reduced in cross-sectional area by more than 60% before being annealed. A too large of a reduction in the cross-sectional area of the metal alloy rod or tube during the drawing process prior to the rod or tube being annealed can result in micro-cracking of the rod or tube. In one non-limiting processing step, the metal alloy rod or tube is annealed prior to about a 50% cross-sectional area size reduction of the metal alloy rod or tube. In another and/or alternative non-limiting processing step, the metal alloy rod or tube is annealed prior to about a 45% cross-sectional area size reduction of the metal alloy rod or tube. In still another and/or alternative non-limiting processing step, the metal alloy rod or tube is annealed prior to about a 1-45% cross-sectional area size reduction of the metal alloy rod or tube. In yet another and/or alternative non-limiting processing step, the metal alloy rod or tube is annealed prior to about a 5-30% cross-sectional area size reduction of the metal alloy rod or tube. In still yet another and/or alternative non-limiting processing step, the metal alloy rod or tube is annealed prior to about a 5-15% cross-sectional area size reduction of the metal alloy rod or tube. When the rod or tube is annealed, the rod or tube is typically heated to a temperature of about 1200-1700°C for a period of about 2-200 minutes; however, other temperatures and/or times can be used. In one non-limiting processing step, the metal alloy rod or tube is annealed at a temperature of about 1400-1600 °C for about 2-100 minutes. The annealing process typically occurs in an inert environment or an oxygen reducing environment so as to limit the amount of impurities that may embed themselves in the novel metal alloy during the annealing process. One non-limiting oxygen reducing environment that can be used during the annealing process is a hydrogen environment; however, it can be appreciated that a vacuum environment can be used or one or more other or additional gasses can be used to create the oxygen reducing environment. At the annealing temperatures, a hydrogen containing atmosphere can further reduce the amount of oxygen in the rod or tube. The chamber in which the rod or tube is annealed should be substantially free of impurities (e.g., carbon, oxygen, and/or nitrogen) so as to limit the amount of impurities that can embed themselves in the rod or tube during the annealing process. The annealing chamber typically is formed of a material that will not impart impurities to the rod or tube as the rod or tube is being annealed. A non-limiting material that can be used to form the annealing chamber includes, but is not limited to, molybdenum, rhenium, tungsten, molybdenum TZM alloy, ceramic, etc. When the rod or tube is restrained in the annealing chamber, the restraining apparatuses that are used to contact the novel metal alloy rod or tube are typically formed of materials that will not introduce impurities to the novel metal alloy during the processing of the rod or tube. Non-limiting examples of materials that can be used to at least partially form the restraining apparatuses include, but are not limited to, molybdenum, titanium, yttrium, zirconium, rhenium and/or tungsten. In still another and/or alternative non-limiting processing step, the parameters for annealing can be changed as the tube as the cross-sectional area or diameter; and/or wall thickness of the tube are changed. It has been found that good grain size characteristics of the tube can be achieved when the annealing parameters are varied as the parameters of the tube change. In one non-limiting processing arrangement, the annealing temperature of the tube having a wall thickness of greater than about 0.015 inch is generally at least about 1480°C for a time period of at least about 5 minutes. In another non-limiting processing arrangement, the annealing temperature of the tube having a wall thickness of about 0.008-0.015 inch is generally about 1450-1480°C for a time period of at least about 5 minutes. In another non-limiting processing arrangement, the annealing temperature of the tube having a wall thickness of less than about 0.008 inch is generally less than about 1450°C for a time period of at least about 5 minutes. As such, as the wall thickness is reduced, the annealing temperature is correspondingly reduced; however, the times for annealing can be increased. As can be appreciated, the annealing temperatures of the tube can be decreased as the wall thickness decreases, but the annealing times can remain the same or also be reduced as the wall thickness reduces. After each annealing process, the grain size of the metal in the tube should be no greater than 5 ASTM. Grain sizes of 7-14 ASTM can be achieved by the annealing process of the present invention. It is believed that as the annealing temperature is reduced as the wall thickness reduces, small grain sizes can be obtained. The grain size of the metal in the tube should be as uniform as possible. In addition, the sigma phase of the metal in the tube should be as reduced as much as possible. The sigma phase is a spherical, elliptical or tetragonal crystalline shape in the metal alloy. The sigma phase is commonly formed of both rhenium and molybdenum, typically with a larger concentration of rhenium. After the final drawing of the tube, a final annealing of the tube can be done for final strengthening of the tube; however, this is not required. This final annealing process, when used, generally occurs at a temperature of about 1300-1600°C for at least about 5 minutes; however, other temperatures and/or time periods can be used.

In another and/or alternative non-limiting aspect, the rod or tube can be cleaned prior to and/or after being annealed. The cleaning process is designed to remove impurities, lubricants (e.g., nitride compounds, molybdenum paste, grease, etc.) and/or other materials from the surfaces of the rod or tube. Impurities that are on one or more surfaces of the rod or tube can become permanently embedded into the rod or tube during the annealing processes. These imbedded impurities can adversely affect the physical properties of the novel metal alloy as the rod or tube is formed into a medical device, and/or can adversely affect the operation and/or life of the medical device. In one non-limiting embodiment, the cleaning process includes a delubrication or degreasing process which is typically followed by pickling process; however, this is not required. The delubrication or degreasing process followed by pickling process are typically used when a lubricant has been used on the rod or tube during a drawing process. Lubricants commonly include carbon compounds, nitride compounds, molybdenum paste, and other types of compounds that can adversely affect the novel metal alloy if such compounds and/or elements in such compounds become associated and/or embedded with the novel metal alloy during an annealing process. The delubrication or degreasing process can be accomplished by a variety of techniques such as, but not limited to, 1) using a solvent (e.g., acetone, methyl alcohol, etc.) and wiping the novel metal alloy with a Kimwipe or other appropriate towel, 2) by at least partially dipping or immersing the novel metal alloy in a solvent and then ultrasonically cleaning the novel metal alloy, 3) sand blasting the novel metal alloy, and/or 4) chemical etching the metal alloy. As can be appreciated, the novel metal alloy can be delubricated or degreased in other or additional ways. After the novel metal alloy rod or tube has been delubricated or degreased, the rod or tube can be further cleaned by use of a pickling process; however, this is not required. The pickling process, when used, includes the use of one or more acids to remove impurities from the surface of the rod or tube. Non-limiting examples of acids that can be used as the pickling solution include, but are not limited to, nitric acid, acetic acid, sulfuric acid, hydrochloric acid, and/or hydrofluoric acid. These acids are typically analytical reagent (ACS) grade acids. The acid solution and acid concentration are selected to remove oxides and other impurities on the rod or tube surface without damaging or over etching the surface of the rod or tube. A rod or tube surface that includes a large amount of oxides and/or nitrides typically requires a stronger pickling solution and/or long picking process times. Non-limiting examples of pickling solutions include 1) 25-60% DI water, 30-60% nitric acid, and 2-20% sulfuric acid; 2) 40-75% acetic acid, 10-35% nitric acid, and 1-12% hydrofluoric acid; and 3) 50-100% hydrochloric acid. As can be appreciated, one or more different pickling solutions can be used during the pickling process. During the pickling process, the rod or tube is fully or partially immersed in the pickling solution for a sufficient amount of time to remove the impurities from the surface of the rod or tube. Typically, the time period for pickling is about 2-120 seconds; however, other time periods can be used. After the rod or tube has been pickled, the rod or tube is typically rinsed with a water (e.g., DI water, etc.) and/or a solvent (e.g., acetone, methyl alcohol, etc.) to remove any pickling solution from the rod or tube and then the rod or tube is allowed to dry. The rod or tube may be keep in a protective environment during the rinse and/or drying process to inhibit or prevent oxides from reforming on the surface of the rod or tube prior to the rod or tube being drawn and/or annealed; however, this is not required.

In yet another and/or alternative non-limiting aspect, the restraining apparatuses that are used to contact the novel metal alloy rod or tube during an annealing process and/or drawing process are typically formed of materials that will not introduce impurities to the novel metal alloy during the processing of the rod or tube. In one non-limiting embodiment, when the metal alloy rod or tube is exposed to temperatures above 150°C, the materials that contact the novel metal alloy rod or tube during the processing of the rod or tube are typically made from molybdenum, rhenium and/or tungsten. When the novel metal alloy rod or tube is processed at lower temperatures (i.e., 150°C or less), materials made from Teflon parts can also or alternatively be used.

In still another and/or alternative non-limiting aspect, the novel metal alloy rod or tube, after being formed to the desired outer cross-sectional area or diameter, inner cross-sectional area or diameter and/or wall thickness, can be cut and/or etched to at least partially form the desired configuration of the medical device (e.g., stent, etc.). In one non limiting embodiment, the novel metal alloy rod or tube is at least partially cut by a laser. The laser is typically desired to have a beam strength which can heat the novel metal alloy rod or tube to a temperature of at least about 2200-2300°C. In one non-limiting aspect of this embodiment, a pulsed Nd:YAG neodymium-doped yttrium aluminum garnet (Nd:Y₃Al₅O₁₂) or CO₂ laser is used to at least partially cut a pattern of medical device out of the novel metal alloy rod or tube. In another and/or alternative non-limiting aspect of this embodiment, the cutting of the novel metal alloy rod or tube by the laser can occur in a vacuum environment, an oxygen reducing environment, or an inert environment; however, this is not required. It has been found that laser cutting of the rod or tube in a non-protected environment can result in impurities being introduced into the cut rod or tube, which introduced impurities can induce micro-cracking of the rod or tube during the cutting of the rod or tube. One non-limiting oxygen reducing environment includes a combination of argon and hydrogen; however, a vacuum environment, an inert environment, or other or additional gasses can be used to form the oxygen reducing environment. In still another and/or alternative non-limiting aspect of this embodiment, the novel metal alloy rod or tube is stabilized so as to limit or prevent vibration of the rod or tube during the cutting process. The apparatus used to stabilize the rod or tube can be formed of molybdenum, rhenium, tungsten, molybdenum TZM alloy, ceramic, etc. so as to not introduce contaminants to the rod or tube during the cutting process; however, this is not required. Vibrations in the rod or tube during the cutting of the rod or tube can result in the formation of micro-cracks in the rod or tube as the rod or tube is cut. The average amplitude of vibration during the cutting of the rod or tube should be no more than about 150% the wall thickness of the rod or tube. In one non-limiting aspect of this embodiment, the average amplitude of vibration should be no more than about 100% the wall thickness of the rod or tube. In another non-limiting aspect of this embodiment, the average amplitude of vibration should be no more than about 75% the wall thickness of the rod or tube. In still another non-limiting aspect of this embodiment, the average amplitude of vibration should be no more than about 50% the wall thickness of the rod or tube. In yet another non-limiting aspect of this embodiment, the average amplitude of vibration should be no more than about 25% the wall thickness of the rod or tube. In still yet another non-limiting aspect of this embodiment, the average amplitude of vibration should be no more than about 15% the wall thickness of the rod or tube.

In still yet another and/or alternative non-limiting aspect, the novel metal alloy rod or tube, after being formed to the desired medical device, can be cleaned, polished, sterilized, nitrided, etc. for final processing of the medical device. In one non-limiting embodiment, the medical device is electropolished. In one non-limiting aspect of this embodiment, the medical device is cleaned prior to being exposed to the polishing solution; however, this is not required. The cleaning process, when used, can be accomplished by a variety of techniques such as, but not limited to, 1) using a solvent (e.g., acetone, methyl alcohol, etc.) and wiping the medical device with a Kimwipe or other appropriate towel, and/or 2) by at least partially dipping or immersing the medical device in a solvent and then ultrasonically cleaning the medical device. As can be appreciated, the medical device can be cleaned in other or additional ways. In another and/or alternative non-limiting aspect of this embodiment, the polishing solution can include one or more acids. One non-limiting formulation of the polishing solution includes about 10-80 percent by volume sulfuric acid. As can be appreciated, other polishing solution compositions can be used. In still another and/or alternative non-limiting aspect of this embodiment, about 5-12 volts are directed to the medical device during the electropolishing process; however, other voltage levels can be used. In yet another and/or alternative non-limiting aspect of this embodiment, the medical device is rinsed with water and/or a solvent and allowed to dry to remove polishing solution on the medical device.

In still another and/or alternative non-limiting aspect, the device can be used in conjunction with one or more other chemical agents that are not on the device. For instance, the success of the device can be improved by infusing, injecting or consuming orally one or more chemical agents. Such chemical agents can be the same and/or different from the one or more chemical agents on and/or in the device. Such use of one or more chemical agents are commonly used in systemic treatment of a patient after a medical procedure such as systemic therapy after the device has been inserted in the treatment area can be reduced or eliminated by use of the novel alloy. Although the device of the present invention can be designed to reduce or eliminate the need for long periods of systemic therapy after the device has been inserted in the treatment area, the use of one or more chemical agents can be used in conjunction with the device to enhance the success of the device and/or reduce or prevent the occurrence of in-stent restenosis, vascular narrowing, and/or thrombosis and/or promote tissue growth (e.g., endothelium and/or neural tissue). For instance, solid dosage forms of chemical agents for oral administration, and/or for other types of administration (e.g., suppositories, etc.) can be used. Such solid forms can include, but are not limited to, capsules, tablets, effervescent tablets, chewable tablets, pills, powders, sachets, granules and gels. The solid form of the capsules, tablets, effervescent tablets, chewable tablets, pills, etc. can have a variety of shapes such as, but not limited to, spherical, cubical, cylindrical, pyramidal, and the like. In such solid dosage form, one or more chemical agents can be admixed with at least one filler material such as, but not limited to, sucrose, lactose or starch; however, this is not required. Such dosage forms can include additional substances such as, but not limited to, inert diluents (e.g., lubricating agents, etc.). When capsules, tablets, effervescent tablets or pills are used, the dosage form can also include buffering chemical agents; however, this is not required. Soft gelatin capsules can be prepared to contain a mixture of the one or more chemical agents in combination with vegetable oil or other types of oil; however, this is not required. Hard gelatin capsules can contain granules of the one or more chemical agents in combination with a solid carrier such as, but not limited to, lactose, potato starch, corn starch, cellulose derivatives of gelatin, etc; however, this is not required. Tablets and pills can be prepared with enteric coatings for additional time release characteristics; however, this is not required. Liquid dosage forms of the one or more chemical agents for oral administration can include pharmaceutically acceptable emulsions, solutions, suspensions, syrups, elixirs, etc.; however, this is not required. In one non-limiting embodiment, when at least a portion of one or more chemical agents is inserted into a treatment area (e.g., gel form, paste form, etc.) and/or provided orally (e.g., pill, capsule, etc.) and/or anally (suppository, etc.), one or more of the chemical agents can be controllably released; however, this is not required. In one non-limiting example, one or more chemical agents can be given to a patient in solid dosage form and one or more of such chemical agents can be controllably released from such solid dosage forms. In another and/or alternative non-limiting example trapidil, trapidil derivatives, taxol, taxol derivatives, cytochalasin, cytochalasin derivatives, paclitaxel, paclitaxel derivatives, rapamycin, rapamycin derivatives, GM-CSF, GM-CSF derivatives, or analogs, or combinations thereof are given to a patient prior to, during and/or after the insertion of the device in a treatment area. Certain types of chemical agents may be desirable to be present in a treated area for an extended period of time in order to utilize the full or nearly full clinical potential of the chemical agent. For instance, Trapidil and/or trapidil derivatives is a compound that has many clinical attributes including, but not limited to, anti-platelet effects, inhibition of smooth muscle cells and monocytes, fibroblast proliferation and increased MAPK-1 which in turn deactivates kinase, a vasodilator, etc. These attributes can be effective in improving the success of a device that has been inserted at a treatment area. In some situations, these positive effects of trapidil and/or Trapidil derivatives need to be prolonged in a treatment area in order to achieve complete clinical competency. Trapidil and/or trapidil derivatives has a half life in vivo of about 2-4 hours with hepatic clearance of 48 hours. In order to utilize the full clinical potential of trapidil and/or trapidil derivatives, trapidil and/or trapidil derivatives should be metabolized over an extended period of time without interruption; however, this is not required. By inserting trapidil and/or trapidil derivatives in a solid dosage form, the trapidil and/or trapidil derivatives could be released in a patient over extended periods of time in a controlled manner to achieve complete or nearly complete clinical competency of the trapidil and/or trapidil derivatives. In another and/or alternative non-limiting example, one or more chemical agents are at least partially encapsulated in one or more polymers. The one or more polymers can be biodegradable, non-biodegradable, porous, and/or non-porous. When the one or more polymers are biodegradable, the rate of degradation of the one or more biodegradable polymers can be used to at least partially control the rate at which one or more chemical agents that are released into a body passageway and/or other parts of the body over time. The one or more chemical agents can be at least partially encapsulated with different polymer coating thicknesses, different numbers of coating layers, and/or with different polymers to alter the rate at which one or more chemical agents are released in a body passageway and/or other parts of the body over time. The rate of degradation of the polymer is principally a function of 1) the water permeability and solubility of the polymer, 2) chemical composition of the polymer and/or chemical agent, 3) mechanism of hydrolysis of the polymer, 4) the chemical agent encapsulated in the polymer, 5) the size, shape and surface volume of the polymer, 6) porosity of the polymer, 7) the molecular weight of the polymer, 8) the degree of cross-linking in the polymer, 9) the degree of chemical bonding between the polymer and chemical agent, and/or 10) the structure of the polymer and/or chemical agent. As can be appreciated, other factors may also affect the rate of degradation of the polymer. When the one or more polymers are biostable, the rate at when the one or more chemical agents are released from the biostable polymer is a function of 1) the porosity of the polymer, 2) the molecular diffusion rate of the chemical agent through the polymer, 3) the degree of cross-linking in the polymer, 4) the degree of chemical bonding between the polymer and chemical agent, 5) chemical composition of the polymer and/or chemical agent, 6) the chemical agent encapsulated in the polymer, 7) the size, shape and surface volume of the polymer, and/or 8) the structure of the polymer and/or chemical agent. As can be appreciated, other factors may also affect the rate of release of the one or more chemical agents from the biostable polymer. Many different polymers can be used such as, but not limited to, aliphatic polyester compounds (e.g., PLA (i.e. poly(D, L-lactic acid), poly(L-lactic acid)), PLGA (i.e. poly(lactide-co-glycoside), etc.), POE, PEG, PLLA, PDLLA, PCL, PDS, PDLGA, parylene, chitosan and/or copolymers, blends, and/or composites of above and/or derivatives of one or more of these polymers. As can be appreciated, the at least partially encapsulated chemical agent can be introduced into a patient by means other than by oral introduction, such as, but not limited to, injection, topical applications, intravenously, eye drops, nasal spray, surgical insertion, suppositories, intrarticularly, intraocularly, intranasally, intradermally, sublingually, intravesically, intrathecally, intraperitoneally, intracranially, intramuscularly, subcutaneously, directly at a particular site, and the like.

In another and/or non-limiting aspect, secondary substances can be combined in part with the polymer and/or chemical agent as a mixture, substrate layer, or at least in part at particular places within the device to modify the degradation and/or absorption of the polymer and/or the release of at least one chemical agent. In a non-limiting aspect, the secondary substance can contain at least one hydrophobic and/or hydrophilic substance which include, but are not limited to, fats, oils, methacrylate or its derivatives, amphiphilc or its derivatives, and at least one biodegradable polymers.

In still another and/or alternative aspect, the device can be an expandable device that can be expanded by use of some other device (e.g., balloon, etc.) and/or is self expanding. The expandable device can be fabricated at least in part from a material that has no or substantially no shape memory characteristics or can be fabricated from a material having shape-memory characteristics. Typically, when one or more shape-memory materials are used, the shape memory material composition is selected such that the shape memory material remains in an unexpanded configuration at a cold temperature (e.g., below body temperature) and/or in a restrained configuration; however, this is not required. When the shape memory material is heated (e.g., to body temperature), the expandable body section can be designed to expand to at least partially seal and secure the device in a body passageway or other region; however, this is not required. In an alternate non-limiting aspect, the shape memory material is in a superelastic state and is not required to be heated in order to expand.

According to the invention, the device is in the form of a stent. The stent can be an expandable stent that is expandable by a balloon and/or is self-expanding. The material used to form the stent is selected to withstand the manufacturing process that is needed to be accomplished in order to produce the stent. These manufacturing processes can include, but are not limited to, electroplating, MEMS technology, electro-polishing, chemical polishing, ion beam deposition or implantation, sputter coating, vacuum deposition, masking, molding, cutting, etching, and/or other coating processes. The device can have one or more body members. The one or more body members can include first and second ends and a wall surface disposed between the first and second ends. Typically, each body member has a first cross-sectional area which permits delivery of the body member into a body passageway, and a second, expanded cross-sectional area. The expansion of one or more body member of the device can be accomplished in a variety of manners. In one manner, one or more body members are expanded to the second cross-sectional area by a radially, outwardly extending force applied at least partially from the interior region of the body member (e.g., by use of a balloon, etc.). The body member can include heat sensitive and/or superelastic materials (e.g., shape memory materials, etc.) that expand upon exposure to heat, thus not requiring a radially, outwardly extending force applied at least partially from the interior region of the body member; however, such force can be used with such a body member. The second cross-sectional area of the device can be fixed or variable. The device can be designed such that one or more body members expand while substantially retaining the original longitudinal length of the body member; however, this is not required. The one or more body members can have a first cross-sectional shape that is generally circular so as to form a substantially tubular body member; however, the one or more body members can have other cross-sectional shapes. When the device includes two or more body members, the two or more body members can be connected together by at least one connector member. The device can include rounded, smooth and/or blunt surfaces to minimize and/or prevent damage to a body passageway as the device is inserted into a body passageway and/or expanded in a body passageway; however, this is not required. The device can be treated with gamma, beta and/or e-beam radiation, and/or otherwise sterilized; however, this is not required.

In still yet another and/or additional non-limiting aspect, one or more portions of the device can include or be made of the chemical agent and/or polymer. When the device is coated with one or more polymers, the polymer can include 1) one or more coatings of non-porous polymers; 2) one or more coatings of a combination of one or more porous polymers and one or more non-porous polymers; 3) one or more coatings of one or more porous polymers and one or more coatings of one or more nonporous polymers; 4) one or more coating of porous polymer, or 5) one or more combinations of options 1, 2, 3 and 4. The thickness of one or more of the polymer layers can be the same or different. Varying types and/or thickness of polymer layers can also be used.

In another and/or alternative non-limiting aspect, the medical device can include a bistable construction. In such a design, the medical device has two or more stable configurations, including a first stable configuration with a first cross-sectional shape and a second stable configuration with a second cross-sectional shape. All or a portion of the medical device can include the bistable construction. The bistable construction can result in a generally uniform change in shape of the medical device, or one portion of the medical device can change into one or more configurations and one or more other portions of the medical device can change into one or more other configurations.

According to the invention, the medical device is in the form of a stent. The stent can be an expandable stent that is expandable by a balloon and/or is self-expanding. The stent can have one or more body members. The one or more body members can include first and second ends and a wall surface disposed between the first and second ends. Typically each body member has a first cross-sectional area which permits delivery of the body member into a body passageway, and a second, expanded cross-sectional area. The expansion of one or more body members of the stent can be accomplished in a variety of manners. In one manner, one or more body members are expanded to the second cross-sectional area by a radially, outwardly extending force applied at least partially from the interior region of the body member (e.g. by use of a balloon, etc.). The body member can include shape memory materials; however, this is not required. The second cross-sectional area of the stent can be fixed or variable. The stent can be designed such that one or more body members expand while substantially retaining the original longitudinal length of the body member; however, this is not required. The one or more body members can have a first cross-sectional shape that is generally circular so as to form a substantially tubular body member; however, the one or more body members can have other cross-sectional shapes. When the stent includes two or more body members, the two or more body members can be connected together by at least one connector member. The stent can include rounded, smooth and/or blunt surfaces to minimize and/or prevent potential damage to a body passageway as the stent is inserted into a body passageway and/or expanded in a body passageway; however, this is not required. The stent can be treated with gamma, beta and/or e-beam radiation, and/or otherwise sterilized; however, this is not required.

In one non-limiting application, there is provided a medical device and method for using (not forming part of the invention) such medical device to inhibit or prevent thrombosis after the medical device has been inserted into a body passageway. The medical device can have one-limiting advantage of reducing or eliminating the need for long periods of body-wide anti-platelet and/or anti-coagulation therapy after the medical device has been inserted in the treatment area. The medical device can have one non-limiting advantage of delivering one or more chemical agents into a treatment area (e.g., body passageway, etc.). Such a medical device can be designed to be inserted in and/or be connected to a body passageway (e.g., blood vessel, etc.) and which medical device inhibits or prevents thrombosis. The medical device can be designed to be used as a biological agent delivery mechanism to deliver one or more chemical agents to and/or into a wall of a body passageway and/or down stream from the site of implantation of the medical device. In one non-limiting design, the medical device is a stent comprised of a base material that includes at least one layer of biological agent and at least one polymer layer that is used to at partially control the release of the biological agent from the medical device. In one non-limiting controlled release arrangement, molecular diffusion through a polymer is used to control the release rate of one or more chemical agents from the medical device When a molecular diffusion mechanism is used, one or more non-porous polymer layers can be used to facilitate in such molecular diffusion; however, this is not required. The molecular composition, molecular structure and/or coating thickness of the non-porous polymer can be selected to control the release rate of one or more chemical agents from the medical device. The polymer layer can include a porous or non-porous polymer. The one or more polymers and/or chemical agents that are used in conjunction with the stent can 1) form at least a portion of the medical device, 2) be coated on one or more regions of the medical device, and/or 3) be contained in one or more regions within the medical device. Non-limiting examples of polymers that can be used include parylene, PLGA, POE, PGA, PLLA, PAA, PEG, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers; however, other or additional polymers can be used. Many different chemical agents can be used. Such chemical agents can include anti-platelet compounds and/or anticoagulant compounds such as, but not limited to, warfarin (Coumadin) and/or derivatives, aspirin and/or derivatives, clopidogrel and/or derivatives, ticlopadine and/or derivatives, hirdun and/or derivatives, dipyridamole and/or derivatives, trapidil and/or derivatives, and/or heparin and/or low molecular weight heparin and/or derivatives. As can be appreciated, one or more other anti-thrombotic chemical agents can be combined with the medical device such as, but not limited to, taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The structure of the medical device during manufacture can be pre-treated (e.g., plasma etching, etc.) to facilitate in the coating of one or more polymers and/or chemical agents on the medical device; however, this is not required. The surface topography of the base structure of the medical device can be uniform or varied to achieve the desired operation and/or biological agent released from the medical device. As can be appreciated, one or more regions of the medical device can be constructed by use of one or more microelectromechanical manufacturing techniques; however, this is not required. Materials that can be used by microelectromechanical manufacturing techniques technology include, but are not limited to, chitosan, a chitosan derivative, PLGA, a PLGA derivative, PLA, a PLA derivative, PEVA, a PEVA derivative, PBMA, a PBMA derivative, POE, a POE derivative, PGA, a PGA derivative, PLLA, a PLLA derivative, PAA, a PAA derivative, PEG, and chitosan, a chitosan derivative, PLGA, a PLGA derivative, PLA, a PLA derivative, PEVA, a PEVA derivative, PBMA, a PBMA derivative, POE, a POE derivative, PGA, a PGA derivative, PLLA, a PLLA derivative, PAA, a PAA derivative, PEG, a PEG derivative, PDLLA, a PDLLA derivative, PCL, a PCL derivative, PDS, a PDS derivative, 85/15 PDLGA, 75/25 PDLGA, 50/50 PDLGA, 25/75 PDLGA, and/or 15/85 PDLGA. The medical device can include one or more surface structures, micro-structures, internal structures that include one or more chemical agents and/or polymers; however, this is not required. These structures can be at least partially formed by MEMS (e.g., micro-machining, etc.) technology and/or other types of technology. The structures can be designed to contain and/or be fluidly connected to a passageway in the medical device that includes one or more chemical agents; however, this is not required. The structures can be used to engage and/or penetrate surrounding tissue or organs once the medical device has been positioned on and/or in a patient; however, this is not required. One or more polymers and/or chemical agents can be inserted in these structures and/or at least partially form these structures of the medical device. The structures can be clustered together or disbursed throughout the surface of the medical device. Similar shaped and/or sized structures can be used, or different shaped and/or sized structures can be used. Typically the micro-structures, when formed, extend from or into the outer surface no more than about 400 microns, and more typically less than about 300 microns, and more typically about 15-250 microns; however, other sizes can be used. The time period one or more chemical agents are released from the medical device is typically dependent on the designed medical treatment and/or other factors. In one non-limiting arrangement, one or more chemical agents are released from the medical device for at least several days after the medical device is inserted in the body of a patient; however, this is not required. In another one non-limiting arrangement, one or more chemical agents are released from the medical device for at least about one week after the medical device is inserted in the body of a patient. In still another one non-limiting arrangement, one or more chemical agents are released from the medical device for at least about two weeks after the medical device is inserted in the body of a patient. In yet another one non-limiting arrangement, one or more chemical agents are released from the medical device for about one week to one year after the medical device is inserted in the body of a patient. As can be appreciated, the time frame that one or more of the chemical agents can be released from the medical device can be longer or shorter. The time period for the release of two or more chemical agents from the medical device can be the same or different. The type of the one or more chemical agents used on the medical device, the release rate of the one or more chemical agents from the medical device, and/or the concentration of the one or more chemical agents being released from the medical device can be the same or different. The controlled release rate of one or more chemical agents from the medical device can result in reduced amounts and/or reduce time period of systemic drug therapy after the medical device is inserted in the treatment area. In one non-limiting arrangement, the medical device releases one or more chemical agents for a period of time such that systemic drug therapy after the medical device is inserted in the treatment area is reduced to less than one year. In another and/or alternative non-limiting arrangement, the medical device releases one or more chemical agents for a period of time such that systemic drug therapy after the medical device is inserted in the treatment area is reduced to less than one month. In still another and/or alternative non-limiting arrangement, the medical device releases one or more chemical agents for a period of time such that systemic drug therapy after the medical device is inserted in the treatment area is reduced to less than one week. The medical device can be temporality used in conjunction with other chemical agents. For instance, the success of the medical device can be improved by infusing, injecting or consuming orally one or more chemical agents. Such chemical agents can be the same and/or different from the one or more chemical agents on and/or in the medical device. For instance, solid dosage forms of chemical agents for oral administration can be used. Such solid forms can include, but are not limited to, capsules, tablets, effervescent tablets, chewable tablets, pills, powders, sachets, granules and gels.

In another and/or alternative non-limiting application, there is provided a medical device that is adapted for introduction into a patient and/or for topical use (e.g., lotion, salve, gel, etc.), which medical device releases one or more chemical agents in a controlled release manner. The medical device can have a variety of applications such as, but not limited to, placement into the vascular system. As can be appreciated, the medical device can have other or additional uses. One or more chemical agents on and/or in the medical device can be released controllably and/or uncontrollably from the medical device. As such, all of the chemical agents can be controllably released from the medical device, all of the chemical agents can be uncontrollably released from the medical device, or one or more chemical agents can be controllably released and one or more chemical agents can be uncontrollably released from the medical device. The controlled release of the one or more chemical agents from the medical device can be at least partially controlled by molecular diffusion through one or more non-porous polymer layers; however, it will be appreciated that other or additional mechanisms can be used to control the rate of release of one or more chemical agents from the medical device. For instance, the one or more chemical agents can be selected so as to be chemically bonded to one or more polymers to control the rate of release of one or more chemical agents from the medical device; however, this is not required. The one or more polymers can include cross-links to control the rate of release of one or more chemical agents from the medical device; however, this is not required. The one or more polymers and/or one or more chemical agents can be hydrophobic or hydrophilic, thus can be used to facilitate in the controlled release of the one or more chemical agents from the medical device; however, this is not required. The thickness of the one or more polymer layers can be selected to facilitate in the controlled release of the one or more chemical agents; however, this is not required. The molecular weight and/or molecular structure of the one or more chemical agents and/or one or more polymer can be selected to facilitate in the release of the one or more chemical agents; however, this is not required. Many different chemical agents can be used. Such chemical agents can include anti-platelet compounds and/or anticoagulant compounds such as, but not limited to, warfarin (Coumadin) and/or derivatives, aspirin and/or derivatives, clopidogrel and/or derivatives, ticlopadine and/or derivatives, hirdun and/or derivatives, dipyridamole and/or derivatives, trapidil and/or derivatives, and/or heparin and/or low molecular weight heparin and/or derivatives. As can be appreciated, one or more other anti-thrombotic chemical agents can be combined with the medical device such as, but not limited to, taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. In still another one non-limiting arrangement, one or more chemical agents are released from the medical device for at least about two weeks after the medical device is inserted in the body of a patient. In yet another one non-limiting arrangement, one or more chemical agents are released from the medical device for about one week to one year after the medical device is inserted in the body of a patient. As can be appreciated, the time frame that one or more of the chemical agents can be released from the medical device can be longer or shorter. The time period for the release of two or more chemical agents from the medical device can be the same or different. The type of the one or more chemical agents used on the medical device, the release rate of the one or more chemical agents from the medical device, and/or the concentration of the one or more chemical agents being released from the medical device can be the same or different.

According to the present invention, there is provided a medical device that is adapted for introduction into a patient, that is designed for insertion in a body passageway, which medical device includes a stent with abluminal and luminal surfaces and comprising at least 60 weight percent of a metal alloy comprising as primary metals molybdenum and rhenium, wherein a "primary metal" is a metal component of the metal alloy that is not a metal impurity, the metal alloy being a solid solution or homogenous solution including the primary metals rhenium and molybdenum in at least about 95 wt.-%, or a solid solution including the primary metals rhenium and molybdenum at about 95-99 wt.-%; a base drug coating applied to the stent, and a bioabsorbable coating. The base drug coating is comprised of 100% drug and is in sufficient amounts and/or strength to suppress inflammation resulting from absorption of the bioabsorbable coating. The base drug coating can be present on the stent until complete absorption of the bioabsorbable coating. The base drug coating can be applied to the abluminal surface of the device. The stent includes a secondary drug coating that is applied to the luminal surface of the stent. The secondary drug coating is in sufficient amounts and/or strength to promote the growth of endothelium on the luminal surface of the stent. The bioabsorbable coating can include PGA, PLA, PLLA, PDLLA, PCL, PDS, 85/15 PDLGA, 75/25 PDLGA, 50/50 PDLGA, 25/75 PDLGA, 15/85 PDLGA, and combinations thereof. The weight percent of bioabsorbable coating to drug on the stent can range from about 1 to about 99 weight percent. The thickness of the abluminal coating on the stent can vary from about 2 to about 50 microns. The thickness of the luminal coating on the stent can vary from about 2 to about 50 microns. The absorption time of the bioabsorbable coating on the stent can be at least about 1 month. The absorption time of the bioabsorbable coating on the stent can be up to about 12 months. The absorption time of the bioabsorbable coating on the stent can be up to about 6 months. The absorption time of the bioabsorbable coating can be designed so as to not be greater than the time for complete elution of drug from the stent. The absorption time of the bioabsorbable coating can be designed to be greater than the time for complete elution of drug from the device. The drug coating on the stent can be designed such that 100 percent of the drug elutes from the bioabsorbable or luminal coating in less than about 12 months. The drug coating on the stent can be designed such that less than 100 percent of the drug elutes from the bioabsorbable or luminal coating within about 60 days. The drug coating on the stent can be designed such that less than 100 percent of the drug elutes from the bioabsorbable or luminal coating within about 30 days. The drug coating on the stent can be designed such that less than about 80 percent of the drug elutes from the bioabsorbable or luminal coating within about 30 days. The bioabsorbable coating can be coated to the luminal surface of the stent. The bioabsorbable coating can be coated to the abluminal surface of the stent.

One non-limiting object is the provision of a medical device having improved procedural success rates.

Another and/or alternative non-limiting object is the provision of a medical device that can be implanted into the vascular system of a mammalian without the need of body wide aggressive anti-platelet and/or anti-coagulation therapy over extended periods of time and a method using such a device.

Still another and/or alternative non-limiting object is the provision of a medical device in the form of a stent that inhibits or prevents the occurrence of thrombosis after the medical device has been inserted into a body passageway.

Yet another and/or alternative non-limiting object is the provision of a medical device that is at least partially formed of, contains and/or is coated with one or more chemical agents.

Still yet another and/or alternative non-limiting object is the provision of a medical device that inhibits or prevents thrombosis by localized release of one or more chemical agents and a method using such a device.

Another and/or alternative non-limiting object is the provision of a medical device that controllably releases one or more chemical agents.

Yet another and/or alternative non-limiting object is the provision of a medical device that inhibits or prevents the occurrence of in-stent restenosis, vascular narrowing and/or restenosis after the medical device has been inserted into a body passageway.

Still another and/or alternative non-limiting object is the provision of a medical device that includes one or more surface structures and/or micro-structures.

Yet another and/or alternative non-limiting object is the provision of a medical device that includes one or more internal structures, micro-structures and/or surface structures that include and/or are coated with one or more chemical agents and/or polymers.

Still another and/or alternative non-limiting object is the provision of a medical device that includes one or more surface structures, micro-structures and/or internal structures and a protective coating that at least partially covers and/or protects such structures.

Still a further and/or alternative non-limiting object is the provision of a medical device that can be used in conjunction with one or more chemical agents not on or in the medical device.

These and other advantages will become apparent to those skilled in the art upon the reading and following of this description taken together with the accompanying drawings.

### BRIEF DESCRIPTION OF THE DRAWINGS

Reference may now be made to the drawings, which illustrate various embodiments that the invention may take in physical form and in certain parts and arrangements of parts wherein:
FIGURE 1 is a perspective view of a section of a medical device in the form of an unexpanded stent which permits delivery of the stent into a body passageway;
FIGURE 2 is a sectional view of the stent of FIGURE 1;
FIGURE 3 is a cross-sectional view along line 3-3 of FIGURE 2 illustrating one type of coating on a medical device;
FIGURE 4 is a cross-sectional view along line 3-3 of FIGURE 2 illustrating another type of coating on a medical device;
FIGURE 5 is a cross-sectional view along line 3-3 of FIGURE 2 illustrating another type of coating on a medical device;
FIGURE 6 is a cross-sectional view along line 3-3 of FIGURE 2 illustrating another type of coating on a medical device;
FIGURE 7 is a cross-sectional view along line 3-3 of FIGURE 2 illustrating pores in the body of the medical device containing a biological agent and a coating on the medical device;
FIGURE 8 is a cross-sectional view along line 3-3 of FIGURE 2 illustrating pores in the body of the medical device containing a biological agent and a biological agent coating on the medical device;
FIGURE 9 is a cross-sectional view along line 3-3 of FIGURE 2 illustrating pores in the body of the medical device containing a biological agent and a biological agent coating on the medical device and a polymer coating over the biological agent;
FIGURE 10 is a cross-sectional view along line 3-3 of FIGURE 2 illustrating micro-needles on the surface of the medical device that are formed of a biological agent;
FIGURE 11 is a cross-sectional view along line 3-3 of FIGURE 2 illustrating micro-needles on the surface of the medical device that are formed of a biological agent and polymer;
FIGURE 12 is a cross-sectional view along line 3-3 of FIGURE 2 illustrating micro-needles on the surface of the medical device that are formed of a biological agent and coated with a polymer;
FIGURE 13 is a cross-sectional view along line 3-3 of FIGURE 2 illustrating micro-needles on the surface of the medical device that are formed of a biological agent and polymer and coated with a polymer;
FIGURE 14 is a cross-sectional view along line 3-3 of FIGURE 2 illustrating micro-needles on the surface of the medical device that are formed of a polymer and includes an internal cavity that includes a biological agent;
FIGURE 15 is a perspective view of a section of medical device in the form of a surgical graft that includes an internal biological agent coating and a polymer coating over the biological agent;
FIGURE 16 is an expanded section of the surgical graft identified in FIGURE 15; and,
FIGURE 17 is a cross-sectional view of a micro-needle on a medical device that is penetrating into the inner surface of a body passageway or organ.

### DETAILED DESCRIPTION OF THE INVENTION

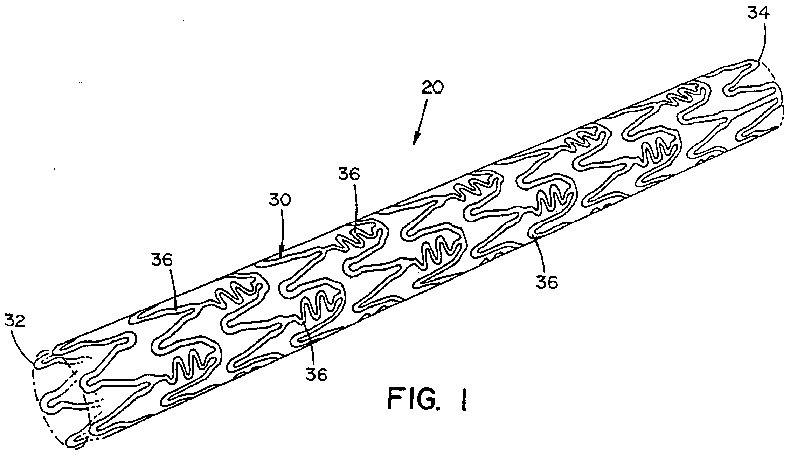

Referring now to the drawings wherein the showing is for the purpose of illustrating preferred embodiments of the invention only and not for the purpose of limiting the same, FIGURES 1-2 disclose a medical device in the form of a stent for use in a body passageway. The medical device can be designed to address one or more of the shortcomings of prior medical devices. One non-limiting feature of the medical device can be to locally deliver one or more chemical agents to a particular body region. Another and/or alternative non-limiting feature of the medical device can be to locally deliver one or more chemical agents to a particular body region and to at least partially release one or more chemical agents in a controlled manner.

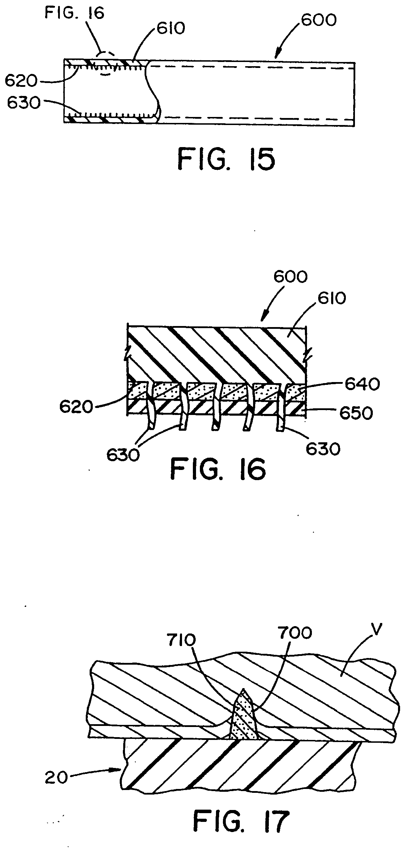

Although, FIGURES 1-2 illustrate the medical device in the form of a stent for use in the cardiovascular field, the medical device can be used in other medical fields such as, but not limited to, orthopedic field, cardiology field, pulmonology field, urology field, nephrology field, gastrointerology field, gynecology field, otolaryngology field or other surgical fields. The medical device can be in a form other than a stent (not forming part of the invention) such as a surgical graft as illustrated in FIGURES 15 & 16), a suture, a staple, an orthopedic implant, a bandage, a valve, a micro or nano device, a vascular implant, a drug delivery catheter, an infusion catheter, a balloon, a catheter tip, a drug pump, tubing, a bag, a lead, a pacemaker, an implantable pulse generator, an implantable cardiac defibrillator, a cardio-verger defibrillator, a defibrillator, a spinal stimulator, a brain stimulator, a sacral nerve stimulator, a chemical sensor, a spinal implant, a membrane surface, a sheath, a guide wire, a balloon catheter, a hypotube, a catheter (e.g., electrophysiology catheters, guide catheter, stent catheter, etc.), a cutting device, a PFO (patent foramen ovale) device, a wrap, a biological glue, a gel, etc. As can be appreciated, the medical device can take other forms (not forming part of the invention) (e.g., lotions, salves, gels, capsules, tablets, effervescent tablets, chewable tablets, pills, powders, sachets, granules, etc.).

The medical device when used for vascular applications, can be used to address various medical problems such as, but not limited to, restenosis, atherosclerosis, atherogenesis, angina, ischemic disease, congestive heart failure or pulmonary edema associated with acute myocardial infarction, atherosclerosis, thrombosis, controlling blood pressure in hypertension, platelet adhesion, platelet aggregation, smooth muscle cell proliferation, vascular complications, wounds, myocardial infarction, pulmonary thromboembolism, cerebral thromboembolism, thrombophiebitis, thrombocytopenia or bleeding disorders.

The medical device can be formed of a variety of materials such as, but not limited to, biostable polymers, biodegradable polymers, metals, plastics, cloth, fibers, or any combination thereof. As can be appreciated, many types of biodegradable polymers and non-biodegradable polymers can be used to at least partially form the medical device. The medical device can be at least partially biostable or at least partially biodegradable. The material or materials used to form the medical device include properties (e.g., strength, durability, hardness, biostability, bendability, coefficient of friction, radial strength, flexibility, tensile strength, longitudinal lengthening, stress-strain properties, improved recoil properties, radiopacity, heat sensitivity, biocompatability, biostability, biodegradability, biocompatability, etc.) that are selected to form a medical device which promotes the success of the medical device. The medical device is in the form of a stent, a stent that can be expandable such as by a balloon and/or self expanding.

The material that is used to form one or more portions of the medical device is typically selected to withstand the manufacturing process used to form the medical device (e.g., electroplating, electro polishing, extrusion, molding, EDM machining, MEMS (e.g., micro-machining, etc.) manufacturing, chemical polishing, ion beam deposition or implantation, sputter coating, vacuum deposition, plasma deposition, etc.).

The medical device can include one or more surface structures, micro-structures and/or internal structures. Such structures can be formed by a variety of processes (e.g., machining, chemical modifications, chemical reactions, micro-machining, etching, etc.). The one or more coatings and/or one or more surface structures, micro-structures and/or internal structures of the medical device can be used for a variety of purposes such as, but not limited to, 1) increasing the bonding and/or adhesion of one or more chemical agents, adhesives, marker materials and/or polymers to the medical device, 2) changing the appearance or surface characteristics of the medical device, and/or 3) controlling the release rate of one or more chemical agents. The techniques employed to deliver the medical device include, but are not limited to, angioplasty, vascular anastomoses, transplantation, implantation, subcutaneous introduction, minimally invasive surgical procedures, injection, topical applications, bolus administration, infusion, interventional procedures, and any combinations thereof. When the medical device is in the form of a surgical graft (not forming part of the invention) or stent as illustrated in FIGURES 1-17, the medical device can be implanted or applied by techniques such as, but not limited to, suturing, staples, adhesive, anastomoses, balloon delivery, sheath catheter delivery, etc.

Referring again to FIGURES 1-2, there is disclosed a medical device in the form of a stent for a body passageway. The stent is an expandable stent for at least partially expanding occluded segments of a body passageway; however, the stent can have other or additional uses. For example, the expandable stent may be used for, but not limited to, such purposes as 1) a supportive stent for placement within a blocked vasculature opened by transluminal recanalization, which are likely to collapse in the absence of an internal support; 2) forming a catheter passage through the mediastinal and/or other veins occluded by inoperable cancers; 3) reinforcement of catheter created intrahepatic communications between portal and/or hepatic veins in patients suffering from portal hypertension; 4) a supportive stent for placement in the narrowing of the esophagus, the intestine, the ureter and/or the urethra; and/or 5) a supportive stent for reinforcement of reopened and/or previously obstructed bile ducts. Accordingly, use of the term "stent" encompasses the foregoing or other usages within various types of body passageways.

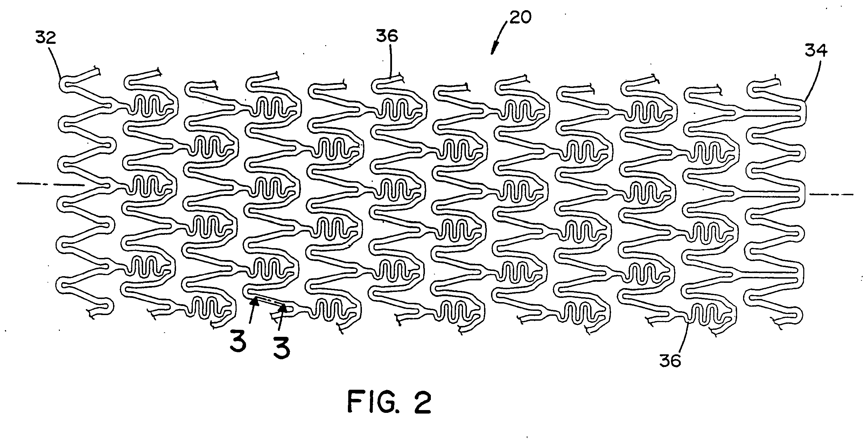

As illustrated in FIGURE 1, the medical device 20 in the form of an expandable stent includes at least one tubular shaped body member 30 having a first end 32, a second end 34, and member structures 36 disposed between the first and second ends. FIGURE 2 illustrates the stent prior to being formed into a generally tubular shape. As can be appreciated, the stent can be formed of a plurality of body members connected together. Body member 30 has a first diameter which permits delivery of the body member into a body passageway. The first diameter of the body member is illustrated as being substantially constant along the longitudinal length of the body member. As can be appreciated, the body member can have a varying first diameter along at least a portion of the longitudinal length of the body member. The body member also has a second expanded diameter, not shown. The second diameter typically varies in size; however, the second diameter can be non-variable in size. The stent can be expanded in a variety of ways such as by a balloon and/or be at least partially self expanding. A balloon expandable stent is typically pre-mounted or crimped onto an angioplasty balloon catheter. The balloon catheter is then positioned into the patient via a guide wire. Once the stent is properly positioned, the balloon catheter is inflated to the appropriate pressure for stent expansion. After the stent has been expanded, the balloon catheter is deflated and withdrawn, leaving the stent deployed at the treatment area. A self expanding stent includes a material that has physical properties that do not require balloon expansion; however, a balloon can be used. These stents are typically manufactured in their final clinically relevant size and are temporarily reduced in size and mounted onto a delivery system; however, this is not required. The deployment strategy is similar to that of the balloon expandable stent except that a retaining system (e.g., sheath, adhesive, etc.) is withdrawn, degrades, breaks, etc. after the stent is positioned in the treatment area. After the retaining system is withdrawn, degrades or is broken, the stent expands. As can be appreciated, expansion of such a stent can be facilitated by use of a balloon, heat, etc.; however, this is not required.

One or more surfaces of the stent can be treated so as to have generally smooth surfaces. Generally, one or more ends of the stent are treated by filing, buffing, polishing, grinding, coating, and/or the like to remove or reduce the number of rough and/or sharp surfaces; however, this is not required. The smooth surfaces of the ends can be used to reduce potential damage to surrounding tissue as the stent is positioned in and/or expanded in a body passageway.

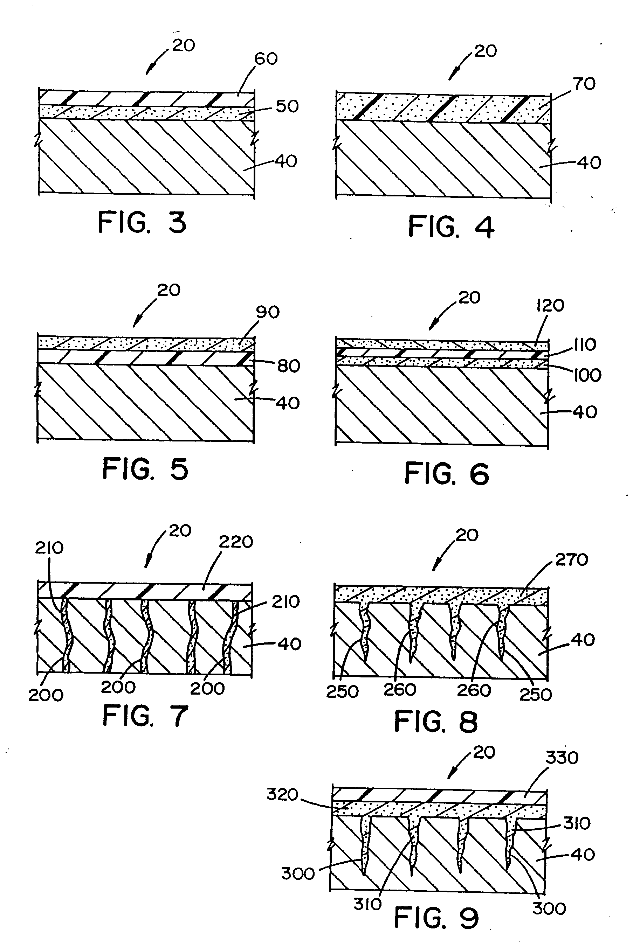

Referring now to FIGURES 3-14, there is illustrated a portion of the stent that includes and/or is coated with one or more chemical agents that are used to improve the functionality and/or success of the medical device such as, but not limited to inhibiting or preventing thrombosis. As can be appreciated, the coating combinations and structural combinations illustrated in FIGURES 3-14 can be used on the surgical graft (not forming part of the invention) as illustrated in FIGURE 15. As illustrated in FIGURES 3-15, the stent can include and/or be coated with one or more polymers and/or chemical agents. The one or more polymers can be porous or non-porous polymers. The one or more chemical agents can be, but are not limited to, anti-biotic agents, anti-body targeted therapy agents, anti-hypertensive agents, anti-microbial agents, anti-mitotic agents, anti-oxidants, anti-polymerases agents, anti-proliferative agents, anti-secretory agents , anti-tumor agents, anti-viral agents, bioactive agents, chemotherapeutic agents, cellular components, cytoskeletal inhibitors, drug, growth factors, growth factor antagonists, hormones, immunosuppressive agents, living cells, non-steroidal anti-inflammatory drugs, radioactive materials, radio-therapeutic agents, thrombolytic agents, vasodilator agents, etc. Non-limiting examples of chemical agents that can be used on a stent for use in the vascular system include, but are not limited to, a vascular active agent that inhibits and/or prevents restenosis, vascular narrowing and/or in-stent restenosis. Non-limiting examples of such chemical agents include anti-platelet compounds and/or anticoagulant compounds such as, but not limited to, warfarin (Coumadin) and/or derivatives, aspirin and/or derivatives, clopidogrel and/or derivatives, ticlopadine and/or derivatives, hirdun and/or derivatives, dipyridamole and/or derivatives, trapidil and/or derivatives, and/or heparin and/or low molecular weight heparin and/or derivatives. As can be appreciated, one or more other anti-thrombotic chemical agents can be combined with the stent such as, but not limited to, taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. For example, the amount of biological agent delivered to a certain region of a patient's body can be controlled by, but not limited to, one or more of the following: a) selecting the type of biological agent to be used on and/or in the stent, b) selecting the amount of biological agent to be used on and/or in the stent, c) selecting the coating thickness of the biological agent to be used on the stent, d) selecting the drug concentration of the biological agent to be used on and/or in the stent, e) selecting the solubility of the biological agent to be used on and/or in the stent, f) selecting the location the biological agent that is to be coated and/or impregnated on and/in the stent, g) selecting the amount of surface area of the stent that is coated and/or impregnated with the biological agent, h) selecting the location of the biological agent on the stent, I) selecting the size, shape, amount and/or location of the one or more surface structures, micro-structures and/or internal structures of the stent that include and/or are integrated with the biological agent, j) selecting the type and/or amount of polymer to be mixed with the biological agent, k) selecting the type, amount and/or coating thickness of the polymer coating used to at least partially coat and/or encapsulate the biological agent, etc. As can be appreciated, the amount of one or more biological agent delivered to a region of the body can be at least partially controlled in other or additional ways.

One or more chemical agents on and/or in the stent can be controllably released and/or immediately released to optimize their effects in a treatment area and/or to compliment the function and success of the stent in a treatment area. The controlled release of one or more chemical agents from the stent can be accomplished by 1) controlling the size and/or shape of the surface structures, micro-structures and/or internal structures in the stent, and 2) combining and/or coating one or more chemical agents with one or more polymers. As can be appreciated, the controlled release of one or more polymers can be accomplished by other and/or additional arrangements. The one or more polymers can also or alternatively be used to assist in binding the one or more chemical agents to the stent. The one or more polymers can be biodegradable or biostable. The one or more polymers can be formulated to form a bond between one or more chemical agents and the stent; however, this is not required. The one or more polymers and one or more chemical agents can be mixed together prior to being applied to the stent; however, this is not required. The one or more polymers can be used to control the release of one or more chemical agents by molecular diffusion and/or by one or more other mechanisms; however, this is not required. The thickness of the one or more polymer layers can be about 0.5-25 µ; however, other coating thickness can be used. When one or more chemical agents are controllably released from the stent, the time period the one or more chemical agents are released from the stent can vary. Generally, one or more biological agent are released from the stent over a period of at least several days after the stent is inserted in the body of a patient. As can be appreciated, the time frame that one or more of the chemical agents can be released from the stent can be longer or shorter. The one or more chemical agents that are released from the stent can be controllably released and/or non-controllably released. The time period for the release of two or more chemical agents from the stent can be the same or different. The type of the one or more chemical agents used on the stent, the release rate of the one or more chemical agents from the stent, and/or the concentration of the one or more chemical agents being released from the stent during a certain time period is typically selected to deliver one or more chemical agents to the area of treatment so as to increase the success of the stent (e.g., inhibit or prevent thrombosis, inhibit or prevent restenosis, vascular narrowing and/or in-stent restenosis after the stent has been implanted in a body passageway, etc.). The stent can be designed such that one or more chemical agents are released from the stent for at least several minutes to at least several days after the stent is inserted in the body of a patient. As can be appreciated, the time frame that one or more of the chemical agents can be released from the stent can be varied. The stent can be designed such that one or more chemical agents are released from the stent so as to inhibit or prevent thrombosis after the stent has been implanted without the need for aggressive anti-platelet and/or anti-coagulation therapy. In one non-limiting design of stent, the stent releases one or more chemical agents over a period of time after being inserted in the body so that no further anti-platelet and/or anti-coagulation therapy is required after the stent has been implanted. In another and/or alternative non-limiting design of stent, the stent releases one or more chemical agents over a period of time after being inserted in the body so that no further anti-platelet and/or anti-coagulation therapy is required about one day to two weeks after the stent has been implanted. In still another and/or alternative non-limiting design of stent, the stent releases one or more chemical agents over a period of time after being inserted in the body so that no further anti-platelet and/or anti-coagulation therapy is required about two weeks to one month after the stent has been implanted. The stent can be used overcomes the requirement of past implanted stents to have the patient on aggressive anti-platelet and/or anti-coagulation therapy for months after the stent has been implanted in the patient.

When the one or more chemical agents are used in combination with a non-porous polymer to controlled release of the one or more chemical agents by molecular diffusion, the one or more polymers include, but are not limited to, polyamide, parylene C, parylene N, parylene F, poly(ethylene oxide), poly(ethylene glycol), poly(propylene oxide), silicone based polymers, polymers of methane, tetrafluoroethylene or tetramethyidisiloxane, a polymer derived from photopolymerizeable monomers and/or derivatives thereof. Such polymer can be coated on the stent by vapor deposition or plasma deposition; however, other or additional coating techniques can be used. The thickness of the one or more non-porous polymer layer, when applied by catalyst-free vapor deposition or plasma deposition is about 0.5-25µ; however, other coating thicknesses can be used.

The surface of the base structure of the stent can be treated to enhance the coating of the stent and/or to enhance the mechanical characteristics of the stent; however, this is not required. Such surface treatment techniques include, but are not limited to, cleaning, buffing, smoothing, etching (chemical etching, plasma etching, etc.), etc. When an etching process is used, various gasses can be used for such a surface treatment process such as, but not limited to, carbon dioxide, nitrogen, oxygen, Freon, helium, hydrogen, etc. The plasma etching process can be used to clean the surface of the stent, change the surface properties of the stent so as to affect the adhesion properties, lubricity properties, etc. of the surface of the stent. As can be appreciated, other or additional surface treatment processes can be used prior to the coating of one or more chemical agents and/or polymers on the surface of the stent.

As illustrated in FIGURES 3-6, various coating combinations can be used on the stent. Referring to FIGURE 3, the base structure 40 of the stent includes a layer 50 of biological agent. The layer of biological agent can include one or more chemical agents. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. A polymer layer 60 is coated on the top of layer 50. The polymer layer can include one or more polymers. The polymer layer can include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. In one non-limiting example, the polymer layer includes one or more non-porous polymers to at least partially control a rate of release by molecular diffusion of the one or more chemical agents of layer 50 from stent 20. The one or more non-porous polymers can include, but is not limited to, parylene C, parylene N, parylene F and/or a parylene derivative.

As illustrated in FIGURE 4, the base structure 40 of stent 20 includes a layer 70 of polymer and biological agent. Layer 70 can include one or more chemical agents mixed with one or more polymers. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The one or more polymers can include one or more porous and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. In one non-limiting example, the one or more polymers included in layer 70 include a non-porous polymer to at least partially control a rate of release by molecular diffusion of the one or more chemical agents in layer 70. The non-porous polymer can include, but is not limited to, parylene C, parylene N, parylene F and/or a parylene derivative.

As illustrated in FIGURE 5, the base structure 40 of stent 20 includes a layer 80 of polymer. Layer 80 can include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. The one or more non-porous polymers, when used, can include, but are not limited to, parylene C, parylene N, parylene F and/or a parylene derivative. A layer 90 of one or more chemical agents is coated on top of polymer layer 80. Polymer layer 8 can be used to facilitate in the securing of layer 90 to the stent; however, this is not required. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The placement of a layer of biological agent on the top surface of the stent can provide a burst of biological agent in the treatment area (e.g., body passageway, etc.) after insertion of the stent. In one non-limiting example, the one or more chemical agents include trapidil and/or derivatives thereof.

As illustrated in FIGURE 6, the base structure 40 of stent 20 includes a layer 100 of one or more chemical agents. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. A polymer layer 110 is coated on the top of layer 100. The polymer layer can include one or more polymers. The polymer layer can include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PDLLA, PCL, PDS, 85/15 PDLGA, 75/25 PDLGA, 50/50 PDLGA, 25/75 PDLGA,15/85 PDLGA, chitosan and/or derivatives of one or more of these polymers. In one non-limiting example, the polymer layer includes one or more non-porous polymers to at least partially control a rate of release by molecular diffusion of the one or more chemical agents of layer 100 from stent 20. The one or more non-porous polymers can include, but are not limited to, parylene C, parylene N, parylene F and/or a parylene derivative. A layer 120 of biological agent is coated on top of polymer layer 110. Layer 120 can include one or more chemical agents. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The placement of a layer of biological agent on the top surface of the stent provide can provide a burst of one or more chemical agents in the treatment area (e.g., body passageway, etc.) after insertion of the stent. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. As can be appreciated, other combinations of polymer layer and layer of biological agent can be used on the stent. These other combinations are also encompassed within the scope of the present invention.

Referring now to FIGURE 7, the base structure 40 of stent 20 includes one or more surface structures and/or micro-structures 200. The one or more surface structures and/or micro-structures can be formed in the base structure during the formation of the base structure and/or from the treatment of the base structure (e.g. etching, mechanical drill, laser cutting, water cutting, etc.) and/or from one or more micro-machining processes. The one or more surface structures and/or micro-structures 200 are shown to include one or more chemical agents 210; however, it can be appreciated that the one or more surface structures and/or micro-structures 200 can include a combination of one or more polymers and one or more chemical agents, or only one or more polymers. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The size of the one or more surface structures and/or micro-structures can be used to at least partially control the rate of release of the one or more chemical agents and/or polymers from the one or more surface structures and/or micro-structures. A polymer layer 220 is coated on the top surface of the base structure 40. The polymer layer can include one or more polymers. The polymer layer can include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PDLLA, PCL, PDS, 85/15 PDLGA, 75/25 PDLGA, 50/50 PDLGA, 25/75 PDLGA,15/85 PDLGA, chitosan and/or derivatives of one or more of these polymers. In one non-limiting example, the polymer layer includes one or more non-porous polymers to at least partially control a rate of release by molecular diffusion of the one or more chemical agents in the one or more surface structures and/or micro-structures 200. The one or more non-porous polymers can include, but are not limited to, parylene C, parylene N, parylene F and/or a parylene derivative.

Referring now to FIGURE 8, the base structure 40 of stent 20 includes one or more surface structures and/or micro-structures 250. The one or more surface structures and/or micro-structures can be formed in the base structure during the formation of the base structure and/or from the treatment of the base structure (e.g. etching, mechanical drill, laser cutting, water cutting, etc.) and/or from one or more micro-machining processes. The one or more surface structures and/or micro-structures 250 are shown to include one or more chemical agents 260; however, it can be appreciated that the one or more surface structures and/or micro-structures 250 can include a combination of one or more polymers and one or more chemical agents, or only one or more polymers. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The size of the one or more surface structures and/or micro-structures can be used to at least partially control the rate of release of the one or more chemical agents and/or polymers from the one or more surface structures and/or micro-structures. A layer 270 of biological agent is coated on the top surface of the base structure. Layer 270 can include one or more chemical agents. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The placement of a layer of biological agent on the top surface of the stent can provide a burst of one or more biological agent in the treatment area after insertion of the stent. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. As can be appreciated, the one or more chemical agents of layer 270 and in the one or more surface structures and/or micro-structures 250 can be the same or different.

Referring now to FIGURE 9, the base structure 40 of stent 20 includes one or more surface structures and/or micro-structures 300. The one or more surface structures and/or micro-structures can be formed in the base structure during the formation of the base structure and/or from the treatment of the base structure (e.g. etching, mechanical drill, laser cutting, water cutting, etc.) and/or from one or more micro-machining processes. The one or more surface structures and/or micro-structures 300 are shown to include one or more chemical agents 310; however, it can be appreciated that the one or more surface structures and/or micro-structures 300 can include a combination of one or more polymers and one or more chemical agents, or only one or more polymers. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The size of the one or more surface structures and/or micro-structures can be used to at least partially control the rate of release of the one or more chemical agents and/or polymers from the one or more surface structures and/or micro-structures. A layer 320 of biological agent is coated on the top surface of the base structure. Layer 320 can include one or more chemical agents. In one non-limiting example, the biological agent includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. As can be appreciated, the one or more chemical agents of layer 320 and in the one or more surface structures and/or micro-structures 300 can be the same or different. A polymer layer 330 is coated on the top surface of the layer 310 of biological agent. The polymer layer can include one or more polymers. The polymer layer can include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. In one non-limiting example, the polymer layer includes one or more non-porous polymers to at least partially control a rate of release by molecular diffusion of the one or more chemical agents in the one or more surface structures and/or micro-structures 300 and/or in layer 310. The one or more non-porous polymers can include, but are not limited to, parylene C, parylene N, parylene F and/or a parylene derivative. As can be appreciated, a layer that includes one or more chemical agents, not shown, can be coated on layer 320 to provide a burst of one or more biological agent in the treatment area after insertion of the stent. As can also be appreciated, other combinations of polymer layer and layer of biological agent can be used on the medical. These other combinations are also encompassed within the scope of the present invention.

Referring now to FIGURE 10, the base structure 40 of stent 20 includes one or more needles or micro-needles 350. The one or more needles or micro-needles are formed on the surface of the base structure. The one or more needles or micro-needles are formed from one or more chemical agents and/or one or more polymer 360. A layer 362 of biological agent and/or polymer is also formed on the surface of the base structure. In one non-limiting example, the one or more needles or micro-needles 350 are formed from one or more chemical agents that include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. In this non-limiting example, layer 362 is also formed from one or more chemical agents that include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. As can be appreciated, the one or more chemical agents in layer 362 and forming the one or more needles or micro-needles 350 can be the same or different. The use of one or more chemical agents to coat the top surface of the base structure and/or to form one or more needles or micro-needles can provide a burst of one or more biological agent in the treatment area (e.g., body passageway, etc.) after insertion of the stent. In another and/or alternative non-limiting example, the one or more needles or micro-needles 350 are formed from one or more chemical agents that include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. In this non-limiting example, layer 362 is formed from one or more polymers. The polymer layer can include one or more polymers. The polymer layer can include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. When the one or more polymers are non-porous polymers, the one or more non-porous polymers can include, but are not limited to, parylene C, parylene N, parylene F and/or a parylene derivative. The use of one or more chemical agents to form one or more needles or micro-needles can provide a burst of one or more biological agent in the treatment area (e.g., body passageway, etc.) after insertion of the stent. In still another and/or alternative non-limiting example, the one or more needles or micro-needles 350 are formed from one or more polymers. The polymer layer can include one or more polymers. The polymer layer can include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PDLLA, PCL, PDS, 85/15 PDLGA, 75/25 PDLGA, 50/50 PDLGA, 25/75 PDLGA,15/85 PDLGA, chitosan and/or derivatives of one or more of these polymers. When the one or more polymers are non-porous polymers, the one or more non-porous polymers can include, but are not limited to, parylene C, parylene N, parylene F and/or a parylene derivative. In this non-limiting example, layer 362 is formed from one or more chemical agents that include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The use of one or more chemical agents to form layer 362 can provide a burst of one or more biological agent in the treatment area (e.g., body passageway, etc.) after insertion of the stent; however, this is not required.

Referring now to FIGURE 11, the base structure 40 of stent 20 includes one or more needles or micro-needles 400. The one or more needles or micro-needles are formed on the surface of the base structure. The one or more needles or micro-needles are formed from one or more chemical agents and one or more polymers 410. A layer 412 of biological agent and/or polymer is also formed on the surface of the base structure. As can be appreciated, the composition of layer 412 and forming the composition of the one or more needles or micro-needles 400 can be the same or different. In one non-limiting example, the one or more chemical agents that at least partially forms layer 412 and/or the one or more needles or micro-needles 400 include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The one or more polymers that at least partially form layer 412 and/or the one or more needles or micro-needles 400 can include one or more porous and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PGA, PDLLA, PCL, PDS, 85/15 PDLGA, 75/25 PDLGA, 50/50 PDLGA, 25/75 PDLGA,15/85 PDLGA, chitosan and/or derivatives of one or more of these polymers. In one non-limiting example, the one or more polymers that at least partially form layer 412 and/or the one or more needles or micro-needles 400 include a non-porous polymer to at least partially control a rate of release by molecular diffusion of the one or more chemical agents that are mixed with the polymer. The inclusion of one or more chemical agents in the one or more needles or micro-needles can provide controlled release of biological agent in the treatment area (e.g., body passageway, etc.) after insertion of the stent; however, this is not required. The use of one or more chemical agents to form layer 412 and/or one or more needles or micro-needles 400 can provide a burst of one or more biological agent in the treatment area (e.g., body passageway, etc.) after insertion of the stent; however, this is not required.

Referring now to FIGURE 12, FIGURE 12 is a modification of the arrangement illustrated in FIGURE 10. In FIGURE 12, a coating 470, that is formed of one or more polymers and/or chemical agents is placed over one or more needles or micro-needles 450 and layer 462. Specifically, the base structure 40 of stent 20 includes one or more needles or micro-needles 450. The one or more needles or micro-needles are formed on the surface of the base structure. The one or more needles or micro-needles are formed from one or more chemical agents and/or polymers 460. A layer 462 of biological agent and/or polymer is also formed on the surface of the base structure. The composition of layer 462 and one or more needles or micro-needles can be the same or different. In one non-limiting example, the one or more chemical agents that can at least partially form layer 463 and/or one or more needles or micro-needles 450 include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The one or more polymers that can at least partially form layer 463 and/or one or more needles or micro-needles include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PGA, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. In one non-limiting example, the one or more polymers that can at least partially form layer 463 and/or one or more needles or micro-needles 450 include one or more non-porous polymer such as, but not limited to, parylene C, parylene N, parylene F and/or a parylene derivative. The one or more non-porous polymers can be used to at least partially control a rate of release by molecular diffusion of the one or more chemical agents in layer 463 and/or in the one or more needles or micro-needles 450; however, this is not required. Layer 470 that is coated on the top of the one or more needles or micro-needles and layer 462 includes one or more chemical agents and/or polymers. In one non-limiting example, the one or more chemical agents that can at least partially form layer 470 include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. In one non-limiting example, the one or more polymers that can at least partially form layer 470 include one or more porous and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PGA, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. When the one or more polymers include one or more non-porous polymers, such non-porous polymer can include, but is not limited to, parylene C, parylene N, parylene F and/or a parylene derivative. The one or more non-porous polymers can be used to at least partially control a rate of release by molecular diffusion of the one or more chemical agents in layer 463, layer 470 and/or in the one or more needles or micro-needles 450; however, this is not required. When one or more chemical agents at least partially form layer 470 and/or are coated on layer 470, not shown, the one or more chemical agents can provide a burst of one or more biological agent in the treatment area (e.g., body passageway, etc.) after insertion of the stent; however, this is not required.

Referring now to FIGURE 13, FIGURE 13 is a modification of the arrangement illustrated in FIGURE 11. In FIGURE 13, a coating 520, that is formed of one or more polymers and/or chemical agents is placed over one or more needles or micro-needles 500 and layer 512. The composition of layer 520 and layer 512 and/or one or more needles or micro-needles can be the same or different. Specifically, the base structure 40 of stent 20 includes one or more needles or micro-needles 500. The one or more needles or micro-needles are formed on the surface of the base structure. The one or more needles or micro-needles are formed from a mixture of one or more chemical agents and one or more polymers 510. A layer 512 of biological agent and polymer is also formed on the surface of the base structure. As can be appreciated, layer 512 and/or one or more needles or micro-needles 500 can be formed only of one or more polymers or one or more chemical agents. The composition of layer 512 and one or more needles or micro-needles 500 can be the same or different. In one non-limiting example, the one or more chemical agents that can at least partially form layer 512 and/or one or more needles or micro-needles 500 include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The one or more polymers that can at least partially form layer 512 and/or one or more needles or micro-needles 500 include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PGA, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. In one non-limiting example, the one or more polymers that can at least partially form layer 512 and/or one or more needles or micro-needles 500 include one or more non-porous polymers such as, but not limited to, parylene C, parylene N, parylene F and/or a parylene derivative. The one or more non-porous polymers can be used to at least partially control a rate of release by molecular diffusion of the one or more chemical agents in layer 512 and/or in the one or more needles or micro-needles 500; however, this is not required. In one non-limiting example, the one or more polymers that can at least partially form layer 520 include one or more porous and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PGA, PDLLA, PCL, PDS, 85/15 PDLGA, 75/25 PDLGA, 50/50 PDLGA, 25/75 PDLGA,15/85 PDLGA, chitosan and/or derivatives of one or more of these polymers. When the one or more polymers include one or more non-porous polymers, such non-porous polymer can include, but not limited to, parylene C, parylene N, parylene F and/or a parylene derivative. The one or more non-porous polymers can be used to at least partially control a rate of release by molecular diffusion of the one or more chemical agents in layer 512, layer 520 and/or in the one or more needles or micro-needles 500; however, this is not required. When one or more chemical agents at least partially forms layer 520 and/or are coated on layer 520, not shown, the one or more chemical agents can provide a burst of one or more biological agent in the treatment area (e.g., body passageway, etc.) after insertion of the stent; however, this is not required.

Referring now to FIGURE 14, FIGURE 14 is another modification of the arrangement illustrated in FIGURE 11. In FIGURE 14, one or more internal channels 570 are formed in one or more needles or micro-needles 550. The one or more internal channels 570 can include one or more biological agent and/or polymers. Specifically, the base structure 40 of stent 20 includes one or more needles or micro-needles 550. The one or more needles or micro-needles are formed on the surface of the base structure. The one or more needles or micro-needles are formed from one or more polymers and/or chemical agents 560. A layer 562 of polymer and/or biological agent is also formed on the surface of the base structure. The composition of layer 562 and one or more needles or micro-needles can be the same or different. The one or more polymers that can at least partially form layer 562 and/or one or more needles or micro-needles 550 include one or more porous polymers and/or non-porous polymers, and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PGA, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. In one non-limiting example, the one or more polymers that can at least partially form layer 562 and/or one or more needles or micro-needles 550 include one or more non-porous polymers such as, but not limited to, parylene C, parylene N, parylene F and/or a parylene derivative. The one or more non-porous polymers can be used to at least partially control a rate of release by molecular diffusion of the one or more chemical agents in layer 562, in the one or more needles or micro-needles 550, and/or in one or more internal channels 570; however, this is not required. One or more of the needles or micro-needles 550 include an internal channel 570. The internal channel is illustrated as including one or more chemical agents 580; however, it can be appreciated that one or more channels can include a mixture of one or more polymers and/or chemical agents, or only one or more polymers. In one non-limiting example, the one or more chemical agents includes trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. The top opening of the channel enables delivery of one or more chemical agents directly into treatment area (e.g., a wall of a body passageway or organ, etc.). The one or more chemical agents in internal channel 570 can pass through and/or molecularly diffuse through the one or more polymers that at least partially form the one or more needles or micro-needles; however, this is not required. The release of the one or more chemical agents through the one or more polymers that at least partially forms the one or more needles or micro-needles can be a controlled or an uncontrolled release rate. As can be appreciated, a layer of biological agent, not shown, can be coated on one or more needles or micro-needles 550. The layer of biological agent could include one or more chemical agents. The placement of the layer of biological agent on the one or more needles or micro-needles 550 can provide a burst of one or more chemical agents in the treatment area; however, this is not required. As can be appreciated, other combinations of polymer layer and/or layer of biological agent can be used on the stent. As can also or alternatively be appreciated, a layer of polymer, not shown, can be coated on one or more needles or micro-needles 550. The layer of polymer could include one or more polymers. The placement of the layer of polymer on the one or more needles or micro-needles 550 can be used to a) at least partially control a release rate of one or more chemical agents from the stent, and/or 2) provide structural support and/or protection to one or more needles or micro-needles. As can be appreciated, the polymer layer, when used, can have other or additional functions. These other combinations are also encompassed within the scope of the present invention.

Referring now to FIGURE 17, there is illustrated an enlarged portion of a surface of a stent 20 which includes a surface needle, micro-needle or other type of structure or microstructure 700. The needle is shown to include at least one biological agent 710; however, the needle can also or alternatively include one or more polymers, adhesives, etc. The stent, when in the form of a stent, is illustrated as being in an expanded state. When the stent is inserted or expanded in a treatment area, the needle 700 on the outer surface of the stent engages and/or at least partially penetrates into blood vessel or organ V. When the needle includes one or more chemical agents, the one or more chemical agents are at least partially locally applied to a treatment area. This can be a significant advantage over system wide treatment with one or more chemical agents. The local treatment with one or more biological agent via the needle can more effectively and/or efficiently direct the desired agents to a treated area. The release of one or more chemical agents from the needle can be controlled, if desired, to direct the desired amount of one or more chemical agents to a treated area over a desired period of time. When the stent is expanded in a blood vessel, the one or more needles enable local delivery of one or more chemical agents into the wall of the blood vessel. This local delivery is especially advantageous in large and/or thick blood vessels wherein system wide drug treatment is not very effective. In addition, the local delivery of biological agent by the needle directly into the blood vessel can be more effective than only releasing the biological agent from the surface of the stent since diffusion from the surface of the stent to the larger and/or thicker blood vessel may not be as effective as direct delivery by the needles to the blood vessel. The one or more needles on the stent surface can also or alternatively be used to facilitate in securing the stent to the treatment area during the expansion and/or insertion of the stent in a treatment area.

Referring now to FIGURE 15, there is provided a surgical graft 600 (not forming part of the invention). The surgical graft is typically at least partially formed of a flexible material. The material used to form the surgical graft is selected to withstand the manufacturing process that is needed to be accomplished in order to produce the surgical graft. These manufacturing processes can include, but are not limited to, ion beam deposition or implantation, sputter coating, vacuum deposition and/or other coating processes. One non-limiting material is Gortex; however, other or additional materials can be used (e.g., polyethylene tetraphthlate (Dacron), expanded polytetrafluoroethylene (e.g., Gortex, Impra, etc.), etc. The surgical graft can be used in a variety of body passageways. One non-limiting use of the surgical graft is to graft to or replace a portion of a damaged blood vessel. The surgical graft 600 has a generally tubular shape; however, many other shapes can be used. As best illustrated in FIGURE 16, the surgical graft (not forming part of the invention) includes a body portion 610. The inner surface 620 of the body portion includes a plurality of threads 630 extending from the inner surface 630 of the body portion. A layer 640 including one or more chemical agents and/or polymers is applied to the inner surface of the body portion. As illustrated in FIGURE 16, layer 640 only partially encapsulates threads 630; however, this is not required. It can be appreciated that layer 640 is applied in sufficient quantity to fully encapsulate threads 630. In one non-limiting example, the one or more chemical agents in layer 640 include trapidil, trapidil derivatives, warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof. As can be appreciated, layer 640 can include a combination of biological agent and polymer, or only a polymer. Referring again to FIGURE 16, a layer 650 is coated on layer 640. Layer 650 can include one or more polymers. The layer can include one or more porous and/or non-porous polymers. and/or one or more biostable and/or biodegradable polymers. Non-limiting examples of one or more polymers that can be used include, but are not limited to, parylene, parylene C, parylene N, parylene F, PLGA, PEVA, PLA, PBMA, POE, PGA, PLLA, PAA, PEG, PGA, PDLLA, PCL, PDS, 85/15 PDLGA, 75/25 PDLGA, 50/50 PDLGA, 25/75 PDLGA, 15/85 PDLGA, chitosan and/or derivatives of one or more of these polymers. In one non-limiting example, the polymer layer includes one or more non-porous polymers to at least partially control a rate of release by molecular diffusion of the one or more chemical agents in layer 640 and/or layer 650. The one or more non-porous polymers can include, but are not limited to, parylene C, parylene N, parylene F and/or a parylene derivative. Layer 650 is shown to only partially encapsulate threads 630. As can be appreciated, sufficient amount of layer 650 can be used to fully encapsulate thread 630. As can be appreciated, a layer of biological agent, not shown, can be coated on layer 650. The layer of biological agent could include one or more chemical agents. The placement of the layer of biological agent on the top surface of layer 650 can provide a burst of one or more chemical agents in the treatment area (e.g., body passageway, etc.) after insertion of the surgical graft; however, this is not required. As can be appreciated, other combinations of polymer layer and layer of biological agent can be used on the surgical graft. Non-limiting examples of such combinations are illustrated in FIGURES 3-14.

The following is a non-limiting example of the manufacture of a medical device in the form of a stent. A medical device structure in the form of a stent for use in a body passageway (e.g., vascular system, etc.) is selected. The base structure is formed of a durable, biostable metal material. As can be appreciated, the base structure can be made of a nonmetallic material and/or a biodegradable material. The surface of the base structure of the medical device is plasma etched and/or cleaned. A porous or non-porous polymer layer is applied to the etched surface of the base structure. One or more chemical agents are then applied to the surface of the polymer layer. As can be appreciated, the one or more chemical agents can be applied to the surface of the stent prior to applying the porous or non-porous polymer layer. At least one non-porous polymer layer is applied over the one or more layers of chemical agents so as to at least partially control the rate of release of the one or more chemical agents by molecular diffusion through the non-porous polymer layer. The one or more non-porous polymers can include, but are not limited to, parylene C, parylene N, parylene F and/or a parylene derivative. A layer of chemical agents can be applied over the final non-porous polymer layer for an additional fast release of the biological agent; however, this is not required. The at least one non-porous layer is applied via polymerization from a monomer vapor which is solvent or catalyst-free and is self curing. At least one layer of the biological agent can be deposited on the surface of the base structure or polymer by a number of methods such as, but not limited to, dipping, rolling, brushing, spraying, particle atomization, sonication or the like. Sonication can be used to deposit one or more chemical agents and/or polymers on the surface of the base structure. Sonication involves the application of ultrasonic waves to a stream of fluid that may or may not contain the biological agent and/or the polymer material. The fluid can include, but is not limited to, methanol, ethanol, isopropanol, acetone, water, saline, or any other organic or inorganic solvent. The sonicated stream is broken into droplets of the fluid that may vary in size from less than 1 micron to several microns in diameter or more. The size of the droplets can be varied by controlling the frequency of the sonicating device. The droplets can be applied to the medical device evenly or in various thickness configurations through controlling the rotation of the medical device. The one or more chemical agents that are applied to the medical device can include, but are not limited to, trapidil, trapidil derivatives. warfarin (Coumadin) and/or derivatives, aspirin and/or derivatives, clopidogrel and/or derivatives, ticlopadine and/or derivatives, hirdun and/or derivatives, dipyridamole and/or derivatives, and/or heparin and/or low molecular weight heparin and/or derivatives. The one or more chemical agents can also or alternatively include taxol, taxol derivatives, cytochalasin, cytochalasin derivatives, paclitaxel, paclitaxel derivatives, rapamycin, rapamycin derivatives, GM-CSF, GM-CSF derivatives, or combinations thereof.

The medical device can be used in conjunction with other chemical agents. For instance, the success of the medical device can be enhanced by infusing, injecting and/or consuming orally the same and/or different chemical agents that are being released from the medical device. The introduction of one or more chemical agents from a source other than the medical device can have an additive or synergistic effect which can enhance the success of the medical device. Solid dosage forms of one or more chemical agents for oral administration can be used. Such solid forms can include, but are not limited to, capsules, tablets, effervescent tablets, chewable tablets, pills, powders, sachets, granules and gels. In such solid dosage forms, the one or more chemical agents can be admixed with at least one filler material such as, but not limited to, sucrose, lactose or starch. Such dosage forms can also comprise, as in normal practice, additional substances such as, but not limited to, inert diluents (e.g., lubricating agents, etc.). When capsules, tablets, effervescent tablets or pills are used, the dosage form can also include buffering agents. Soft gelatin capsules can be prepared to contain a mixture of the biological agent in combination with vegetable oil or other types of oil. Hard gelatin capsules can contain granules of the biological agent in combination with a solid carrier such as, but not limited to, lactose, potato starch, corn starch, cellulose derivatives of gelatin, etc. Tablets and pills can be prepared with enteric coatings for additional time release characteristics. Liquid dosage forms of the biological agent for oral administration can include pharmaceutically acceptable emulsions, solutions, suspensions, syrups, elixirs, etc.

## Claims

1. A medical device designed for insertion in a body passageway comprising:
a. a stent with abluminal and luminal surfaces and comprising at least 60 weight percent of a metal alloy comprising as primary metals molybdenum and rhenium, wherein a "primary metal" is a metal component of the metal alloy that is not a metal impurity, the metal alloy being
(i) a solid solution or homogenous solution including the primary metals (rhenium and molybdenum) in at least about 95 wt.-%, or
(ii) a solid solution including the primary metals rhenium and molybdenum at about 95-99 wt.-%;
b. a base drug coating applied to the device;
c. a bioabsorbable coating;
d. a secondary drug coating;
wherein the base drug coating is comprised of 100% drug and is sufficient to suppress inflammation resulting from absorption of the bioabsorbable coating;
wherein the secondary drug coating is applied to the luminal surface of the device;
wherein the secondary drug coating is sufficient to promote the growth of endothelium on the luminal surface of the stent.

2. The device as defined in claim 1, wherein the base drug coating is present on the device until complete absorption of the bioabsorbable coating.

3. The device as defined in any one of claims 1 or 2, wherein the base drug coating is applied to the abluminal surface of the device.

4. The device as defined in claim 3, wherein the thickness of the abluminal coating varies from about 2 to about 50 microns.

5. The device as defined in any one of claims 1-4, wherein the thickness of the luminal coating varies from about 2 to about 50 microns.

6. The device as defined in any one of claims 1-5, wherein the bioabsorbable coating is selected from the group consisting of PGA, PLA, PLLA, PDLLA, PCL, PDS, 85/15 PDLGA, 75/25 PDLGA, 50/50 PDLGA, 25/75 PDLGA, 15/85 PDLGA, and combinations thereof.

7. The device as defined in any one of claims 1-6, wherein the weight percent of bioabsorbable coating to drug ranges from about 1 to about 99 weight percent.

8. The device as defined in any one of claims 1-7, wherein the absorption time of the bioabsorbable coating is at least about 1 month; or wherein the absorption time of the bioabsorbable coating is up to about 12 months; or wherein the absorption time of the bioabsorbable coating is up to about 6 months.

9. The device as defined in any one of claims 1-8, wherein the absorption time of the bioabsorbable coating is not greater than the time for complete elution of drug from the device; or wherein the absorption time of the bioabsorbable coating is greater than the time for complete elution of drug from the device.

10. The device as defined in any one of claims 1-9, wherein 100 percent of the drug elutes from the bioabsorbable or luminal coating in less than about 12 months; or wherein less than 100 percent of the drug elutes from the bioabsorbable or luminal coating within about 60 days; or wherein less than 100 percent of the drug elutes from the bioabsorbable or luminal coating within about 30 days; or wherein less than 80 percent of the drug elutes from the bioabsorbable or luminal coating within about 30 days.

11. The device in as defined in any one of claims 1-10, wherein the bioabsorbable coating is coated to the luminal surface of the device.

12. The device as defined in any one of claims 1-11, wherein the bioabsorbable coating is coated to the abluminal surface of the device.

## Patentansprüche

1. Medizinische Vorrichtung, die zum Einsetzen in einen Körperdurchgang bestimmt ist, umfassend:
a. einen Stent mit abluminalen und luminalen Oberflächen, der mindestens 60 Gewichtsprozent einer Metalllegierung umfasst, die als primäre Metalle Molybdän und Rhenium umfasst, wobei ein "primäres Metall" eine Metallkomponente der Metalllegierung ist, die keine Metallverunreinigung ist, wobei die Metalllegierung
(i) eine feste Lösung oder homogene Lösung ist, die die primären Metalle (Rhenium und Molybdän) in mindestens etwa 95 Gew.-% enthält, oder
(ii) eine feste Lösung ist, die die primären Metalle Rhenium und Molybdän zu etwa 95-99 Gew.-% enthält;
b. eine Arzneimittelgrundbeschichtung, die auf die Vorrichtung aufgebracht ist;
c. eine bioresorbierbare Beschichtung;
d. eine sekundäre Arzneimittelbeschichtung;
wobei die Arzneimittelgrundbeschichtung zu 100 % aus Arzneimittel besteht und ausreicht, um eine Entzündung zu unterdrücken, die aus der Absorption der bioresorbierbaren Beschichtung resultiert;
wobei die sekundäre Arzneimittelbeschichtung auf die luminale Oberfläche der Vorrichtung aufgebracht ist;
wobei die sekundäre Arzneimittelbeschichtung ausreicht, um das Wachstum von Endothel auf der luminalen Oberfläche des Stents zu fördern.

2. Vorrichtung nach Anspruch 1, wobei die Arzneimittelgrundbeschichtung bis zur vollständigen Absorption der bioresorbierbaren Beschichtung auf der Vorrichtung vorhanden ist.

3. Vorrichtung nach einem der Ansprüche 1 oder 2, wobei die Arzneimittelgrundbeschichtung auf die abluminale Oberfläche der Vorrichtung aufgebracht ist.

4. Vorrichtung nach Anspruch 3, wobei die Dicke der abluminalen Beschichtung von etwa 2 bis etwa 50 Mikrometer variiert.

5. Vorrichtung nach einem der Ansprüche 1-4, wobei die Dicke der luminalen Beschichtung von etwa 2 bis etwa 50 Mikrometer variiert.

6. Vorrichtung nach einem der Ansprüche 1-5, wobei die bioresorbierbare Beschichtung aus der Gruppe bestehend aus PGA, PLA, PLLA, PDLLA, PCL, PDS, 85/15 PDLGA, 75/25 PDLGA, 50/50 PDLGA, 25/75 PDLGA, 15/85 PDLGA und Kombinationen davon ausgewählt ist.

7. Vorrichtung nach einem der Ansprüche 1-6, wobei die Gewichtsprozent der bioresorbierbaren Beschichtung zu Arzneimittel im Bereich von etwa 1 bis etwa 99 Gewichtsprozent liegen.

8. Vorrichtung nach einem der Ansprüche 1 bis 7, wobei die Absorptionszeit der bioresorbierbaren Beschichtung mindestens etwa 1 Monat beträgt; oder wobei die Absorptionszeit der bioresorbierbaren Beschichtung bis zu etwa 12 Monate beträgt; oder wobei die Absorptionszeit der bioresorbierbaren Beschichtung bis zu etwa 6 Monate beträgt.

9. Vorrichtung nach einem der Ansprüche 1-8, wobei die Absorptionszeit der bioresorbierbaren Beschichtung nicht größer ist als die Zeit bis zur vollständigen Elution des Arzneimittels aus der Vorrichtung; oder wobei die Absorptionszeit der bioresorbierbaren Beschichtung größer ist als die Zeit bis zur vollständigen Elution des Arzneimittels aus der Vorrichtung.

10. Vorrichtung nach einem der Ansprüche 1-9, wobei 100 Prozent des Arzneimittels aus der bioresorbierbaren oder luminalen Beschichtung in weniger als etwa 12 Monaten eluiert werden; oder wobei weniger als 100 Prozent des Arzneimittels aus der bioresorbierbaren oder luminalen Beschichtung innerhalb von etwa 60 Tagen eluiert werden; oder wobei weniger als 100 Prozent des Arzneimittels aus der bioresorbierbaren oder luminalen Beschichtung innerhalb von etwa 30 Tagen eluiert werden; oder wobei weniger als 80 Prozent des Arzneimittels aus der bioresorbierbaren oder luminalen Beschichtung innerhalb von etwa 30 Tagen eluiert werden.

11. Vorrichtung nach einem der Ansprüche 1-10, wobei die bioresorbierbare Beschichtung auf die luminale Oberfläche der Vorrichtung aufgebracht ist.

12. Vorrichtung nach einem der Ansprüche 1-11, wobei die bioresorbierbare Beschichtung auf die abluminale Oberfläche der Vorrichtung aufgebracht ist.

## Revendications

1. Dispositif médical conçu pour être inséré dans un passage corporel comprenant :
a. un stent avec des surfaces abluminale et luminale et comprenant au moins 60 pourcent en poids d'un alliage métallique comprenant du molybdène et du rhénium en tant que métaux primaires, dans lequel un « métal primaire » est un composant métallique de l'alliage métallique lequel n'est pas une impureté métallique, l'alliage métallique étant
(i) une solution solide ou une solution homogène incluant les métaux primaires (rhénium et molybdène) dans au moins 95% en poids, ou
(ii) une solution solide incluant les métaux primaires rhénium et molybdène à environ 95-99% en poids ;
b. un enrobage de médicament de base appliqué sur le dispositif ;
c. un enrobage bio-absorbable ;
d. un enrobage de médicament secondaire ;
dans lequel l'enrobage de médicament de base est constitué à 100% de médicament et suffit pour supprimer une inflammation résultant de l'absorption de l'enrobage bio-absorbable ;
dans lequel l'enrobage de médicament secondaire est appliqué sur la surface luminale du dispositif ;
dans lequel l'enrobage de médicament secondaire suffit pour favoriser la croissance de l'endothélium sur la surface luminale du stent.

2. Dispositif selon la revendication 1, dans lequel l'enrobage de médicament de base est présent sur le dispositif jusqu'à l'absorption complète de l'enrobage bio-absorbable.

3. Dispositif selon l'une quelconque des revendications 1 ou 2, dans lequel l'enrobage de médicament de base est appliqué sur la surface abluminale du dispositif.

4. Dispositif selon la revendication 3, dans lequel l'épaisseur de l'enrobage abluminal varie entre environ 2 et environ 50 microns.

5. Dispositif selon l'une quelconque des revendications 1 à 4, dans lequel l'épaisseur de l'enrobage luminal varie entre environ 2 et environ 50 microns.

6. Dispositif selon l'une quelconque des revendications 1 à 5, dans lequel l'enrobage bio-absorbable est sélectionné parmi le groupe comprenant le PGA, le PLA, le PLLA, le PDLLA, le PCL, le PDS, le PDLGA 85/15, le PDLGA 75/25, le PDLGA 50/50, le PDLGA 25/75, le PDLGA 15/85, et des combinaisons de ceux-ci.

7. Dispositif selon l'une quelconque des revendications 1 à 6, dans lequel le pourcentage en poids de l'enrobage bio-absorbable par rapport au médicament varie entre environ 1 et environ 99 pourcent en poids.

8. Dispositif selon l'une quelconque des revendications 1 à 7, dans lequel le temps d'absorption de l'enrobage bio-absorbable est au moins d'environ 1 mois ; ou dans lequel le temps d'absorption de l'enrobage bio-absorbable va jusqu'à environ 12 mois ; ou dans lequel le temps d'absorption de l'enrobage bio-absorbable va jusqu'à environ 6 mois.

9. Dispositif selon l'une quelconque des revendications 1 à 8, dans lequel le temps d'absorption de l'enrobage bio-absorbable n'est pas supérieur au temps nécessaire à l'élution complète du médicament par rapport au dispositif ; ou dans lequel le temps d'absorption de l'enrobage bio-absorbable est supérieur au temps nécessaire à l'élution complète du médicament par rapport au dispositif.

10. Dispositif selon l'une quelconque des revendications 1 à 9, dans lequel 100 pourcent du médicament s'élue de l'enrobage bio-absorbable ou luminal en moins d'environ 12 mois ; ou dans lequel moins de 100 pourcent du médicament s'élue de l'enrobage bio-absorbable ou luminal sous environ 60 jours ; ou dans lequel moins de 100 pourcent du médicament s'élue de l'enrobage bio-absorbable ou luminal sous environ 30 jours ; ou dans lequel moins de 80 pourcent du médicament s'élue de l'enrobage bio-absorbable ou luminal sous environ 30 jours.

11. Dispositif selon l'une quelconque des revendications 1 à 10, dans lequel l'enrobage bio-absorbable est appliqué sur la surface luminale du dispositif.

12. Dispositif selon l'une quelconque des revendications 1 à 11, dans lequel l'enrobage bio-absorbable est appliqué sur la surface abluminale du dispositif.
